# EUROPEAN PATENT SPECIFICATION

(11) **EP 3 237 638 B1**
(45) Date of publication and mention of the grant of the patent: **15.01.2020**
(21) Application number: 15831090.4
(22) Date of filing: 23.12.2015
(51) Int. Cl.: C12Q 1/68

(54) **THERAPEUTIC, DIAGNOSTIC AND PROGNOSTIC METHODS FOR CANCER OF THE BLADDER**
THERAPEUTISCHES, DIAGNOSTISCHES UND PROGNOSTISCHES VERFAHREN FÜR BLASENKREBS
MÉTHODES DE TRAITEMENT, DE DIAGNOSTIC ET DE PRONOSTIC DU CANCER DE LA VESSIE

(30) Priority: 24.12.2014 US 201462096741 P
(43) Date of publication of application: 01.11.2017
(60) Divisional application: 19212435.2
(73) Proprietor: F. Hoffmann-La Roche AG, 4070 Basel (CH)
(72) Inventor: CHOI, Younjeong, South San Francisco, CA 94080-4990 (US); KABBARAH, Omar, South San Francisco, CA 94080-4990 (US); KIM, Doris, South San Francisco, CA 94080-4990 (US)
(74) Representative: Brodbeck, Michel
(86) International application number: PCT/US2015/000237
(87) International publication number: WO 2016/105503

(56) References cited:
- WO-A1-2014/135655
- WO-A1-2014/151853
- US-A1- 2013 059 303
- MOOSO BENJAMIN A ET AL: "The role of EGFR family inhibitors in muscle invasive bladder cancer: a review of clinical data and molecular evidence.", THE JOURNAL OF UROLOGY JAN 2015, vol. 193, no. 1, 23 August 2014 (2014-08-23), pages 19-29, XP029113056, ISSN: 1527-3792
- PARK JINSUNG ET AL: "Do molecular biomarkers have prognostic value in primary T1G3 bladder cancer treated with bacillus Calmette-Guerin intravesical therapy?", UROLOGIC ONCOLOGY AUG 2013, vol. 31, no. 6, August 2013 (2013-08), pages 849-856, XP002756572, ISSN: 1873-2496
- KIM WUN-JAE ET AL: "A four-gene signature predicts disease progression in muscle invasive bladder cancer.", MOLECULAR MEDICINE (CAMBRIDGE, MASS.) 2011 MAY-JUN, vol. 17, no. 5-6, May 2011 (2011-05), pages 478-485, XP002756573, ISSN: 1528-3658
- CHAKRAVARTI A ET AL: "Expression of the epidermal growth factor receptor and Her-2 are predictors of favorable outcome and reduced complete response rates, respectively, in patients with muscle-invading bladder cancers treated by concurrent radiation and cisplatin-based chemotherapy: A report from the Radiation Therapy O", INTERNATIONAL JOURNAL OF RADIATION: ONCOLOGY BIOLOGY PHYSICS, PERGAMON PRESS, USA, vol. 62, no. 2, 1 June 2005 (2005-06-01), pages 309-317, XP025262773, ISSN: 0360-3016, DOI: 10.1016/J.IJROBP.2004.09.047 [retrieved on 2005-06-01]
- SHINOHARA ASANO ET AL: "Association of TP53 and MDM2 polymorphisms with survival in bladder cancer patients treated with chemoradiotherapy", CANCER SCIENCE, vol. 100, no. 12, December 2009 (2009-12), pages 2376-2382, XP002756574, ISSN: 1347-9032
- MAZZOLA CLARISSE R ET AL: "Dovitinib: rationale, preclinical and early clinical data in urothelial carcinoma of the bladder", EXPERT OPINION ON INVESTIGATIONAL DRUGS, vol. 23, no. 11, November 2014 (2014-11), pages 1553-1562, XP002756575,
- GUST KILIAN M ET AL: "Fibroblast Growth Factor Receptor 3 Is a Rational Therapeutic Target in Bladder Cancer", MOLECULAR CANCER THERAPEUTICS, vol. 12, no. 7, July 2013 (2013-07), pages 1245-1254, XP002756576,
- GAKIS GEORGIOS ET AL: "Current status of molecular markers for prognostication and outcome in invasive bladder cancer", BJU INTERNATIONAL, vol. 110, no. 2, July 2012 (2012-07), pages 233-237, XP002756577,
- LIANG CHENG ET AL: "Bladder cancer: translating molecular genetic insights into clinical practice", HUMAN PATHOLOGY, SAUNDERS, PHILADELPHIA, PA, US, vol. 42, no. 4, 8 July 2010 (2010-07-08), pages 455-481, XP028367813, ISSN: 0046-8177, DOI: 10.1016/J.HUMPATH.2010.07.007 [retrieved on 2010-08-11]
- C Blick ET AL: "Hypoxia regulates FGFR3 expression via HIF-1[alpha] and miR-100 and contributes to cell survival in non-muscle invasive bladder cancer", British Journal of Cancer, vol. 109, no. 1, 18 June 2013 (2013-06-18) , pages 50-59, XP055501346, GB ISSN: 0007-0920, DOI: 10.1038/bjc.2013.240
- Cristina Balbás-Martínez ET AL: "ARID1A Alterations Are Associated with FGFR3-Wild Type, Poor-Prognosis, Urothelial Bladder Tumors", PLoS ONE, vol. 8, no. 5, 1 May 2013 (2013-05-01), page e62483, XP055501364, DOI: 10.1371/journal.pone.0062483

## Description

### FIELD OF THE INVENTION

The present invention is directed to methods for treating, diagnosing, and providing prognoses for cancer, e.g., bladder cancer.

### BACKGROUND

Cancer remains one of the most deadly threats to human health. In the United States, cancer affects nearly 1.3 million new patients each year, and is the second leading cause of death after heart disease, accounting for approximately 1 in 4 deaths. Solid tumors are responsible for most of those deaths. Malignant tumors metastasize and grow rapidly in an uncontrolled manner, making timely detection and treatment extremely difficult.

Bladder cancer is the fifth-most common malignancy worldwide, with close to 400,000 newly diagnosed cases and approximately 150,000 associated deaths reported per year. Approximately 75-80% of bladder cancer patients present with non-muscle-invasive bladder cancer (NMIBC) at the time of initial diagnosis. Although confined to the lamina propria and typically not life-threatening, approximately 50-80% of NMIBCs recur, often requiring costly clinical intervention. NMIBCs can progress in about 20-30% of instances to the more serious muscle-invasive bladder cancer (MIBC). MIBCs (T2 and T3) constitute only ∼10-15% of new cases, but they confer a higher risk for developing metastatic bladder cancer (pT4). Metastatic bladder cancer is associated with a dismal 5-year survival likelihood and represents a major unmet medical need with few effective therapies to date.

Therefore, there remains a need for effective means for treating, diagnosing, and providing prognoses for cancer, for example, bladder cancer.

### SUMMARY OF THE INVENTION

The present invention is directed to methods for treating, diagnosing, and providing prognoses for cancer, e.g., bladder cancer.

In one aspect, the invention features a method of treating a patient suffering from a bladder cancer, the method comprising administering to the patient a therapeutically effective amount of an anti-cancer therapy, wherein the expression level of at least one of the following genes: *FGFR3, TP53,* and *EGFR,* in a sample obtained from the patient has been determined to be increased relative to a reference level of the at least one gene.

In another aspect, the invention features a method for diagnosing a bladder cancer in a patient, the method comprising the steps of: (a) determining the expression level of at least one of the following genes: *FGFR3, TP53,* and *EGFR,* in a sample obtained from the patient; and (b) comparing the expression level of the at least one gene to a reference level of the at least one gene, wherein an increase in the expression level of the at least one gene in the patient sample relative to the reference level identifies a patient having a bladder cancer. In some embodiments, the method further comprises (c) informing the patient that they have a bladder cancer. In some embodiments, the method further comprises (d) selecting an anti-cancer therapy for treatment of said patient when an increase in the level of expression of the at least one gene in the patient sample relative to the reference level is detected. In some embodiments, the method further comprises (e) administering a therapeutically effective amount of an anti-cancer therapy to the patient.

In another aspect, the invention features a method for the prognosis of a patient suffering from bladder cancer, the method comprising: (a) determining the expression level of at least one of the following genes: *FGFR3, TP53,* and *EGFR,* in a sample obtained from the patient; (b) comparing the expression level of the at least one gene to a reference level of the at least one gene; and (c) determining a prognosis for the patient, wherein a poor prognosis is indicated by an expression level of the at least one gene in the patient sample that is increased relative to the reference level. In some embodiments, the prognosis is a prognosis of survival. In some embodiments, the method is carried out prior to administering an anti-cancer therapy to the patient. In some embodiments, the method further comprises (d) identifying the patient as likely to benefit from administration of an anti-cancer therapy when the patient is determined to have a poor prognosis of survival. In some embodiments, the method further comprises (e) administering a therapeutically effective amount of an anti-cancer therapy to the patient, if the patient is determined to have a poor prognosis of survival. In some embodiments, the survival is disease-free survival or overall survival.

In another aspect, the invention features a method of determining whether a patient having a bladder cancer is likely to respond to treatment with an anti-cancer therapy, the method comprising: (a) determining the expression level of at least one of the following genes: *FGFR3, TP53,* and *EGFR,* in a sample obtained from the patient; and (b) comparing the expression level of the at least one gene to a reference level of the at least one gene, wherein an increase in the expression level of the at least one gene in the patient sample relative to the reference level identifies a patient who is likely to respond to treatment comprising an anti-cancer therapy. In some embodiments, the method further comprises (c) administering a therapeutically effective amount of an anti-cancer therapy to the patient.

In another aspect, the invention features a method of optimizing therapeutic efficacy of an anti-cancer therapy for a patient having a bladder cancer, the method comprising: (a) determining the expression level of at least one of the following genes: *FGFR3, TP53,* and *EGFR,* in a sample obtained from the patient; and (b) comparing the expression level of the at least one gene to a reference level of the at least one gene, wherein an increase in the expression level of the at least one gene in the patient sample relative to the reference level identifies a patient who is likely to respond to treatment comprising an anti-cancer therapy. In some embodiments, the method further comprises (c) administering a therapeutically effective amount of an anti-cancer therapy to the patient.

In another aspect, the invention features a method of treating a patient suffering from a bladder cancer, the method comprising administering to the patient a therapeutically effective amount of an anti-cancer therapy other than Bacillus Calmette-Guerin (BCG) vaccine, wherein the expression level of *TP53* in a sample obtained from the patient has been determined to be increased relative to a reference level of *TP53.*

In some embodiments of any one of the above aspects, the anti-cancer therapy comprises an FGFR3 antagonist, a TP53 antagonist, and/or an EGFR antagonist. In some embodiments, the anti-cancer therapy comprises an FGFR3 antagonist and an EGFR antagonist. In some embodiments, the FGFR3 antagonist, EGFR antagonist, or TP53 antagonist is an antibody or a functional fragment thereof. In some embodiments, the FGFR3 antagonist is an anti-FGFR3 antibody, or a functional fragment thereof. In some embodiments, the EGFR antagonist is an anti-EGFR antibody, or a functional fragment thereof. In some embodiments, the TP53 antagonist is an anti-TP53 antibody, or a functional fragment thereof. In some embodiments, the FGFR3 antagonist, TP53 antagonist, or EGFR antagonist is a small molecule antagonist. In some embodiments, the FGFR3 antagonist or EGFR antagonist is a tyrosine kinase inhibitor. In some embodiments, the EGFR antagonist is erlotinib (TARCEVA™). In some embodiments, the anti-cancer therapy further comprises (i) an agent selected from the group consisting of an anti-neoplastic agent, a chemotherapeutic agent, a growth-inhibitory agent, and a cytotoxic agent, (ii) radiotherapy, or (iii) a combination thereof.

In some embodiments of any one of the above aspects, the expression level of the at least one gene in the sample obtained from the patient is determined by measuring mRNA. In some embodiments, the expression level of the at least one gene in the sample obtained from the patient is determined by a polymerase chain reaction (PCR) assay. In some embodiments, the PCR assay is a quantitative PCR assay.

In some embodiments of any one of the above aspects, the expression level of the at least one gene in the sample obtained from the patient is determined by measuring protein. In some embodiments, the expression level of the at least one gene in the sample obtained from the patient is determined by an immunohistochemical (IHC) method.

In some embodiments of any one of the above aspects, the sample obtained from the patient is a tumor sample. In some embodiments, the tumor sample is a formalin-fixed paraffin-embedded (FFPE) tumor sample.

In some embodiments of any one of the above aspects, the method further comprises determining the expression level of at least two of the genes. In some embodiments, the method further comprises determining the expression level of all three of the genes.

In some embodiments of any one of the above aspects, the expression level of *FGFR3* has been determined to be increased at least 2-fold relative to a reference level. In some embodiments, the expression level of *FGFR3* has been determined to be increased at least 4-fold relative to a reference level.

In some embodiments of any one of the above aspects, the expression level of *EGFR* has been determined to be increased at least 4-fold relative to a reference level. In some embodiments, the expression level of *EGFR* has been determined to be increased at least 8-fold relative to a reference level.

In some embodiments of any one of the above aspects, the method further comprises determining the expression level of at least one additional gene selected from the group consisting of: *DUSB3*, *FRS2*, *TSC1*, *ERBB3*, *CDKN1A*, *CCND1*, *TP63*, *MMP2*, *ZEB2*, *PIK3CB*, *PIK3R1*, *MDM2*, *SNAI2*, *AXL*, *ZEB1*, *BCL2B*, *TSC2*, *RB1*, *FGFR32*, *PIK3IP1*, *MTOR*, *PIK3CA*, *PTEN*, *AKT1*, *BCL2A*, *FRS3*, *ERBB2*, *FGFR31*, *FGF1*, *SNAI1*, *FGFR34*, *FGF9*, and *FGF2*, in a sample obtained from the patient, wherein the expression level of the at least one additional gene is changed relative to a reference level of the at least one additional gene.

In some embodiments of any one of the above aspects, the bladder cancer is non-muscle-invasive bladder cancer, muscle-invasive bladder cancer, or metastatic bladder cancer. In some embodiments, the non-muscle-invasive bladder cancer is a recurrent non-muscle-invasive bladder cancer.

### BRIEF DESCRIPTION OF THE DRAWINGS

FIGURE 1 is a table showing information regarding the patients and clinicopathologic features of the bladder cancer tumor specimens which were analyzed in the Examples section (e.g., Examples 1-6).
FIGURES 2A and 2B are graphs showing principle components analysis (PCA) on expression of 18,000 genes from Sjödahl et al. Clin. Cancer Res. 18(12): 3377-3386, 2012 (Figure 2A) and using only 82 genes that overlap with the custom bladder cancer Fluidigm gene expression panel described in the Examples section (see, e.g., Table 1) (Figure 2B). Both analyses show a similar distribution of samples belonging to the bladder cancer subtypes described by Sjödahl et al. (*supra*).
FIGURES 2C and 2D are graphs showing that cross-validation with a centroid-based classifier results in similar classification accuracy of the bladder cancer subtypes of Sjödahl et al. *(supra)* based on 1,000 genes (Figure 2C) and based on 82 genes that overlap with the custom bladder cancer Fluidigm gene expression panel (Figure 2D). The data presented in Figures 2A-2D show that the custom bladder cancer Fluidigm gene expression panel can accurately classify bladder cancer into molecularly-defined subtypes. The line markers are for clarity and do not indicate data points.
FIGURES 3A and 3B are graphs showing that Fluidigm assay performance was not affected by RNA input amount, as shown for FGFR3 (Figure 3A) and for PIK3CA (Figure 3B) with decreasing input amounts of universal RNA (uRNA) controls.
FIGURES 3C and 3D are graphs showing that run-to-run data reproducibility of the custom bladder cancer Fluidigm gene expression panel was high for clinical tissues. Figure 3C (HP-52719) and Figure 3D (HP-50331) show two representative formalin-fixed paraffin-embedded (FFPE)-derived RNA samples that each show R² values of >0.98 between runs from different days.
FIGURE 3E is a graph showing high chip-to-chip data reproducibility of the custom bladder cancer Fluidigm gene expression panel.
FIGURE 4A is a heatmap of transcriptionally-defined tissue clusters from Fluidigm analysis (Tissues) and groups of co-regulated genes (Genes). The Green, Yellow, and Red tissue groups contained the majority of samples.
FIGURE 4B is a graph showing that the Green, Yellow, and Red tissue clusters (see, e.g., Figure 4A) are associated with distinct disease-free survival (DFS) probabilities. The Red group samples had the best DFS profile (Red vs. Yellow: HR=0.54, P=0.03), and the Green group samples were associated with the worst DFS likelihoods (Red vs. Green: HR=0.29, P=0.004).
FIGURE 4C is a graph showing non-invasive and invasive/metastases histology calls indicating that the Green and Red groups included a significantly higher frequency of non-invasive samples than the Yellow group, which included mostly invasive tissues and all metastases (Green vs. Red: P=0.1029, not significant (NS); Green vs. Yellow and Red vs. Yellow: P<0.0001 for both comparisons).
FIGURE 4D is a graph showing the frequency of tumors with micropapillary histology in the three main tissue clusters. A higher frequency of micropapillary histology was observed in the Yellow group (Green vs. Red: P=0.2351, NS; Green vs. Yellow: P=0.1167, NS; Red vs. Yellow: P=0.0063).
FIGURE 4E is a graph showing no significant differences between the Green, Red, and Yellow groups with respect to the prevalence of immune infiltration-positive samples (Green vs. Red: P=0.3332, NS; Green vs. Yellow: P=0.5197, NS; Red vs. Yellow: P=1, NS).
FIGURE 4F is a graph showing the treatment frequency distribution for the three groups. The Green group was more heavily-treated than the Red and Yellow groups (P<0.0001 for both comparisons), and the Red and Yellow groups were treated at a comparable rate (P=0.8836, NS). Treatments included Bacillus Calmette-Guerin (BCG) vaccine, chemotherapy, radiation, or combinations thereof.
FIGURE 5A is a heatmap showing that expression analysis by the custom bladder cancer Fluidigm gene expression panel described in Example 2 identified 3 main subsets of tissues represented by the Green, Yellow, and Red groups, and 2 minor subtypes represented by the Blue and Purple groups.

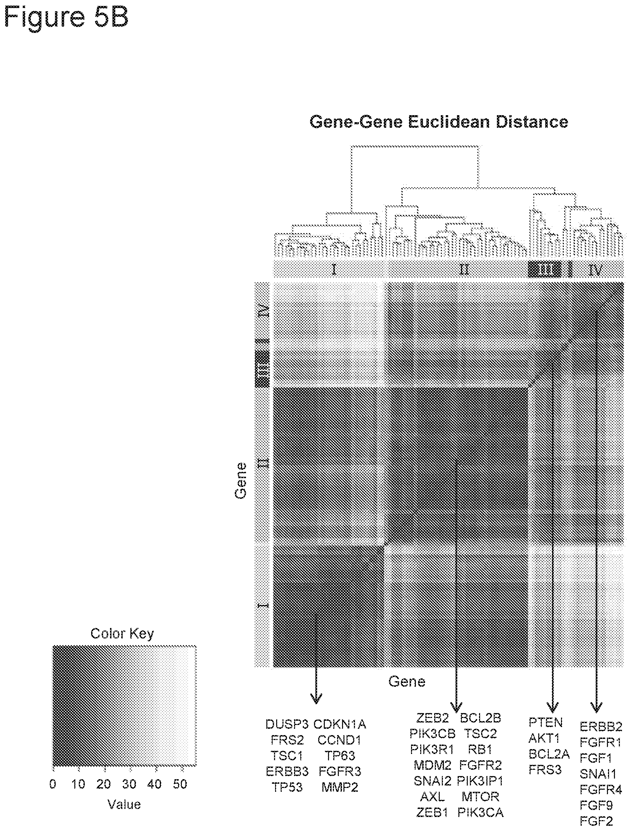

FIGURE 5B is a heatmap showing that expression analysis by the custom bladder cancer Fluidigm gene expression panel as described in Example 2 identified 4 major gene expression clusters associated with different biologies. Arrows point to representative genes for each cluster, including *TP53* and *FGFR3* (pink cluster I); epithelial-to-mesenchymal transition (EMT) genes like *ZEB1*/*2* and phosphoinositide 3-kinase (PI3K) genes such as *PIK3CA* and *PIK3R1* (light blue cluster II); other *PI3K* genes such as *PTEN* and *AKT1* (magenta cluster III); and fibroblast growth factor (FGF) ligands and receptors (light brown cluster IV).
FIGURE 5C is a graph showing that the tissue subtypes described in Figure 5A were associated with distinct disease-free survival (DFS) likelihoods. The line markers are for clarity and do not indicate data points.
FIGURES 6A-6D are representative hematoxylin & eosin (H&E)-stained sections showing histological features of (Figure 6A) non-muscle invasive bladder cancer; (Figure 6B) muscle-invasive bladder cancer; (Figure 6C) lymphoid infiltration-positive tissue; and (Figure 6D) bladder tumor with micropapillary histology. Scale bar indicates 100 µm.
FIGURE 7A is a color map showing *FGFR3* mutation status, FGFR3 immunohistochemistry (IHC), and histopathology calls for specimens in the Green, Red, and Yellow groups.
FIGURE 7B is a graph showing that *FGFR3* transcript levels were higher in mutant (MT) compared to wild-type (WT) samples (P<0.0001) as determined by Fluidigm gene expression analysis.
FIGURE 7C is a graph showing that *FGFR3* mutant samples expressed higher levels of the protein than wild-type tissues, as measured by IHC (P<0.0001).
FIGURE 7D is a graph showing that the *FGFR3* mutation rate was significantly higher in the Green group compared to both the Red and invasive/metastatic Yellow groups (P<0.0001 for both comparisons).
FIGURE 7E is a graph showing that samples in the Green group expressed significantly higher *FGFR3* transcript levels than samples in the Red and Yellow groups (P<0.0001 for both comparisons).
FIGURE 7F is a graph showing that FGFR3 protein levels by IHC were higher in the Green group than in the Red group, and were the lowest in the Yellow group (Green vs. Red: P=0.0343; Red vs. Yellow: P<0.0001).
FIGURE 7G is a series of pie charts showing FGFR3 expression in non-invasive, invasive, and bladder cancer metastases. High protein levels of FGFR3 (IHC 2+/3+) were observed in 66%, 32%, and 33% of cases, respectively.
FIGURE 7H is a series of graphs showing the 3- or 5-year DFS rates for patients with FGFR3-high and -low invasive tumors and bladder cancer metastases, demonstrating lower DFS rates in high-expressing tumors in both settings (expression cutoff = 50th percentile).
FIGURE 7I is a series of graphs showing that rates of overall survival (OS) at 3 and 5 years are lower for FGFR3-high versus low-expressing cases (expression cutoff = 25th percentile; high FGFR3, N=15; low FGFR3, N=24).
FIGURE 7J shows an analysis of a publically-available dataset from Kim et al. Mol. Cancer 9: 3, 2010 indicating a significantly worse OS profile for patients with advanced bladder cancers that express high ("hi") versus low ("lo") levels of FGFR3 by both Kaplan-Meier plots (left panel) and by 3- and 5-year OS rate (right panel) (expression cutoff=75th percentile; high FGFR3, N=10; low FGFR3, N=52).
FIGURE 8 is a graph showing that INGENUITY® software (Qiagen, Redwood City, CA) analysis identified the p53, PI3K-AKT, EMT, and ERBB and FGFR3 pathways as differentially-expressed between the non-invasive rapidly-recurring Green group and the less-aggressive Red group. The 45/96 genes that were significantly differentially-expressed between the Green and Red groups (P<0.05, with multiple testing correct) were used as inputs for the analysis.
FIGURE 9A is a color map of next generation sequencing (NGS) results and mutation status as determined by Fluidigm analysis (MutMap; see Schleifman et al. PloS One 9: e90761, 2014) in samples from the Green, Yellow, and Red groups. Validated mutations were those identified by both NGS and MutMap.
FIGURE 9B is a schematic diagram (lollipop plot) of the *TP53* gene showing the *TP53* mutations identified by NGS in samples for the Green, Red, and Yellow groups. Colored boxes denote tissue group assignment. Boxes with similar numbers point to mutations co-detected in the same samples. Most mutations cluster in the DNA-binding domain and are non-overlapping.
FIGURE 9C is a graph showing that there was a significantly higher rate of *TP53* mutation in the rapidly-recurring Green group compared to the more benign Red group (P=0.03). *TP53* mutation frequencies were not significantly different between the Red and Yellow or the Green and Yellow groups (P=0.1227, NS; and P=0.3605, NS).
FIGURE 9D is a graph showing that expression levels of *TP53* were higher in the Green group compared to both Red and Yellow groups, as determined by Fluidigm gene expression analysis (P=0.0187 and P=0.0020, respectively).
FIGURE 9E is a graph showing that expression levels of the TP53 transcriptional target p21 were significantly higher in the Green compared to both the Red and Yellow groups (P<0.0001 for both comparisons), as determined by Fluidigm gene expression analysis.
FIGURE 9F is a graph showing that mutant *TP53* samples expressed higher levels of TP53 protein than did wild-type cases (P=0.0201), as determined by IHC.
FIGURE 9G is a Kaplan-Meier plot showing similar DFS profiles for TP53-high and -low cases from the overall bladder cancer cohort described in Example 1 (all tumors stages; P=0.4218; TP53 high N=53; TP53 low N=73). Hi, high; Lo, low.
FIGURE 9H is a Kaplan-Meier plot showing that in non-invasive tumors, high TP53 expression is associated with a trend towards a worse DFS probability (HR=1.99; P=0.1292).
FIGURE 9I is a Kaplan-Meier plot showing that in the non-invasive tumor setting, high TP53 expression is linked to significantly worse DFS likelihoods in BCG-treated patients (HR=4.2; P=0.0405).
FIGURE 9J is a Kaplan-Meier plot showing that high TP53 expression in non-invasive tumors is not associated with poor DFS probabilities for patients who did not receive treatment (HR=0.57; P=0.5749).

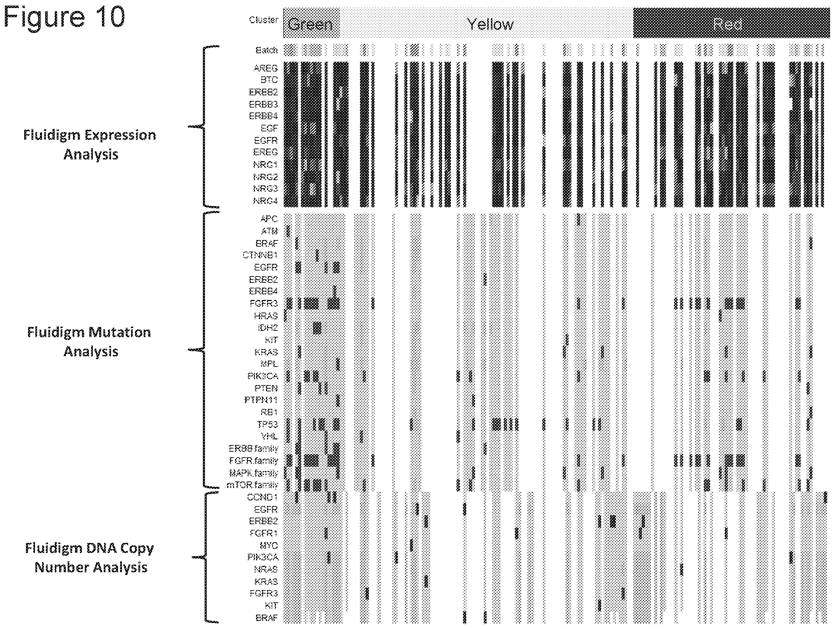

FIGURE 10 is a color map of Fluidigm analysis of ERBB pathway ligands and receptor expression in a subset of bladder cancer specimens, and overlapping mutation and copy number alterations in key pathways in the same samples. Expression analysis of ERBB receptors and ligand, mutational analysis, and copy number analyses were carried out on custom Fluidigm panels (Schleifman et al. PloS One 9: e90761, 2014). For Fluidigm mutation analysis: dark grey bar, mutant; medium grey bar; no call due to technical reasons; white bar, not applicable; light grey bar, wild-type. For Fluidigm DNA copy number analysis: dark grey bar, DNA copy number gain; medium grey bar, no call due to technical reasons; light grey bar, no DNA copy number change; white bar, not applicable.
FIGURE 11A is a graph showing that EGFR was expressed at significantly higher levels in the Green group compared to both the Red and Yellow groups, as determined by Fluidigm (P=0.012 and P=0.0012, respectively).
FIGURE 11B is a graph showing that in non-invasive bladder cancer tumors, high EGFR expression levels were associated with reduced rates of 3- and 5-year DFS (expression cutoff=25th percentile; high EGFR, N=22; low EGFR, N=66).
FIGURE 11C is a graph showing that high EGFR expression levels were associated with a reduced DFS rate for patients with bladder cancer metastases (expression cutoff=25th percentile; high EGFR, N=6; low EGFR, N=4).
FIGURE 11D is a graph showing that in patients with bladder cancer metastases, high EGFR expression levels were associated with lower 3- and 5-year OS rates (expression cutoff=50th percentile; high EGFR, N=3; low EGFR, N=7).
FIGURE 11E is a graph showing an analysis of an independent dataset from Sjödahl et al. Clin. Cancer Res. 18(12): 3377-3386, 2012. The graph shows that high EGFR expression were associated with worse 3- and 5-year OS frequencies (expression cutoff = 50th percentile; high EGFR, N=23; low EGFR, N=28).
FIGURE 11F is a graph showing an analysis of an independent dataset from Kim et al. Mol. Cancer 9: 3, 2010. The graph shows that high EGFR expression is associated with a lower rate of OS at 3 and 5 years (expression cutoff = 25th percentile; high EGFR N=48; low EGFR N=14).
FIGURE 11G is a graph showing that treatment of bladder cancer cell lines with the EGFR inhibitor erlotinib (TARCEVA™) identified three sensitive cell lines that exhibit >25% growth inhibition (GI) (UMUC5, UMUC10, and UMUC17), and four resistant cell lines that display <25% GI in response to treatment (RT112, UMUC3, SW780, and BFTC905).
FIGURE 11H is a graph showing that EGFR is expressed at significantly higher levels in bladder cancer cell lines are sensitive to erlotinib (TARCEVA™) (>25% GI) than in cell lines that exhibit minimal GI in response to treatment (P=0.0106).
FIGURE 12A is a Venn diagram showing genes comprising the custom bladder cancer Fluidigm gene expression panel described in Example 1 (e.g., Table 1) illustrating overlapping and unique genes belonging to three main pathway groups based on INGENUITY® analysis: (1) FGFR, RTK, MAPK, and PI3K pathways; (2) development and epithelial-to-mesenchymal transition (EMT) axes; and (3) TP53, genome stability, and cell cycle regulation networks. The numbers correspond to the number of unique genes in each portion of the Venn diagram.
FIGURE 12B is a graph showing the results of unsupervised hierarchical clustering of samples from a large public dataset and corresponding tumor grade, stage, and molecular class, as defined by Sjödahl et al., *supra.*

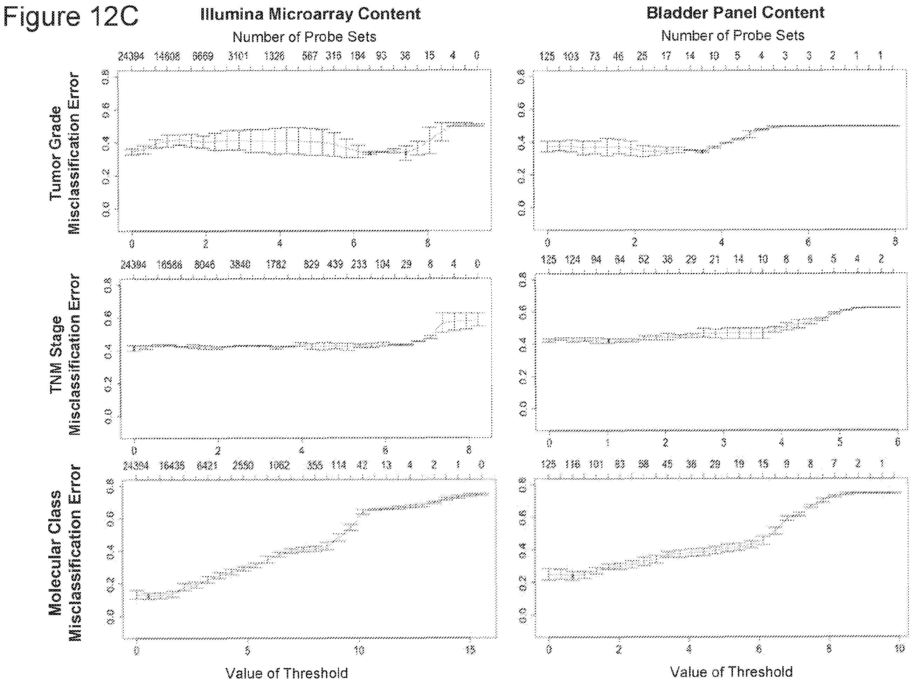

FIGURE 12C is a series of graphs showing cross-validated misclassification error curves for tumors from the Sjödahl et al. *(supra)* dataset based on decreasing numbers of Illumina probes compared to probes corresponding to the custom bladder cancer Fluidigm gene expression panel. Each graph shows the amount of classification error made while attempting to predict tumor grade, TNM stage, or molecular class using a nearest shrunken centroid classifier with a subset of the total genes. The number of genes is shown along the top x-axis with the corresponding "shrinkage factor" shown on the bottom x-axis.
FIGURE 12D is a series of graphs showing unsupervised hierarchical clustering of samples from four public datasets based on the signals from probes corresponding to genes of the custom bladder cancer Fluidigm gene expression panel and corresponding published basal/luminal status (Damrauer et al. Proc. Natl. Acad. Sci. USA 111:3110-3115, 2014).
FIGURE 13A is a series of graphs showing the linear performance of six representative assays and their raw CT values with respect to increasing input amounts of universal RNA (uRNA) controls.
FIGURE 13B is a series of graphs showing run-to-run data reproducibility for archival FFPE tissues, as seen for two representative FFPE-derived RNA samples run on different days.
FIGURE 14A is a heatmap showing the results of unsupervised clustering of genes and samples from public data sets, as well as NMIBCs, MIBCs, and metastases (METs) from the bladder cancer FFPE tissue cohort described in Example 1 and Figure 1. White blocks represent genes that are not found in the respective data sets.
FIGURE 14B is a graph showing basal profile (red bars) and luminal profile (blue bars) for bladder cancer panel genes calculated from the Damrauer et al. *(supra)* discovery samples. Mean-centering and unit variance normalization was applied to the log-transformed expression values, and mean log normalized expression levels were calculated independently for basal and luminal sample groups to form the final profiles.
FIGURE 14C is a diagram showing methodology used for computing basal/luminal signatures from public data and then applying these signatures to bladder panel data to measure basal/luminal similarity of samples.
FIGURE 15A shows the results of unsupervised hierarchical clustering (average-linkage, 1 - Pearson correlation distance metric) of 204 FFPE samples (see Example 1 and Figure 1) based on bladder cancer panel gene expression and corresponding B/L scores, histology, and mutational status of cancer-relevant genes. See Example 6 for additional details.
FIGURES 15B-15E are graphs showing statistical analysis of the B/L scores (Figure 15B), distribution of NMIBCs, MIBCs, and METs (Figure 15C), prevalence of FGFR3 mutations (Figure 15D), and FGFR3 IHC scores (Figure 15E) in samples from the transcriptionally-defined luminal and basal clusters in Figure 15A.
FIGURE 15F shows INGENUITY® pathway activation scores based on the expression of genes that were significantly differentially expressed between luminal and basal samples in Figure 15A.
FIGURE 16A is a graph showing a correlation plot between the B/L scores in primary tumors (X-axis) and corresponding B/L scores in matched metastases (Y-axis) from the same patients. Dot color reflects the significance of samples being matched to the same patient based on SNP genotyping. Log10 (p-value) = -4 (P=0.0001), Log10 (p-value) = -3 (P=0.001), and Log10 (p-value) of -2 (P=0.01). Dotted lines represent the 95% confidence interval beyond which metastases and primary sample B/L scores are different at a p-value of 0.05.
FIGURE 16B is a table showing the B/L score of primary tumors ("pri"), matched metastases ("met"), and corresponding p-values for divergence in B/L status in the matched pairs.
FIGURES 17A and 17B are diagrams depicting the results of INGENUITY® analysis showing predictive activation of the estrogen receptor in luminal compared to basal samples based on genes significantly differentially expressed between the two groups in FFPE tissues.
FIGURE 18 is a Pearson correlation matrix using next generation sequencing (NGS) AMPLISEQ™ data comparing the allele frequency at all variant sites with at least 100x read coverage and frequency > 10% (on average, 120 sites were compared between any two samples).

### DETAILED DESCRIPTION OF EMBODIMENTS OF THE INVENTION

### I. Introduction

The present invention provides therapeutic, diagnostic, and prognostic methods and compositions for cancer, for example, bladder cancer. The invention is based on the discovery that determination of expression levels (e.g., a changed expression level relative to a reference sample) of at least one of the genes set forth in Table 1, including *FGFR3, TP53,* and/or *EGFR,* is useful for treating a patient suffering from a cancer, for diagnosing a patient suffering from a cancer, for determining a prognosis of a patient suffering from a cancer, for determining whether a patient having a cancer is likely to respond to treatment with an anti-cancer therapy, and/or for optimizing therapeutic efficacy of an anti-cancer therapy for a patient having a cancer. In some embodiments, an anti-cancer therapy can then be selected for the patient and, further, an anti-cancer therapy can optionally be administered to the patient.

### II. Definitions

The terms "expression level," "level of expression," or "level" are used interchangeably and generally refer to the amount of a polynucleotide (e.g., a messenger RNA (mRNA)) or an amino acid product or protein in a biological sample. "Expression" generally refers to the process by which gene-encoded information is converted into the structures present and operating in the cell. Therefore, according to the invention, "expression" of a gene may refer to transcription into a polynucleotide (e.g., an mRNA), translation into a polypeptide (e.g., a protein), or even post-translational modification of the polypeptide. Fragments of the transcribed polynucleotide, the translated polypeptide, or the post-translationally modified polypeptide shall also be regarded as expressed whether they originate from a transcript generated by alternative splicing or a degraded transcript, or from a post-translational processing of the protein, e.g., by proteolysis. "Expressed genes" include those that are transcribed into a polynucleotide as mRNA and then translated into a protein, and also those that are transcribed into RNA but not translated into a protein (e.g., transfer and ribosomal RNAs).

The terms "marker" and "biomarker" are used interchangeably herein to refer to a DNA, RNA, protein, carbohydrate, or glycolipid-based molecular marker, the expression or presence of which in a subject's or patient's sample can be detected by standard methods (or methods disclosed herein) and is useful, for example, for diagnosing a bladder cancer in a patient, for prognosis of a patient suffering from bladder cancer, determining whether a patient having a bladder cancer is likely to respond to treatment with an anti-cancer therapy, optimizing therapeutic efficacy of an anti-cancer patient having a bladder cancer, and/or in therapeutic methods that involve determining the expression level of such a marker. Such biomarkers include, but are not limited to, the genes set forth in Table 1. In some embodiments, the gene may be one or more of *FGFR3, TP53,* and *EGFR.* In some embodiments, the gene is *FGFR3.* In some embodiments, the gene is *TP53.* In some embodiments, the gene is *EGFR.* In other embodiments, the gene(s) may be selected from *DUSB3*, *FRS2*, *TSC1*, *ERBB3*, *CDKN1A*, *CCND1*, *TP63*, *MMP2*, *ZEB2*, *PIK3CB*, *PIK3R1*, *MDM2*, *SNAI2*, *AXL*, *ZEB1*, *BCL2B*, *TSC2*, *RB1*, *FGFR32*, *PIK3IP1*, *MTOR*, *PIK3CA*, *PTEN*, *AKT1*, *BCL2A*, *FRS3*, *ERBB2*, *FGFR31*, *FGF1*, *SNAI1*, *FGFR34*, *FGF9*, or *FGF2.* Expression of such a biomarker may be determined to be higher or lower in a sample obtained from a patient than a reference level. Individuals having an expression level that is greater than or less than the reference expression level of at least one gene can be identified as subjects/patients who are suffering from a bladder cancer, as patients who have a particular prognosis (e.g., a favorable or poor prognosis), or as one who is likely to respond to treatment with an anti-cancer therapy.

In certain embodiments, the term "reference level" herein refers to a predetermined value. As the skilled artisan will appreciate, the reference level is predetermined and set to meet the requirements in terms of, for example, specificity and/or sensitivity. These requirements can vary, e.g., from regulatory body to regulatory body. It may be, for example, that assay sensitivity or specificity, respectively, has to be set to certain limits, e.g., 80%, 90%, 95% or 99%. These requirements may also be defined in terms of positive or negative predictive values. Nonetheless, based on the teaching given in the present invention it will always be possible to arrive at the reference level meeting those requirements. In one embodiment, the reference level is determined in healthy individuals. The reference level in one embodiment has been predetermined in the disease entity to which the patient belongs (e.g., a cancer type, such as bladder cancer, or a subtype of a bladder cancer). In certain embodiments, the reference level can be set to any percentile between the 20^{th} and 95^{th} percentile (e.g., the 20^{th}, 25^{th}, 30^{th}, 35^{th}, 40^{th}, 45^{th}, 50^{th}, 55^{th}, 60^{th}, 65^{th}, 70^{th}, 75^{th}, 80^{th}, 85^{th}, 90^{th}, or 95^{th} percentile) of the overall distribution of the values in a disease entity investigated. In particular embodiments, the reference level is set to the 25^{th} percentile of the overall distribution of the values in a disease entity investigated. In other particular embodiments, the reference level is set to the 50^{th} percentile of the overall distribution of the values in a disease entity investigated. In other embodiments, the reference level can be set to, for example, the median, tertiles, quartiles, or quintiles as determined from the overall distribution of the values in a disease entity investigated or in a given population. In other embodiments, the reference level can be set to, for example, the mean, as determined from the overall distribution of the values in a disease entity investigated or in a given population.

In certain embodiments, the term "increase" or "above" refers to a level at the reference level or to an overall increase of 5%, 10%, 20%, 25%, 30%, 40%, 50%, 60%, 70%, 80%, 85%, 90%, 95%, 100%, 2-fold, 3-fold, 4-fold, 5-fold, 6-fold, 7-fold, 8-fold, 9-fold, 10-fold, 11-fold, 12-fold, 13-fold, 14-fold, 15-fold, or greater, in the level of a marker (e.g., *FGFR3, TP53,* or *EGFR)* detected by the methods described herein, as compared to the level from a reference sample.

In certain embodiments, the term "decrease" or "below" herein refers to a level below the reference level or to an overall reduction of 5%, 10%, 20%, 25%, 30%, 40%, 50%, 60%, 70%, 80%, 85%, 90%, 95%, 96%, 97%, 98%, 99% or greater, in the level of a marker (e.g., *FGFR3, TP53,* or *EGFR)* detected by the methods described herein, as compared to the level from a reference sample.

In certain embodiments, the term "at a reference level" refers to a level of a marker (e.g., *FGFR3, TP53,* and/or *EGFR)* that is the same as the level, detected by the methods described herein, from a reference sample.

The term "diagnosis" is used herein to refer to the identification or classification of a molecular or pathological state, disease or condition (e.g., cancer, including bladder cancer). For example, "diagnosis" may refer to identification of a particular type of cancer. "Diagnosis" may also refer to the classification of a particular subtype of cancer, e.g., by histopathological criteria, or by molecular features (e.g., a subtype characterized by expression of one or a combination of biomarkers (e.g., particular genes or proteins encoded by said genes)). In some embodiments, a subtype of cancer may be a non-muscle-invasive bladder cancer (NMIBC; T0, T1), a muscle-invasive bladder cancer (MIBC; T2, T3) or a metastatic bladder cancer.

A "response" of a patient or a patient's "responsiveness" to treatment or therapy, for example treatment comprising an effective amount of an anti-cancer therapy (e.g., an anti-cancer therapy comprising an FGFR3 antagonist, a TP53 antagonist, and/or a EGFR antagonist), refers to the clinical or therapeutic benefit imparted to a patient at risk for or having a cancer (e.g., a bladder cancer) from or as a result of the treatment. Such benefit may include cellular or biological responses, a complete response, a partial response, a stable disease (without progression or relapse), or a response with a later relapse of the patient from or as a result of the treatment with the antagonist. A skilled person will readily be in position to determine whether a patient is responsive. For example, with respect to bladder cancer, a response may be reflected by decreased suffering from bladder cancer, such as a diminished and/or halted tumor growth, reduction of the size of a tumor, and/or amelioration of one or more symptoms of bladder cancer, for example, blood in urine (hematuria), changes in urination, other urinary symptoms, back pain, or pelvic pain. In some embodiments, the response may be reflected by decreased or diminished indices of the metastatic conversion of the cancer or indices of the cancer, e.g., the prevention of the formation of metastases or a reduction of number or size of metastases. For example, a response can be reduced tumor size, disease-free survival, progression-free survival, or overall survival in a patient diagnosed as expressing increased or decreased levels of one or more of the biomarkers set forth in Table 1 (e.g., *FGFR3, TP53,* and *EGFR)* relative to a reference level.

The terms "sample" and "biological sample" are used interchangeably to refer to any biological sample obtained from an individual including body fluids, body tissue (e.g., tumor tissue), cells, or other sources. Body fluids are, for example, lymph, sera, whole fresh blood, peripheral blood mononuclear cells, frozen whole blood, plasma (including fresh or frozen), urine, saliva, semen, synovial fluid and spinal fluid. Samples also include breast tissue, renal tissue, colonic tissue, brain tissue, muscle tissue, synovial tissue, skin, hair follicle, bone marrow, and tumor tissue. Methods for obtaining tissue biopsies and body fluids from mammals are well known in the art.

A "tumor sample" herein is a sample derived from, or comprising tumor cells from, a patient's tumor. Examples of tumor samples herein include, but are not limited to, tumor biopsies, circulating tumor cells, circulating plasma proteins, ascitic fluid, primary cell cultures or cell lines derived from tumors or exhibiting tumor-like properties, as well as preserved tumor samples, such as formalin-fixed, paraffin-embedded tumor samples or frozen tumor samples.

An "antagonist" (interchangeably termed "inhibitor") of a polypeptide of interest is an agent that interferes with activation or function of the polypeptide of interest, e.g., partially or fully blocks, inhibits, or neutralizes a biological activity mediated by a polypeptide of interest. For example, an antagonist of polypeptide X refers to any molecule that partially or fully blocks, inhibits, or neutralizes a biological activity mediated by polypeptide X. Examples of inhibitors include antibodies; ligand antibodies; small molecule antagonists; antisense and inhibitory RNA (e.g., shRNA) molecules. Preferably, the inhibitor is an antibody or small molecule which binds to the polypeptide of interest. In a particular embodiment, an inhibitor has a binding affinity (dissociation constant) to the polypeptide of interest of about 1,000 nM or less. In another embodiment, an inhibitor has a binding affinity to the polypeptide of interest of about 100 nM or less. In another embodiment, an inhibitor has a binding affinity to the polypeptide of interest of about 50 nM or less. In a particular embodiment, an inhibitor is covalently bound to the polypeptide of interest. In a particular embodiment, an inhibitor inhibits signaling of the polypeptide of interest with a half maximal inhibitory concentration (IC50) of 1,000 nM or less. In another embodiment, an inhibitor inhibits signaling of the polypeptide of interest with an IC50 of 500 nM or less. In another embodiment, an inhibitor inhibits signaling of the polypeptide of interest with an IC50 of 50 nM or less. In certain embodiments, the antagonist reduces or inhibits, by at least 10%, 20%, 30%, 40%, 50%, 60%, 70%, 80%, 90%, 95%, 99%, or more, the expression level or biological activity of the polypeptide of interest. In some embodiments, the polypeptide of interest is FGFR3 or a ligand of FGFR3. In some embodiments, the polypeptide of interest is EGFR or a ligand of FGFR3. In some embodiments, the polypeptide of interest is TP53.

The term "polypeptide" as used herein, refers to any native polypeptide (e.g., protein) of interest from any vertebrate source, including mammals such as primates (e.g., humans) and rodents (e.g., mice and rats), unless otherwise indicated. The term encompasses "full-length," unprocessed polypeptide as well as any form of the polypeptide that results from processing in the cell. The term also encompasses naturally occurring variants of the polypeptide, e.g., splice variants or allelic variants. In some embodiments, the polypeptide is FGFR3. In other embodiments, the polypeptide is TP53. In other embodiments, the polypeptide is EGFR.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR2 ("amidate"), P(O)R, P(O)OR', CO or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

The term "small molecule" refers to any molecule with a molecular weight of about 2000 daltons or less, preferably of about 500 daltons or less.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

The terms "anti-polypeptide of interest antibody" and "an antibody that binds to" a polypeptide of interest refer to an antibody that is capable of binding a polypeptide of interest with sufficient affinity such that the antibody is useful as a diagnostic and/or therapeutic agent in targeting a polypeptide of interest. In one embodiment, the extent of binding of an anti-polypeptide of interest antibody to an unrelated, non-polypeptide of interest protein is less than about 10% of the binding of the antibody to a polypeptide of interest as measured, e.g., by a radioimmunoassay (RIA). In certain embodiments, an antibody that binds to a polypeptide of interest has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g., 10⁻⁸ M or less, e.g., from 10⁻⁸ M to 10⁻¹³ M, e.g., from 10⁻⁹ M to 10⁻¹³ M). In certain embodiments, an anti-polypeptide of interest antibody binds to an epitope of a polypeptide of interest that is conserved among polypeptides of interest from different species. In some embodiments, the polypeptide of interest is FGFR3. In some embodiments, the polypeptide of interest is EGFR. In some embodiments, the polypeptide of interest is TP53.

A "blocking antibody" or an "antagonist antibody" is one which inhibits or reduces biological activity of the antigen it binds. Preferred blocking antibodies or antagonist antibodies substantially or completely inhibit the biological activity of the antigen.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein.

An "antibody fragment" refers to a molecule other than an intact antibody that comprises a portion of an intact antibody that binds the antigen to which the intact antibody binds. A "functional fragment" is an antibody fragment that maintains a function of the full-length antibody. Examples of antibody fragments include but are not limited to Fv, Fab, Fab', Fab'-SH, F(ab')2; diabodies; linear antibodies; single-chain antibody molecules (e.g., scFv); and multispecific antibodies formed from antibody fragments.

The term "chimeric" antibody refers to an antibody in which a portion of the heavy and/or light chain is derived from a particular source or species, while the remainder of the heavy and/or light chain is derived from a different source or species.

The terms "full length antibody," "intact antibody," and "whole antibody" are used herein interchangeably to refer to an antibody having a structure substantially similar to a native antibody structure or having heavy chains that contain an Fc region.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human hypervariable regions (HVRs) and amino acid residues from human framework regions (FRs). In certain embodiments, a humanized antibody will comprise substantially all of at least one, and typically two, variable domains, in which all or substantially all of the HVRs (e.g., complementarity determining regions (CDRs)) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

An "immunoconjugate" is an antibody conjugated to one or more heterologous molecule(s), including but not limited to a cytotoxic agent.

The terms "fibroblast growth factor receptor 3" and "FGFR3" as used herein, refers to any native FGFR3 from any vertebrate source, including mammals such as primates (e.g., humans) and rodents (e.g., mice and rats), unless otherwise indicated. The term encompasses "full-length," unprocessed FGFR3 as well as any form of FGFR3 that results from processing in the cell. The term also encompasses naturally occurring variants of the FGFR3, e.g., splice variants or allelic variants. An exemplary wild-type sequence of human FGFR3 polypeptide comprises the amino acid sequence from UniProt database of P22607-1 or P22607-2.

As used herein, the term "FGFR3 antagonist" and "FGFR3 inhibitor" refers to any FGFR3 antagonist that is currently known in the art or that will be identified in the future, and includes any chemical entity that, upon administration to a patient, results in inhibition of a biological activity associated with activation of FGFR3 in the patient, including any of the downstream biological effects otherwise resulting from the binding to FGFR3 of its natural ligand. Such FGFR3 antagonists include any agent that can block FGFR3 activation or any of the downstream biological effects of FGFR3 activation that are relevant to treating cancer in a patient. Such an antagonist can act by binding directly to the intracellular domain of the receptor and inhibiting its kinase activity. Alternatively, such an antagonist can act by occupying the ligand binding site or a portion thereof of the FGFR3 receptor, thereby making the receptor inaccessible to its natural ligand so that its normal biological activity is prevented or reduced. Alternatively, such an antagonist can act by modulating the dimerization of FGFR3 polypeptides, or interaction of FGFR3 polypeptide with other proteins, or enhance ubiquitination and endocytotic degradation of FGFR3. FGFR3 antagonists include but are not limited to small molecule inhibitors, antibodies or antibody fragments, antisense constructs, small inhibitory RNAs (i.e., RNA interference by dsRNA; RNAi), and ribozymes. In a preferred embodiment, the FGFR3 antagonist is a small molecule or an antibody that binds specifically to human FGFR3. Exemplary FGFR3 antagonist antibodies are described, for example, in U.S. Patent No. 8,410,250. For example, U.S. Patent No. 8,410,250 describes the FGFR3 antagonist antibody clones 184.6, 184.6.1, and 184.6.1N54S (these clones are also referred to as "R3 Mab").

The terms "epidermal growth factor receptor (EGFR)," "ErbB1," "HER1," and "EGFR kinase" are used interchangeably herein and refer to EGFR as disclosed, for example, in Carpenter et al. Ann. Rev. Biochem. 56:881-914 (1987), including naturally occurring mutant forms thereof (e.g., a deletion mutant EGFR as in Humphrey et al. PNAS (USA) 87:4207-4211 (1990)). *EGFR* or *ERBB1* refers to the gene encoding the EGFR protein product. The amino acid sequence of an exemplary human EGFR protein may be found, for example, under UniProt Accession Number P00533.

As used herein, the term "EGFR antagonist" and "EGFR inhibitor" refers to any EGFR antagonist that is currently known in the art or that will be identified in the future, and includes any chemical entity that, upon administration to a patient, results in inhibition of a biological activity associated with activation of the EGF receptor in the patient, including any of the downstream biological effects otherwise resulting from the binding to EGFR of its natural ligand. Such EGFR antagonists include any agent that can block EGFR activation or any of the downstream biological effects of EGFR activation that are relevant to treating cancer in a patient. Such an antagonist can act by binding directly to the intracellular domain of the receptor and inhibiting its kinase activity. Alternatively, such an antagonist can act by occupying the ligand binding site or a portion thereof of the EGF receptor, thereby making the receptor inaccessible to its natural ligand so that its normal biological activity is prevented or reduced. Alternatively, such an antagonist can act by modulating the dimerization of EGFR polypeptides, or interaction of EGFR polypeptide with other proteins, or enhance ubiquitination and endocytotic degradation of EGFR. EGFR antagonists include but are not limited to small molecule inhibitors, antibodies or antibody fragments, antisense constructs, small inhibitory RNAs (i.e., RNA interference by dsRNA; RNAi), and ribozymes. In a preferred embodiment, the EGFR antagonist is a small molecule or an antibody that binds specifically to human EGFR.

Exemplary EGFR antagonists suitable for use in the invention include, for example, small molecule EGFR antagonists including quinazoline EGFR kinase inhibitors, pyrido-pyrimidine EGFR kinase inhibitors, pyrimido-pyrimidine EGFR kinase inhibitors, pyrrolo-pyrimidine EGFR kinase inhibitors, pyrazolo-pyrimidine EGFR kinase inhibitors, phenylamino-pyrimidine EGFR kinase inhibitors, oxindole EGFR kinase inhibitors, indolocarbazole EGFR kinase inhibitors, phthalazine EGFR kinase inhibitors, isoflavone EGFR kinase inhibitors, quinalone EGFR kinase inhibitors, and tyrphostin EGFR kinase inhibitors, such as those described in the following patent publications, and all pharmaceutically acceptable salts and solvates of the EGFR kinase inhibitors: International Patent Publication Nos. WO 96/33980, WO 96/30347, WO 97/30034, WO 97/30044, WO 97/38994, WO 97/49688, WO 98/02434, WO 97/38983, WO 95/19774, WO 95/19970, WO 97/13771, WO 98/02437, WO 98/02438, WO 97/32881, WO 98/33798, WO 97/32880, WO 97/3288, WO 97/02266, WO 97/27199, WO 98/07726, WO 97/34895, WO 96/31510, WO 98/14449, WO 98/14450, WO 98/14451, WO 95/09847, WO 97/19065, WO 98/17662, WO 99/35146, WO 99/35132, WO 99/07701, and WO 92/20642; European Patent Application Nos. EP 520722, EP 566226, EP 787772, EP 837063, and EP 682027; U.S. Patent Nos. 5,747,498, 5,789,427, 5,650,415, and 5,656,643; and German Patent Application No. DE 19629652. Additional non-limiting examples of small molecule EGFR antagonists include any of the EGFR kinase inhibitors described in Traxler, Exp. Opin. Ther. Patents 8(12):1599-1625 (1998).

Specific preferred examples of small molecule EGFR antagonists that can be used according to the present invention include [6,7-bis(2-methoxyethoxy)-4-quinazolin-4-yl]-(3-ethynylphenyl) amine (also known as OSI-774, erlotinib, or TARCEVA™ (erlotinib HCl); OSI Pharmaceuticals/Genentech/Roche) (U.S. Pat. No. 5,747,498; International Patent Publication No. WO 01/34574, and Moyer et al. Cancer Res. 57:4838-4848 (1997)); CI-1033 (formerly known as PD183805; Pfizer) (Sherwood et al., Proc. Am. Assoc. Cancer Res. 40:723 (1999)); PD-158780 (Pfizer); AG-1478 (University of California); CGP-59326 (Novartis); PKI-166 (Novartis); EKB-569 (Wyeth); GW-2016 (also known as GW-572016 or lapatinib ditosylate ; GSK); and gefitinib (also known as ZD1839 or IRESSA™; Astrazeneca) (Woodburn et al., Proc. Am. Assoc. Cancer Res. 38:633 (1997)). A particularly preferred small molecule EGFR kinase inhibitor that can be used according to the present invention is [6,7-bis(2-methoxyethoxy)-4-quinazolin-4-yl]-(3-ethynylphenyl) amine (i.e., erlotinib), its hydrochloride salt (i.e., erlotinib HCl, TARCEVA™), or other salt forms (e.g., erlotinib mesylate).

Exemplary EGFR antagonist antibodies include those described in Modjtahedi, et al., Br. J. Cancer 67:247-253 (1993); Teramoto et al., Cancer 77:639-645 (1996); Goldstein et al., Clin. Cancer Res. 1:1311-1318 (1995); Huang, et al., Cancer Res. 15:59(8):1935-40 (1999); and Yang et al., Cancer Res. 59:1236-1243 (1999). Thus, in some embodiments the EGFR antagonist antibody can be the monoclonal antibody Mab E7.6.3 (Yang et al. Cancer Res. 59:1236-43 (1999)), or Mab C225 (ATCC Accession No. HB-8508), or an antibody or antibody fragment having the binding specificity thereof. Suitable monoclonal EGFR antagonist antibodies include, but are not limited to, IMC-C225 (also known as cetuximab or ERBITUX™; Imclone Systems), ABX-EGF (Abgenix), EMD 72000 (Merck KgaA, Darmstadt), RH3 (York Medical Bioscience Inc.), and MDX-447 (Medarex/ Merck KgaA).

The terms *"TP53,"* "cellular tumor antigen p53," and "p53," used interchangeably herein, refer to a potent tumor suppressor protein encoding a 393 amino acid phosphoprotein. TP53 is negatively regulated or mutated in many cancers. Absence or inactivation of TP53 may contribute to cancer. A wide variety of TP53 mutations exist. In some cases, a cancer may overexpress TP53, in particular, mutant versions of TP53. A "wild type" TP53 is TP53 found in normal (i.e., non-cancerous cells) or TP53 that does not have a mutation correlated to a cancer. The TP53 status of a sample (e.g., whether the sample includes wild-type or mutant TP53) may be assessed as for example described in U.S. Pat. No. 6,090,566 issued to Vogelstein et al., or using standard techniques such as described herein or known in the art. A TP53 molecule may include, without limitation, polypeptides containing sequences substantially identical to that set forth in for example UniProt Accession No. P04637 and nucleic acid molecules encoded by those sequences.

A "tyrosine kinase inhibitor" is an antagonist molecule which inhibits to some extent tyrosine kinase activity of a tyrosine kinase such as an EGFR receptor or an FGFR3 receptor.

"Bacillus Calmette-Guerin (BCG) vaccine" refers to a vaccine used to prevent tuberculosis (TB) in people who are at a high risk of TB or where TB is common. It is made from a weakened form of a bacterium called *Mycobacterium bovis* (bacillus Calmette-Guerin), which is similar to the bacteria that cause TB. The vaccine may help the body's immune system make antibodies to destroy the TB bacteria. It also may help the immune system kill cancer cells, and is used, for example, as a treatment in bladder cancer.

The terms "cancer" and "cancerous" refer to or describe the physiological condition in mammals that is typically characterized by unregulated cell growth. Included in this definition are benign and malignant cancers. By "early stage cancer" or "early stage tumor" is meant a cancer that is not invasive or metastatic or is classified as a Stage 0, I, or II cancer. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include bladder cancer, squamous cell cancer (e.g., epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer and multiple myeloma.

The term "pre-cancerous" refers to a condition or a growth that typically precedes or develops into a cancer. A "pre-cancerous" growth will have cells that are characterized by abnormal cell cycle regulation, proliferation, or differentiation, which can be determined by markers of cell cycle regulation, cellular proliferation, or differentiation.

"Tumor," as used herein, refers to all neoplastic cell growth and proliferation, whether malignant or benign, and all pre-cancerous and cancerous cells and tissues. The terms "cancer," "cancerous," "cell proliferative disorder," and "tumor" are not mutually exclusive as referred to herein.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

By "non-metastatic" is meant a cancer that is benign or that remains at the primary site and has not penetrated into the lymphatic or blood vessel system or to tissues other than the primary site. Generally, a non-metastatic cancer is any cancer that is a Stage 0, I, or II cancer, and occasionally a Stage III cancer.

The term "anti-cancer therapy" refers to a therapy useful in treating cancer. Examples of anti-cancer therapeutic agents include, but are not limited to, antagonists, chemotherapeutic agents, growth inhibitory agents, cytotoxic agents, agents used in radiation therapy, anti-angiogenesis agents, apoptotic agents, anti-tubulin agents, and other agents to treat cancer, anti-CD20 antibodies, platelet derived growth factor inhibitors (e.g., GLEEVEC™ (Imatinib Mesylate)), a COX-2 inhibitor (e.g., celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. In particular embodiments, an antagonist is an FGFR3 antagonist, a TP53 antagonist, or an EGFR antagonist. Combinations thereof are also included in the invention.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

As used herein, "treatment," "treating," or other grammatical variations thereof refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, an anti-cancer therapy (e.g., an anti-cancer therapy including an FGFR3 antagonist, a TP53 antagonist, and/or an EGFR antagonist) is used to delay development of a disease or disorder.

An "effective amount" refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. The term "therapeutically effective amount" refers to an amount of an antibody, polypeptide or antagonist of this invention effective to "treat" a disease or disorder in a mammal (e.g., a patient). In the case of cancer, the therapeutically effective amount of the drug can reduce the number of cancer cells; reduce the tumor size or weight; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug can prevent growth and/or kill existing cancer cells, it can be cytostatic and/or cytotoxic. In one embodiment, the therapeutically effective amount is a growth inhibitory amount. For cancer therapy, efficacy *in vivo* can, for example, be measured by assessing the duration of survival, time to disease progression (TTP), duration of disease free survival (DFS), duration of progression free survival (PFS), the response rates (RR), duration of response, and/or quality of life.

The term "survival" refers to the patient remaining alive, and includes overall survival as well as disease free survival.

The term "overall survival" refers to the patient remaining alive for a defined period of time, such as 1 year, 5 years, etc. from the time of diagnosis or treatment.

The phrase "progression-free survival" in the context of the present invention refers to the length of time during and after treatment during which, according to the assessment of the treating physician or investigator, a patient's disease does not become worse, i.e., does not progress. As the skilled person will appreciate, a patient's progression-free survival is improved or enhanced if the patient experiences a longer length of time during which the disease does not progress as compared to the average or mean progression free survival time of a control group of similarly situated patients.

The phrase "disease-free survival (DFS)" refers to the length of time after primary treatment for a cancer ends that the patient survives without any signs or symptoms of that cancer.

By "extending survival" is meant increasing survival, for example, overall or disease-free survival, in a treated patient relative to an untreated patient (i.e., relative to a patient not treated with an anti-cancer therapy (e.g., an anti-cancer therapy including a FGFR3 antagonist, a TP53 antagonist, and/or an EGFR antagonist), or relative to a patient who does not express *FGFR3*, *TP53*, and/or *EGFR* at a designated reference level.

The term "cytotoxic agent" as used herein refers to a substance that inhibits or prevents the function of cells and/or causes destruction of cells. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1l and calicheamicin omegal1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE® docetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin. Additional chemotherapeutic agents include the cytotoxic agents useful as antibody drug conjugates, such as maytansinoids (DM1, for example) and the auristatins MMAE and MMAF, for example.

"Chemotherapeutic agents" also include "anti-hormonal agents" that act to regulate, reduce, block, or inhibit the effects of hormones that can promote the growth of cancer, and are often in the form of systemic, or whole-body treatment. They may be hormones themselves. Examples include antiestrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGFR); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth and/or proliferation of a cell (e.g., a bladder cancer cell) either *in vitro* or *in vivo.* Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as the anthracycline antibiotic doxorubicin ((8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione), epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in *"*The Molecular Basis of Cancer," Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

As used herein, the terms "patient" or "subject" are used interchangeably and refer to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

As used herein, "administering" is meant a method of giving a dosage of a compound (e.g., an antagonist) or a pharmaceutical composition (e.g., a pharmaceutical composition including an antagonist) to a subject (e.g., a patient). Administering can be by any suitable means, including parenteral, intrapulmonary, and intranasal, and, if desired for local treatment, intralesional administration. Parenteral infusions include, for example, intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

The term "effective amount" refers to an amount of a medicament that is effective for treating a disorder, for example, for treating a cancer.

The term "pharmaceutical formulation" refers to a sterile preparation that is in such form as to permit the biological activity of the medicament to be effective, and which contains no additional components that are unacceptably toxic to a subject to which the formulation would be administered.

A "sterile" formulation is aseptic or free from all living microorganisms and their spores.

A "package insert" is used to refer to instructions customarily included in commercial packages of therapeutic products or medicaments, that contain information about the indications, usage, dosage, administration, contraindications, other therapeutic products to be combined with the packaged product, and/or warnings concerning the use of such therapeutic products or medicaments, etc.

A "kit" is any manufacture (e.g., a package or container) comprising at least one reagent, e.g., a medicament for treatment of a cancer (e.g., a bladder cancer), or a probe for specifically detecting a biomarker gene or protein of the invention. The manufacture is preferably promoted, distributed, or sold as a unit for performing the methods of the present invention.

The word "label" when used herein refers to a compound or composition that is conjugated or fused directly or indirectly to a reagent such as a nucleic acid probe or an antibody and facilitates detection of the reagent to which it is conjugated or fused. The label may itself be detectable (e.g., radioisotope labels or fluorescent labels) or, in the case of an enzymatic label, may catalyze chemical alteration of a substrate compound or composition which is detectable. The term is intended to encompass direct labeling of a probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

### III. Therapeutic Methods

The present invention provides methods for treating a patient suffering from cancer. The term cancer embraces a collection of proliferative disorders, including but not limited to pre-cancerous growths, benign tumors, and malignant tumors. Benign tumors remain localized at the site of origin and do not have the capacity to infiltrate, invade, or metastasize to distant sites. Malignant tumors will invade and damage other tissues around them. They can also gain the ability to break off from the original site and spread to other parts of the body (metastasize), usually through the bloodstream or through the lymphatic system where the lymph nodes are located. Primary tumors are classified by the type of tissue from which they arise; metastatic tumors are classified by the tissue type from which the cancer cells are derived. Over time, the cells of a malignant tumor become more abnormal and appear less like normal cells. This change in the appearance of cancer cells is called the tumor grade, and cancer cells are described as being well-differentiated (low grade), moderately-differentiated, poorly-differentiated, or undifferentiated (high grade). Well-differentiated cells are quite normal appearing and resemble the normal cells from which they originated. Undifferentiated cells are cells that have become so abnormal that it is no longer possible to determine the origin of the cells. In particular embodiments, invention provides methods of treating bladder cancer.

Cancer staging systems describe how far the cancer has spread anatomically and attempt to put patients with similar prognosis and treatment in the same staging group. Several tests may be performed to help stage cancer including biopsy and certain imaging tests such as a chest x-ray, mammogram, bone scan, CT scan, and MRI scan. Blood tests and a clinical evaluation are also used to evaluate a patient's overall health and detect whether the cancer has spread to certain organs.

To stage cancer, the American Joint Committee on Cancer first places the cancer, particularly solid tumors, in a letter category using the TNM classification system. Cancers are designated the letter T (tumor size), N (palpable nodes), and/or M (metastases). T1, T2, T3, and T4 describe the increasing size of the primary lesion; N0, N1, N2, N3 indicates progressively advancing node involvement; and M0 and M1 reflect the absence or presence of distant metastases.

In the second staging method, also known as the Overall Stage Grouping or Roman Numeral Staging, cancers are divided into stages 0 to IV, incorporating the size of primary lesions as well as the presence of nodal spread and of distant metastases. In this system, cases are grouped into four stages denoted by Roman numerals I through IV, or are classified as "recurrent." For some cancers, stage 0 is referred to as "in situ" or "Tis," such as ductal carcinoma in situ or lobular carcinoma in situ for breast cancers. High grade adenomas can also be classified as stage 0. In general, stage I cancers are small localized cancers that are usually curable, while stage IV usually represents inoperable or metastatic cancer. Stage II and III cancers are usually locally advanced and/or exhibit involvement of local lymph nodes. In general, the higher stage numbers indicate more extensive disease, including greater tumor size and/or spread of the cancer to nearby lymph nodes and/or organs adjacent to the primary tumor. These stages are defined precisely, but the definition is different for each kind of cancer and is known to the skilled artisan.

Many cancer registries, such as the NCI's Surveillance, Epidemiology, and End Results Program (SEER), use summary staging. This system is used for all types of cancer. It groups cancer cases into five main categories:
*In situ* is early cancer that is present only in the layer of cells in which it began.

*Localized* is cancer that is limited to the organ in which it began, without evidence of spread.

*Regional* is cancer that has spread beyond the original (primary) site to nearby lymph nodes or organs and tissues.

*Distant* is cancer that has spread from the primary site to distant organs or distant lymph nodes.

*Unknown* is used to describe cases for which there is not enough information to indicate a stage.

In addition, it is common for cancer to return months or years after the primary tumor has been removed. Cancer that recurs after all visible tumor has been eradicated, is called recurrent disease. Disease that recurs in the area of the primary tumor is locally recurrent, and disease that recurs as metastases is referred to as a distant recurrence.

The tumor can be a solid tumor or a non-solid or soft tissue tumor, Examples of soft tissue tumors include leukemia (e.g., chronic myelogenous leukemia, acute myelogenous leukemia, adult acute lymphoblastic leukemia, acute myelogenous leukemia, mature B-cell acute lymphoblastic leukemia, chronic lymphocytic leukemia, polymphocytic leukemia, or hairy cell leukemia) or lymphoma (e.g., non-Hodgkin's lymphoma, cutaneous T-cell lymphoma, or Hodgkin's disease). A solid tumor includes any cancer of body tissues other than blood, bone marrow, or the lymphatic system. Solid tumors can be further divided into those of epithelial cell origin and those of non-epithelial cell origin. Examples of epithelial cell solid tumors include tumors of the bladder, gastrointestinal tract, colon, breast, prostate, lung, kidney, liver, pancreas, ovary, head and neck, oral cavity, stomach, duodenum, small intestine, large intestine, anus, gall bladder, labium, nasopharynx, skin, uterus, male genital organ, urinary organs, and skin. Solid tumors of non-epithelial origin include sarcomas, brain tumors, and bone tumors. Other examples of tumors are described in the Definitions section.

In some embodiments, the methods of the invention include administering to the patient a therapeutically effective amount of an anti-cancer therapy, wherein the expression level at least one of the biomarkers of the invention (for example, a biomarker listed in Table 1) in a sample obtained from the patient has been determined to be changed relative to a reference level of the biomarker of the invention. For example, in some embodiments, the invention provides a method of treating a cancer (e.g., bladder cancer) that involves administering to the patient a therapeutically effective amount of an anti-cancer therapy, wherein the expression level of at least one gene listed in Table 1 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 genes listed in Table 1) has been determined to be changed relative to a reference level of the at least one gene.

In another example, in some embodiments, the invention provides a method of treating a cancer (e.g., bladder cancer) that involves administering to the patient a therapeutically effective amount of an anti-cancer therapy, wherein the expression level of at least one gene listed in Table 4 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes listed in Table 4) has been determined to be changed relative to a reference level of the at least one gene. In some embodiments, the change is an increase. In other embodiments, the change is a decrease.

In particular embodiments, the methods of the invention include administering to the patient a therapeutically effective amount of an anti-cancer therapy, wherein the expression level of at least one of the following genes: *FGFR3, TP53,* and/or *EGFR* in a sample obtained from the patient has been determined to be increased relative to a reference level of the at least one gene. The expression level of biomarkers of the invention (e.g., a gene listed in Table 1, for example, *FGFR3, TP53,* and/or *EGFR)* can be determined using any of the methods or assays described herein (e.g., methods or assays described in Section IV of the Detailed Description of the Invention or in the Examples). In some embodiments, the bladder cancer is NMIBC. In other embodiments, the bladder cancer is MIBC. In yet other embodiments, the bladder cancer is metastatic bladder cancer. The patient may optionally have an advanced, refractory, recurrent, and/or chemotherapy-resistant form of the cancer. For example, in some embodiments, a patient may have a recurrent bladder cancer, for example, a recurrent NMIBC.

In any of the preceding methods of the invention, the expression level of a biomarker of the invention (e.g., a gene listed in Table 1, for example, *FGFR3, TP53,* and/or *EGFR)* in a sample obtained from the patient may be changed at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of biomarker. For instance, in some embodiments, the expression level of a biomarker of the invention in a sample obtained from the patient may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of biomarker. In other embodiments, the expression level of a biomarker of the invention in a sample obtained from the patient may be decreased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of biomarker.

In particular embodiments, the expression level of at least one (e.g., 1, 2, or 3) of the following: *FGFR3*, *TP53*, and/or *EGFR* in a sample obtained from the patient may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of the at least one gene. For instance, the expression level of *FGFR3* may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of *FGFR3.* In another instance, the expression level of *TP53* may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of *TP53.* In yet another instance, the expression level of *EGFR* may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of *EGFR.*

In some embodiments, the reference level may be set to any percentile between, for example, the 20^{th} percentile and the 99^{th} percentile (e.g., the 20^{th}, 25^{th}, 30^{th}, 35^{th}, 40^{th}, 45^{th}, 50^{th}, 55^{th}, 60^{th}, 65^{th}, 70^{th}, 75^{th}, 80^{th}, 85^{th}, 90^{th}, 95^{th}, or 99^{th} percentile) of the overall distribution of the expression level of a biomarker (for example, *FGFR3*, *TP53*, or *EGFR*) in a given cancer type (e.g., bladder cancer). In particular embodiments, the reference level may be set to the 25^{th} percentile of the overall distribution of the values in a bladder cancer. In other particular embodiments, the reference level may be set to the 50^{th} percentile of the overall distribution of the values in a bladder cancer. In other embodiments, the reference level may be the median of the overall distribution of the values in a bladder cancer.

In some embodiments, the methods of the invention include administering to the patient an anti-cancer therapy that includes one or more (e.g., 1, 2, or 3) of the following: an FGFR3 antagonist, a TP53, and/or an EGFR antagonist. For example, in some embodiments, the methods of the invention include administering an anti-cancer therapy comprising an FGFR3 antagonist, a TP53 antagonist, or an EGFR antagonist. In other instances, the method may involve administering an anti-cancer therapy comprising an FGFR3 antagonist and an EGFR antagonist. In other instances, the method may involve administering an anti-cancer therapy comprising an FGFR3 antagonist, a TP53 antagonist, and an EGFR antagonist.

In any of the preceding methods, the FGFR3 antagonist may be an FGFR3 antagonist antibody or a small molecule FGFR3 antagonist. Exemplary FGFR3 antagonist antibodies, such as 184.6, 184.6.1, and 184.6.1N54S, are described, for example, in U.S. Patent No. 8,410,250. In some embodiments, the small molecule FGFR3 antagonist is a tyrosine kinase inhibitor.

In any of the preceding methods, the TP53 antagonist may be a TP53 antagonist antibody or a small molecule TP53 antagonist.

In any of the preceding methods, the EGFR antagonist may be an EGFR antagonist antibody or a small molecule EGFR antagonist. In some embodiments, the small molecule FGFR3 antagonist is a tyrosine kinase inhibitor. In particular embodiments, the small molecule FGFR3 antagonist is erlotinib (TARCEVA™).

Exemplary EGFR antagonists that can be used in the methods of the invention include, for example, small molecule EGFR antagonists including quinazoline EGFR kinase inhibitors, pyrido-pyrimidine EGFR kinase inhibitors, pyrimido-pyrimidine EGFR kinase inhibitors, pyrrolo-pyrimidine EGFR kinase inhibitors, pyrazolo-pyrimidine EGFR kinase inhibitors, phenylamino-pyrimidine EGFR kinase inhibitors, oxindole EGFR kinase inhibitors, indolocarbazole EGFR kinase inhibitors, phthalazine EGFR kinase inhibitors, isoflavone EGFR kinase inhibitors, quinalone EGFR kinase inhibitors, and tyrphostin EGFR kinase inhibitors, such as those described in the following patent publications, and all pharmaceutically acceptable salts and solvates of the EGFR kinase inhibitors: International Patent Publication Nos. WO 96/33980, WO 96/30347, WO 97/30034, WO 97/30044, WO 97/38994, WO 97/49688, WO 98/02434, WO 97/38983, WO 95/19774, WO 95/19970, WO 97/13771, WO 98/02437, WO 98/02438, WO 97/32881, WO 98/33798, WO 97/32880, WO 97/3288, WO 97/02266, WO 97/27199, WO 98/07726, WO 97/34895, WO 96/31510, WO 98/14449, WO 98/14450, WO 98/14451, WO 95/09847, WO 97/19065, WO 98/17662, WO 99/35146, WO 99/35132, WO 99/07701, and WO 92/20642; European Patent Application Nos. EP 520722, EP 566226, EP 787772, EP 837063, and EP 682027; U.S. Patent Nos. 5,747,498, 5,789,427, 5,650,415, and 5,656,643; and German Patent Application No. DE 19629652. Additional non-limiting examples of small molecule EGFR antagonists include any of the EGFR kinase inhibitors described in Traxler, Exp. Opin. Ther. Patents 8(12):1599-1625 (1998).

Specific preferred examples of small molecule EGFR antagonists that can be used in the methods of the invention include [6,7-bis(2-methoxyethoxy)-4-quinazolin-4-yl]-(3-ethynylphenyl) amine (also known as OSI-774, erlotinib, or TARCEVA™ (erlotinib HCl); OSI Pharmaceuticals/Genentech/Roche) (U.S. Pat. No. 5,747,498; International Patent Publication No. WO 01/34574, and Moyer et al., Cancer Res. 57:4838-4848 (1997)); CI-1033 (formerly known as PD183805; Pfizer) (Sherwood et al., 1999, Proc. Am. Assoc. Cancer Res. 40:723); PD-158780 (Pfizer); AG-1478 (University of California); CGP-59326 (Novartis); PKI-166 (Novartis); EKB-569 (Wyeth); GW-2016 (also known as GW-572016 or lapatinib ditosylate; GSK); and gefitinib (also known as ZD1839 or IRESSA™; Astrazeneca) (Woodburn et al., 1997, Proc. Am. Assoc. Cancer Res. 38:633).

Exemplary EGFR antagonist antibodies that can be used in the methods of the invention include those described in Modjtahedi, et al., Br. J. Cancer 67:247-253 (1993); Teramoto et al., Cancer 77:639-645 (1996); Goldstein et al., Clin. Cancer Res. 1:1311-1318 (1995); Huang, et al., Cancer Res. 15:59(8):1935-40 (1999); and Yang et al., Cancer Res. 59:1236-1243 (1999). Thus, in some embodiments the EGFR antagonist antibody can be the monoclonal antibody Mab E7.6.3 (Yang et al., Cancer Res. 59:1236-43 (1999)), or Mab C225 (ATCC Accession No. HB-8508), or an antibody or antibody fragment having the binding specificity thereof. Suitable monoclonal EGFR antagonist antibodies include, but are not limited to, IMC-C225 (also known as cetuximab or ERBITUX™; Imclone Systems), ABX-EGF (Abgenix), EMD 72000 (Merck KgaA, Darmstadt), RH3 (York Medical Bioscience Inc.), and MDX-447 (Medarex/ Merck KgaA).

In certain embodiments, the cancer expresses FGFR3, amplified FGFR3, translocated FGFR3, and/or mutated FGFR3. In certain embodiments, the cancer expresses activated FGFR3. In certain embodiments, the cancer expresses translocated FGFR3 (e.g., a t(4;14) translocation). In certain embodiments, the cancer expresses constitutive FGFR3. In some embodiments, the constitutive FGFR3 comprises a mutation in the tyrosine kinase domain and/or the juxtamembrane domain and/or a ligand-binding domain. In certain embodiments, the cancer expresses ligand-independent FGFR3. In some embodiments, the cancer expresses ligand-dependent FGFR3.

In some embodiments, the cancer expresses FGFR3 comprising a mutation corresponding to FGFR3-IIIb^{S248C}. In some embodiments, the cancer expresses FGFR3-IIIb ^{S248C} and/or FGFR3-IIIc^{S248C}.

In some embodiments, the cancer expresses FGFR3 comprising a mutation corresponding to FGFR3-IIIb^{K652E}. In some embodiments, the cancer expresses FGFR3-IIIb ^{K652E} and/or FGFR3-IIIc^{K650E}.

FGFR3 comprising a mutation corresponding to FGFR3-IIIb^{S249C}. In some embodiments, the cancer expresses FGFR3-IIIb^{S249C} and/or FGFR3-IIIc^{S249C}.

In one aspect, the cancer expresses FGFR3 comprising a mutation corresponding to FGFR3-IIIb^{G372C}. In some embodiments, the cancer expresses FGFR3-IIIb^{G372C} and/or FGFR3-IIIc^{G370C}.

In one aspect, the cancer expresses FGFR3 comprising a mutation corresponding to FGFR3-IIIb^{Y375C}. In some embodiments, the cancer expresses FGFR3-IIIb^{Y375C} and/or FGFR3-IIIc^{Y373C}.

In some embodiments, the cancer expresses (a) FGFR3-IIIb^{K652E} and (b) one or more of FGFR3-IIIb^{R248C}, FGFR3-IIIb^{Y375C}, FGFR3 -IIIb^{S249C}, and FGFR3IIIb ^{G372C}.

In some embodiments, the cancer expresses (a) FGFR3-IIIb^{R248C} and (b) one or more of FGFR3-IIIb^{K652E}, FGFR3-IIIb^{Y375C}, FGFR3-IIIb^{S249C}, and FGFR3-IIIb^{G372C}.

In some embodiments, the cancer expresses (a) FGFR3-IIIb^{G372C} and (b) one or more of FGFR3-IIIb^{K652E}, FGFR3-IIIb^{Y375C}, FGFR3-IIIb^{S249C}, and FGFR3-IIIb^{R248C}.

In some embodiments, the cancer expresses FGFR3-IIIb^{R248C}, FGFR3-IIIb^{K652E}, FGFR3-IIIb^{Y375C}, FGFR3-IIIb^{S249C}, and FGFR3-IIIb^{G372C}.

In certain embodiments, the cancer expresses increased levels of phospho-FGFR3, phospho-FRS2 and/or phospho-MAPK relative to a control sample (e.g., a sample of normal tissue) or level.

The composition comprising an anti-cancer therapy, for example, an anti-cancer therapy comprising an antagonist (e.g., an FGFR3 antagonist, a TP53 antagonist, and/or a EGFR antagonist) will be formulated, dosed, and administered in a fashion consistent with good medical practice. Factors for consideration in this context include the particular type of cancer being treated, the particular mammal being treated, the clinical condition of the individual patient, the cause of the cancer, the site of delivery of the agent, possible side-effects, the type of antagonist, the method of administration, the scheduling of administration, and other factors known to medical practitioners. The effective amount of the antagonist to be administered will be governed by such considerations.

The therapeutic agents of the invention are administered to a human patient, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. An *ex vivo* strategy can also be used for therapeutic applications. *Ex vivo* strategies involve transfecting or transducing cells obtained from the subject with a polynucleotide encoding an antagonist. The transfected or transduced cells are then returned to the subject. The cells can be any of a wide range of types including, without limitation, hemopoietic cells (e.g., bone marrow cells, macrophages, monocytes, dendritic cells, T cells, or B cells), fibroblasts, epithelial cells, endothelial cells, keratinocytes, or muscle cells.

For example, if the anti-cancer therapy includes an antagonist antibody (e.g., an FGFR3 antagonist antibody, a TP53 antagonist antibody, or an EGFR antagonist antibody), the antibody is administered by any suitable means, including parenteral, subcutaneous, intraperitoneal, intrapulmonary, and intranasal, and, if desired for local immunosuppressive treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Preferably the dosing is given by injections, most preferably intravenous or subcutaneous injections, depending in part on whether the administration is brief or chronic.

In another example, the antagonist compound is administered locally, for example, by direct injections, when the disorder or location of the tumor permits, and the injections can be repeated periodically. The antagonist can also be delivered systemically to the subject or directly to the tumor cells, e.g., to a tumor or a tumor bed following surgical excision of the tumor, in order to prevent or reduce local recurrence or metastasis.

An anti-cancer therapy may be combined in a pharmaceutical combination formulation, or dosing regimen as combination therapy, with at least one additional compound having anti-cancer properties. The at least one additional compound of the pharmaceutical combination formulation or dosing regimen preferably has complementary activities to the antagonist composition such that they do not adversely affect each other.

The anti-cancer therapy may include a chemotherapeutic agent, a cytotoxic agent, a cytokine, a growth inhibitory agent, an anti-hormonal agent, and combinations thereof. Such molecules are suitably present in combination in amounts that are effective for the purpose intended. For example, a pharmaceutical composition containing an antagonist (e.g., an FGFR3 antagonist, a TP53 antagonist, and/or an EGFR antagonist) may also comprise a therapeutically effective amount of an anti-neoplastic agent, a chemotherapeutic agent a growth inhibitory agent, a cytotoxic agent, or combinations thereof.

Administration of therapeutic agents in combination typically is carried out over a defined time period (usually minutes, hours, days or weeks depending upon the combination selected). Combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner.

The therapeutic agent can be administered by the same route or by different routes. For example, an antagonist antibody in the combination may be administered by intravenous injection while a chemotherapeutic agent in the combination may be administered orally. Alternatively, for example, both of the therapeutic agents may be administered orally, or both therapeutic agents may be administered by intravenous injection, depending on the specific therapeutic agents. The sequence in which the therapeutic agents are administered also varies depending on the specific agents.

Depending on the type and severity of the disease, about 1 µg/kg to 100 mg/kg of each therapeutic agent is an initial candidate dosage for administration to the patient, whether, for example, by one or more separate administrations, or by continuous infusion. A typical daily dosage might range from about 1 µg/kg to about 100 mg/kg or more, depending on the factors mentioned above. For repeated administrations over several days or longer, depending on the condition, the treatment is sustained until the cancer is treated, as measured by the methods described above. However, other dosage regimens may be useful. Preparation and dosing schedules for constituents of an anti-cancer therapy, for example, an anti-cancer therapy comprising one or more antagonists (e.g., FGFR3 antagonists, TP53 antagonists, and/or EGFR antagonists), chemotherapeutic agents, etc. may be used according to manufacturer's instructions or as determined empirically by the skilled practitioner. Preparation and dosing schedules for such chemotherapy are also described in "Chemotherapy Service," Ed., M.C. Perry, Williams & Wilkins, Baltimore, Md (1992).

The combination therapy may provide "synergy" and prove "synergistic", that is, the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds are administered or delivered sequentially, for example, by different injections in separate syringes. In general, during alternation therapy, an effective dosage of each active ingredient is administered sequentially, i.e., serially, whereas in combination therapy, effective dosages of two or more active ingredients are administered together.

Aside from administration of an anti-cancer therapy to the patient by traditional routes as noted above, the present invention includes administration by gene therapy. Such administration of nucleic acids encoding the antagonist is encompassed by the expression "administering an effective amount of an anti-cancer therapy." See, for example, WO 1996/07321 concerning the use of gene therapy to generate intracellular antibodies. Such a method may be useful, for example, to target intracellular proteins such as TP53.

There are two major approaches to getting the nucleic acid (optionally contained in a vector) into the patient's cells; *in vivo* and *ex vivo.* For *in vivo* delivery the nucleic acid is injected directly into the patient, usually at the site where the antagonist is required. For *ex vivo* treatment, the patient's cells are removed, the nucleic acid is introduced into these isolated cells and the modified cells are administered to the patient either directly or, for example, encapsulated within porous membranes which are implanted into the patient (see, e.g., U.S. Patent Nos. 4,892,538 and 5,283,187). There are a variety of techniques available for introducing nucleic acids into viable cells. The techniques vary depending upon whether the nucleic acid is transferred into cultured cells *in vitro* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, *etc.* A commonly used vector for *ex vivo* delivery of the gene is a retrovirus.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent specific for the target cells, such as an antibody specific for a cell-surface membrane protein on the target cell, a ligand for a receptor on the target cell, *etc.* Where liposomes are employed, proteins that bind to a cell-surface membrane protein associated with endocytosis may be used for targeting and/or to facilitate uptake, e.g., capsid proteins or fragments thereof tropic for a particular cell type, antibodies for proteins that undergo internalization in cycling, and proteins that target intracellular localization and enhance intracellular half-life. The technique of receptor-mediated endocytosis is described, for example, by Wu et al., J. Biol. Chem. 262:4429-4432 (1987); and Wagner et al., PNAS USA 87:3410-3414 (1990). Gene-marking and gene-therapy protocols are described, for example, in Anderson et al., Science 256:808-813 (1992) and WO 1993/25673.

### IV. Diagnostic and Prognostic Methods

The present invention provides methods for diagnosing a patient suffering from a cancer, for determining a prognosis of a patient suffering from cancer, for determining whether a patient having a cancer is likely to respond to treatment with an anti-cancer therapy, and for optimizing therapeutic efficacy of an anti-cancer therapy for a patient having a cancer. The methods are useful, *inter alia,* for diagnosing subtypes of cancer, for predicting treatment outcomes, and for increasing the likelihood that administration of an anti-cancer therapy to a patient will be efficacious. In several embodiments, the methods include determining an expression level at least one of the biomarkers of the invention (for example, a biomarker listed in Table 1) in a sample obtained from the patient and comparing the expression level to a reference level of the biomarker of the invention. In particular embodiments, the methods include determining an expression level of at least one of the following genes: *FGFR3, TP53,* and/or *EGFR* in a sample obtained from the patient and comparing the expression level to a reference level of the at least one gene. The expression level of biomarkers of the invention (e.g., *FGFR3, TP53,* and/or *EGFR)* can be determined using any of the methods or assays described below or by any method or assay known in the art. In particular embodiments, the cancer is bladder cancer. In some embodiments, the bladder cancer is NMIBC. In other embodiments, the bladder cancer is MIBC. In yet other embodiments, the bladder cancer is metastatic bladder cancer. The patient may optionally have an advanced, refractory, recurrent, and/or chemotherapy-resistant form of the cancer. For example, in some embodiments, a patient may have a recurrent bladder cancer, for example, a recurrent NMIBC.

For instance, in some embodiments, the invention provides a method for diagnosing a cancer in a patient, the method comprising the steps of: (a) determining the expression level of at least one of the genes listed in Table 1 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 genes listed in Table 1) in a sample obtained from the patient; and (b) comparing the expression level of the at least one gene to a reference level of the at least one gene, wherein a change in the expression level of the at least one gene in the patient sample relative to the reference level identifies a patient having a cancer. In some embodiments, the change may be an increase. In other embodiments, the change may be a decrease. In some embodiments, the cancer is bladder cancer. For example, in particular embodiments, the invention provides a method for diagnosing a cancer in a patient, the method comprising the steps of: (a) determining the expression level of at least one (e.g., 1, 2, or 3) of the following genes: *FGFR3, TP53,* and *EGFR,* in a sample obtained from the patient; and (b) comparing the expression level of the at least one gene to a reference level of the at least one gene, wherein an increase in the expression level of the at least one gene in the patient sample relative to the reference level identifies a patient having a cancer. In some embodiments, the cancer is bladder cancer.

In some instances, the invention provides a method for diagnosing a cancer in a patient, the method comprising the steps of: (a) determining the expression level of *FGFR3* in a sample obtained from the patient; and (b) comparing the expression level of *FGFR3* to a reference level of *FGFR3,* wherein an increase in the expression level of *FGFR3* in the patient sample relative to the reference level identifies a patient having a cancer. In some embodiments, the cancer is bladder cancer.

In some instances, the invention provides a method for diagnosing a cancer in a patient, the method comprising the steps of: (a) determining the expression level of *TP53* in a sample obtained from the patient; and (b) comparing the expression level of *TP53* to a reference level of *TP53*, wherein an increase in the expression level of *TP53* in the patient sample relative to the reference level identifies a patient having a cancer. In some embodiments, the cancer is bladder cancer.

In some instances, the invention provides a method for diagnosing a cancer in a patient, the method comprising the steps of: (a) determining the expression level of *EGFR* in a sample obtained from the patient; and (b) comparing the expression level of *EGFR* to a reference level of *EGFR,* wherein an increase in the expression level of *EGFR* in the patient sample relative to the reference level identifies a patient having a cancer. In some embodiments, the cancer is bladder cancer.

In another example, in some embodiments, the invention provides a method for diagnosing a cancer in a patient, the method comprising the steps of: (a) determining the expression level of at least one of the genes listed in Table 4 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes listed in Table 4) in a sample obtained from the patient; and (b) comparing the expression level of the at least one gene to a reference level of the at least one gene, wherein a change in the expression level of the at least one gene in the patient sample relative to the reference level identifies a patient having a cancer. In some embodiments, the change may be an increase. In other embodiments, the change may be a decrease. In some embodiments, the cancer is bladder cancer.

In any of the preceding methods, the method may further include (c) informing the patient that they have a cancer. In any of the preceding methods, the method may further include (d) selecting an anti-cancer therapy for treatment of the patient, when an increase in the level of expression of the at least one gene in the patient sample relative to the sample is detected. In any of the preceding embodiments, the method may further include administering a therapeutically effective amount of an anti-cancer therapy to the patient, for example, as described in Section III of the Detailed Description of the Invention.

In other instances, the inventions provides a method for the prognosis of a patient suffering from cancer, the method comprising: (a) determining the expression level of at least one of the genes listed in Table 1 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 genes listed in Table 1) in a sample obtained from the patient; (b) comparing the expression level of the at least one gene to a reference level of the at least one gene; and (c) determining a prognosis for the patient, wherein a poor prognosis is indicated by an expression level of the at least one gene in the patient sample that is changed relative to the reference level. In some embodiments, the change may be an increase. In other embodiments, the change may be a decrease. In some embodiments, the cancer is bladder cancer.

For example, in some instances, the invention provides a method for the prognosis of a patient suffering from cancer, the method comprising: (a) determining the expression level of at least one of the following genes: *FGFR3, TP53,* and *EGFR,* in a sample obtained from the patient; (b) comparing the expression level of the at least one gene to a reference level of the at least one gene; and (c) determining a prognosis for the patient, wherein a poor prognosis is indicated by an expression level of the at least one gene in the patient sample that is increased relative to the reference level.

In some instances, the invention provides a method for the prognosis of a patient suffering from cancer, the method comprising: (a) determining the expression level of *FGFR3* in a sample obtained from the patient; (b) comparing the expression level of *FGFR3* to a reference level of *FGFR3*; and (c) determining a prognosis for the patient, wherein a poor prognosis is indicated by an expression level of the at least one gene in the patient sample that is increased relative to the reference level. In some embodiments, the cancer is bladder cancer.

In other instances, the invention provides a method for the prognosis of a patient suffering from cancer, the method comprising: (a) determining the expression level of *TP53* in a sample obtained from the patient; (b) comparing the expression level of *TP53* to a reference level of *TP53*; and (c) determining a prognosis for the patient, wherein a poor prognosis is indicated by an expression level of the at least one gene in the patient sample that is increased relative to the reference level. In some embodiments, the cancer is bladder cancer.

For example, in some instances, the invention provides a method for the prognosis of a patient suffering from cancer, the method comprising: (a) determining the expression level of *EGFR* in a sample obtained from the patient; (b) comparing the expression level of *EGFR* to a reference level of *EGFR*; and (c) determining a prognosis for the patient, wherein a poor prognosis is indicated by an expression level of the at least one gene in the patient sample that is increased relative to the reference level. In some embodiments, the cancer is bladder cancer.

In other instances, the inventions provides a method for the prognosis of a patient suffering from cancer, the method comprising: (a) determining the expression level of at least one of the genes listed in Table 4 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes listed in Table 4) in a sample obtained from the patient; (b) comparing the expression level of the at least one gene to a reference level of the at least one gene; and (c) determining a prognosis for the patient, wherein a poor prognosis is indicated by an expression level of the at least one gene in the patient sample that is changed relative to the reference level. In some embodiments, the change may be an increase. In other embodiments, the change may be a decrease. In some embodiments, the cancer is bladder cancer.

In any of the preceding methods, the prognosis may be a prognosis of survival. In some instances, the method is carried out prior to administering an anti-cancer therapy to the patient. In other embodiments, the method is carried out during administering an anti-cancer therapy to the patient. In other embodiments, the method is carried out after administering an anti-cancer therapy to the patient.

In any of the preceding methods, the method may further include identifying the patient as likely to benefit from administration of an anti-cancer therapy when the patient is determined to have a poor prognosis of survival. In other instances, the method may further include identifying the patient as less likely to benefit from administration of an anti-cancer therapy when the patient is determined to have a poor prognosis of survival.

In any of the preceding methods, the method may further include administering a therapeutically effective amount of an anti-cancer therapy to the patient, if the patient is determined to have a poor prognosis of survival, as described, for example, in Section III of the Detailed Description of the Invention.

In any of the preceding methods, the survival may be disease free survival, progression free survival, or overall survival.

In other instances, the invention provides a method of determining whether a patient having a cancer is likely to respond to treatment with an anti-cancer therapy, the method comprising: (a) determining the expression level of at least one of the genes listed in Table 1 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 genes listed in Table 1) in a sample obtained from the patient; and (b) comparing the expression level of the at least one gene to a reference level of the at least one gene, wherein a change in the expression level of the at least one gene in the patient sample relative to the reference level identifies a patient who is likely to respond to treatment comprising an anti-cancer therapy. In some embodiments, the change may be an increase. In other embodiments, the change may be a decrease. In some embodiments, the cancer is bladder cancer.

For example, in some instances, the invention provides a method of determining whether a patient having a cancer is likely to respond to treatment with an anti-cancer therapy, the method comprising: (a) determining the expression level of at least one of the following genes: *FGFR3, TP53,* and *EGFR,* in a sample obtained from the patient; and (b) comparing the expression level of the at least one gene to a reference level of the at least one gene, wherein an increase in the expression level of the at least one gene in the patient sample relative to the reference level identifies a patient who is likely to respond to treatment comprising an anti-cancer therapy. In some embodiments, the cancer is bladder cancer.

For example, in some instances, the invention provides a method of determining whether a patient having a cancer is likely to respond to treatment with an anti-cancer therapy, the method comprising: (a) determining the expression level of *FGFR3* in a sample obtained from the patient; and (b) comparing the expression level of *FGFR3* to a reference level of *FGFR3,* wherein an increase in the expression level of *FGFR3* in the patient sample relative to the reference level identifies a patient who is likely to respond to treatment comprising an anti-cancer therapy. In some embodiments, the cancer is bladder cancer.

In other instances, the invention provides a method of determining whether a patient having a cancer is likely to respond to treatment with an anti-cancer therapy, the method comprising: (a) determining the expression level of *TP53* in a sample obtained from the patient; and (b) comparing the expression level of *TP53* to a reference level of *TP53,* wherein an increase in the expression level of *TP53* in the patient sample relative to the reference level identifies a patient who is likely to respond to treatment comprising an anti-cancer therapy. In some embodiments, the cancer is bladder cancer.

In yet other instances, the invention provides a method of determining whether a patient having a cancer is likely to respond to treatment with an anti-cancer therapy, the method comprising: (a) determining the expression level of *EGFR* in a sample obtained from the patient; and (b) comparing the expression level of *EGFR* to a reference level of *EGFR*, wherein an increase in the expression level of *EGFR* in the patient sample relative to the reference level identifies a patient who is likely to respond to treatment comprising an anti-cancer therapy. In some embodiments, the cancer is bladder cancer.

In other instances, the invention provides a method of determining whether a patient having a cancer is likely to respond to treatment with an anti-cancer therapy, the method comprising: (a) determining the expression level of at least one of the genes listed in Table 4 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes listed in Table 4) in a sample obtained from the patient; and (b) comparing the expression level of the at least one gene to a reference level of the at least one gene, wherein a change in the expression level of the at least one gene in the patient sample relative to the reference level identifies a patient who is likely to respond to treatment comprising an anti-cancer therapy. In some embodiments, the change may be an increase. In other embodiments, the change may be a decrease. In some embodiments, the cancer is bladder cancer.

In some instances, the invention provides a method of optimizing therapeutic efficacy of an anti-cancer therapy for a patient having a cancer, the method comprising: (a) determining the expression level of at least one of the genes listed in Table 1 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 genes listed in Table 1) in a sample obtained from the patient; and (b) comparing the expression level of the at least one gene to a reference level of the at least one gene, wherein a change in the expression level of the at least one gene in the patient sample relative to the reference level identifies a patient who is likely to respond to treatment comprising an anti-cancer therapy. In some embodiments, the change may be an increase. In other embodiments, the change may be a decrease. In some embodiments, the cancer is bladder cancer.

For example, in some instances, the invention provides a method of optimizing therapeutic efficacy of an anti-cancer therapy for a patient having a cancer, the method comprising: (a) determining the expression level of at least one (e.g., 1, 2, or 3) of the following genes: *FGFR3, TP53,* and *EGFR* in a sample obtained from the patient; and (b) comparing the expression level of the at least one gene to a reference level of the at least one gene, wherein an increase in the expression level of the at least one gene in the patient sample relative to the reference level identifies a patient who is likely to respond to treatment comprising an anti-cancer therapy. In some embodiments, the change may be an increase. In other embodiments, the change may be a decrease. In some embodiments, the cancer is bladder cancer.

In some instances, the invention provides a method of optimizing therapeutic efficacy of an anti-cancer therapy for a patient having a cancer, the method comprising: (a) determining the expression level of *FGFR3* in a sample obtained from the patient; and (b) comparing the expression level of *FGFR3* to a reference level of *FGFR3,* wherein an increase in the expression level of *FGFR3* in the patient sample relative to the reference level identifies a patient who is likely to respond to treatment comprising an anti-cancer therapy. In some embodiments, the change may be an increase. In other embodiments, the change may be a decrease. In some embodiments, the cancer is bladder cancer.

In some instances, the invention provides a method of optimizing therapeutic efficacy of an anti-cancer therapy for a patient having a cancer, the method comprising: (a) determining the expression level of at least one of the genes listed in Table 4 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes listed in Table 4) in a sample obtained from the patient; and (b) comparing the expression level of the at least one gene to a reference level of the at least one gene, wherein a change in the expression level of the at least one gene in the patient sample relative to the reference level identifies a patient who is likely to respond to treatment comprising an anti-cancer therapy. In some embodiments, the change may be an increase. In other embodiments, the change may be a decrease. In some embodiments, the cancer is bladder cancer.

In any of the preceding methods, the method may further comprise further comprising determining the expression level of at least one additional gene selected from the group consisting of: *DUSB3*, *FRS2*, *TSC1*, *ERBB3*, *CDKN1A*, *CCND1*, *TP63*, *MMP2*, *ZEB2*, *PIK3CB*, *PIK3R1*, *MDM2*, *SNAI2*, *AXL*, *ZEB1*, *BCL2B*, *TSC2*, *RB1*, *FGFR32*, *PIK3IP1*, *MTOR*, *PIK3CA*, *PTEN*, *AKT1*, *BCL2A*, *FRS3*, *ERBB2*, *FGFR31*, *FGF1*, *SNAI1*, *FGFR34*, *FGF9*, and *FGF2*, in a sample obtained from the patient, wherein the expression level of the at least one additional gene is changed relative to a reference level of the at least one additional gene.

In some instances, the invention provides a method of treating a patient suffering from a bladder cancer, the method comprising administering to the patient a therapeutically effective amount of an anti-cancer therapy other than Bacillus Calmette-Guerin (BCG) vaccine, wherein the expression level of *TP53* in a sample obtained from the patient has been determined to be increased relative to a reference level of *TP53.*

In any of the preceding methods, the method may further include administering a therapeutically effective amount of an anti-cancer therapy to the patient, as described, for example, in Section III of the Detailed Description of the Invention.

In any of the preceding methods, the expression level of a biomarker of the invention, e.g., a gene listed in Table 1, for example, *FGFR3, TP53,* and/or *EGFR,* may be determined by measuring messenger RNA (mRNA), as described, for example below or by methods known in the art. In some instances, the expression level of a biomarker of the invention in a sample obtained from the patient is determined by a polymerase chain reaction (PCR) assay. In some instances, the PCR assay is a quantitative PCR assay. In some embodiments, the quantitative PCR assay is a TAQMAN® assay. In some embodiments, the assay is performed using a Fluidigm assay.

In any of the preceding methods, the expression level of a biomarker of the invention, e.g., a gene listed in Table 1, for example, *FGFR3, TP53,* and/or *EGFR,* may be determined by measuring protein, as described, for example below or by methods known in the art. In some instances, the expression level of a biomarker of the invention in a sample obtained from the patient is determined by an immunohistochemical method.

An exemplary method for determining the expression level for FGFR3 by immunohistochemistry is provided below. Similar assays could be used for other biomarkers of the invention.

In one embodiment, FGFR3 overexpression may be analyzed by IHC. Paraffin-embedded tissue sections from a tumor biopsy may be subjected to the IHC assay and accorded a FGFR3 protein staining intensity criteria as follows:
*Score 0:* no staining is observed or membrane staining is observed in less than 10% of tumor cells.
*Score 1+:* a faint/barely perceptible membrane staining is detected in more than 10% of the tumor cells. The cells are only stained in part of their membrane.
*Score 2+:* a weak to moderate complete membrane staining is observed in more than 10% of the tumor cells.
*Score 3+:* a moderate to strong complete membrane staining is observed in more than 10% of the tumor cells.

In some embodiments, those tumors with 0 or 1+ scores for FGFR3 overexpression assessment may be characterized as not overexpressing FGFR3, whereas those tumors with 2+ or 3+ scores may be characterized as overexpressing FGFR3.

In some embodiments, tumors overexpressing FGFR3 may be rated by immunohistochemical scores corresponding to the number of copies of FGFR3 molecules expressed per cell, and can been determined biochemically:
0=0-90 copies/cell,
1+=at least about 100 copies/cell,
2+=at least about 1000 copies/cell,
3+=at least about 10,000 copies/cell.

In some instances, the sample obtained from the patient is a tumor sample.

In any of the preceding methods of the invention, the expression level of a biomarker of the invention (e.g., a gene listed in Table 1) in a sample obtained from the patient may be changed at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of biomarker. For instance, in some embodiments, the expression level of a biomarker of the invention in a sample obtained from the patient may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of biomarker. In other embodiments, the expression level of a biomarker of the invention in a sample obtained from the patient may be decreased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of biomarker.

In particular embodiments, the expression level of at least one (e.g., 1, 2, or 3) of the following: *FGFR3, TP53,* and/or *EGFR* in a sample obtained from the patient may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of the at least one gene. For instance, the expression level of *FGFR3* may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of *FGFR3.* In another instance, the expression level of *TP53* may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of *TP53.* In yet another instance, the expression level of *EGFR* may be increased at least 10%, 20%, 30%, 40%, 50%, 60%, 70%, 80%, 90%, 100%, 2-fold, 3-fold, 5-fold, 6-fold, 7-fold, 8-fold, 9-fold, 10-fold, 11-fold, 12-fold, 13-fold, 14-fold, 15-fold, 16-fold, or more relative to a reference level of *EGFR.*

In some embodiments, the reference level may be set to any percentile between, for example, the 20^{th} percentile and the 99^{th} percentile (e.g., the 20^{th}, 25^{th}, 30^{th}, 35^{th}, 40^{th}, 45^{th}, 50^{th}, 55^{th}, 60^{th}, 65^{th}, 70^{th}, 75^{th}, 80^{th}, 85^{th}, 90^{th}, 95^{th}, or 99^{th} percentile) of the overall distribution of the expression level of a biomarker (for example, *FGFR3*, *TP53*, or *EGFR*) in a given cancer type (e.g., bladder cancer). In particular embodiments, the reference level may be set to the 25^{th} percentile of the overall distribution of the values in a bladder cancer. In other particular embodiments, the reference level may be set to the 50^{th} percentile of the overall distribution of the values in a bladder cancer. In other embodiments, the reference level may be the median of the overall distribution of the values in a bladder cancer.

The disclosed methods and assays provide for convenient, efficient, and potentially cost-effective means to obtain data and information useful in assessing appropriate or effective therapies for treating patients. For example, a patient could provide a sample (e.g., a tumor biopsy or a blood sample) for diagnosis of a particular cancer, or subtype of cancer. In some instances, the diagnosis could be for bladder cancer. In other instances, the diagnosis could be for a subtype of bladder cancer, for example, NMIBC, MIBC, or metastatic bladder cancer. In other instances, a patient could provide a sample (e.g., a tumor biopsy or a blood sample) before treatment with an anti-cancer therapy and the sample could be examined by way of various *in vitro* assays to determine whether the patient's cells would be sensitive to an anti-cancer therapy, for example, an anti-cancer therapy including an FGFR3 antagonist, a TP53 antagonist, and/or an EGFR antagonist.

One of skill in the medical arts, particularly pertaining to the application of diagnostic tests and treatment with therapeutics, will recognize that biological systems are somewhat variable and not always entirely predictable, and thus many good diagnostic tests or therapeutics are occasionally ineffective. Thus, it is ultimately up to the judgment of the attending physician to determine the most appropriate course of treatment for an individual patient, based upon test results, patient condition and history, and his or her own experience. There may even be occasions, for example, when a physician will choose to treat a patient with an anti-cancer therapy, for example, an anti-cancer therapy including an antagonist (e.g., an FGFR3 antagonist, a TP53 antagonist, and/or an EGFR antagonist), even when a patient is not predicted to be particularly sensitive to VEGF antagonists, based on data from diagnostic tests or from other criteria, particularly if all or most of the other obvious treatment options have failed, or if some synergy is anticipated when given with another treatment.

The sample may be taken from a patient who is suspected of having, or is diagnosed as having a cancer (e.g., bladder cancer), and hence is likely in need of treatment, or from a normal individual who is not suspected of having any disorder. For assessment of marker expression, patient samples, such as those containing cells, or proteins or nucleic acids produced by these cells, may be used in the methods of the present invention. In the methods of this invention, the level of a biomarker can be determined by assessing the amount (e.g., the absolute amount or concentration) of the markers in a sample, preferably a tissue sample (e.g., a tumor tissue sample, such as a biopsy). In addition, the level of a biomarker can be assessed in bodily fluids or excretions containing detectable levels of biomarkers. Bodily fluids or secretions useful as samples in the present invention include, e.g., blood, urine, saliva, stool, pleural fluid, lymphatic fluid, sputum, ascites, prostatic fluid, cerebrospinal fluid (CSF), or any other bodily secretion or derivative thereof. The word blood is meant to include whole blood, plasma, serum, or any derivative of blood. Assessment of a biomarker in such bodily fluids or excretions can sometimes be preferred in circumstances where an invasive sampling method is inappropriate or inconvenient. However, in the case of samples that are bodily fluids, the sample to be tested herein is preferably blood, synovial tissue, or synovial fluid, most preferably blood.

The sample may be frozen, fresh, fixed (e.g., formalin-fixed), centrifuged, and/or embedded (e.g., paraffin-embedded), etc. The cell sample can, of course, be subjected to a variety of well-known post-collection preparative and storage techniques (e.g., nucleic acid and/or protein extraction, fixation, storage, freezing, ultrafiltration, concentration, evaporation, centrifugation, *etc.*) prior to assessing the amount of the marker in the sample. Likewise, biopsies may also be subjected to post-collection preparative and storage techniques, e.g., fixation.

As noted above, any of the preceding methods further can, optionally, include selection of an anti-cancer therapy for administration to the patient and further include, optionally, administration of an anti-cancer therapy to the patient.

### A. Detection of Gene Expression

The genetic biomarkers described herein can be detected using any method known in the art. For example, tissue or cell samples from mammals can be conveniently assayed for, e.g., mRNAs or DNAs from a genetic biomarker of interest using Northern, dot-blot, or polymerase chain reaction (PCR) analysis, array hybridization, RNase protection assay, or using DNA SNP chip microarrays, which are commercially available, including DNA microarray snapshots. For example, real-time PCR (RT-PCR) assays such as quantitative PCR assays are well known in the art. In an illustrative embodiment of the invention, a method for detecting mRNA from a genetic biomarker of interest in a biological sample comprises producing cDNA from the sample by reverse transcription using at least one primer; amplifying the cDNA so produced; and detecting the presence of the amplified cDNA. In addition, such methods can include one or more steps that allow one to determine the levels of mRNA in a biological sample (e.g., by simultaneously examining the levels a comparative control mRNA sequence of a "housekeeping" gene such as an actin family member). Optionally, the sequence of the amplified cDNA can be determined. Exemplary methods for detecting the expression level of FGFR3 are provided, for example, in U.S. Patent Application Publication No. 2014/0030259 and in U.S. Patent No. 8,410,250.

### 1. Detection of Nucleic Acids

In one specific embodiment, expression of the genes set forth in Table 1 can be performed by RT-PCR technology. Probes used for PCR may be labeled with a detectable marker, such as, for example, a radioisotope, fluorescent compound, bioluminescent compound, a chemiluminescent compound, metal chelator, or enzyme. Such probes and primers can be used to detect the presence of expressed genes set forth in Table 1 in a sample. As will be understood by the skilled artisan, a great many different primers and probes may be prepared based on the sequences provided in herein and used effectively to amplify, clone and/or determine the presence and/or levels of expressed genes set forth in Table 1 and/or Table 4. In some embodiments, the RT-PCR may be quantitative RT-PCR. In some instances, quantitative RT-PCR may be performed in a microfluidic device, for example, a Fluidigm BIOMARK™ BMK-M-96.96 platform.

Other methods include protocols that examine or detect mRNAs from at least one of the genes set forth in Table 1 (e.g., *FGFR3, TP53,* and *EGFR* mRNAs), in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes that have potential to be expressed in certain disease states may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment (see, e.g., WO 2001/75166). See, for example, U.S. Patent 5,700,637, U.S. Patent 5,445,934, and U.S. Patent 5,807,522, Lockart, Nature Biotechnology, 14:1675-1680 (1996); and Cheung et al., Nature Genetics 21(Suppl):15-19 (1999) for a discussion of array fabrication.

In addition, the DNA profiling and detection method utilizing microarrays described in EP 1753878 may be employed. This method rapidly identifies and distinguishes between different DNA sequences utilizing short tandem repeat (STR) analysis and DNA microarrays. In an embodiment, a labeled STR target sequence is hybridized to a DNA microarray carrying complementary probes. These probes vary in length to cover the range of possible STRs. The labeled single-stranded regions of the DNA hybrids are selectively removed from the microarray surface utilizing a post-hybridization enzymatic digestion. The number of repeats in the unknown target is deduced based on the pattern of target DNA that remains hybridized to the microarray.

One example of a microarray processor is the Affymetrix GENECHIP® system, which is commercially available and comprises arrays fabricated by direct synthesis of oligonucleotides on a glass surface. Other systems may be used as known to one skilled in the art.

Other methods for determining the level of the biomarker besides RT-PCR or another PCR-based method include proteomics techniques, as well as individualized genetic profiles that are necessary to treat a cancer based on patient response at a molecular level. The specialized microarrays herein, e.g., oligonucleotide microarrays or cDNA microarrays, may comprise one or more biomarkers having expression profiles that correlate with either sensitivity or resistance to one or more anti-cancer therapies. Other methods that can be used to detect nucleic acids, for use in the invention, involve high throughput RNA sequence expression analysis, including RNA-based genomic analysis, such as, for example, RNASeq.

Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987)). Northern blot analysis is a conventional technique well known in the art and is described, for example, in Molecular Cloning, a Laboratory Manual, second edition, 1989, Sambrook, Fritch, Maniatis, Cold Spring Harbor Press, 10 Skyline Drive, Plainview, NY 11803-2500. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis).

### 2. Detection of Proteins

As to detection of protein biomarkers such as those listed in Table 1 (for example, FGFR3, TP53, and/or EGFR), various protein assays are available including, for example, antibody-based methods as well as mass spectroscopy and other similar means known in the art. In the case of antibody-based methods, for example, the sample may be contacted with an antibody specific for said biomarker under conditions sufficient for an antibody-biomarker complex to form, and then detecting said complex. Detection of the presence of the protein biomarker may be accomplished in a number of ways, such as by Western blotting (with or without immunoprecipitation), 2-dimensional SDS-PAGE, immunoprecipitation, fluorescence activated cell sorting (FACS), flow cytometry, and ELISA procedures for assaying a wide variety of tissues and samples, including plasma or serum. A wide range of immunoassay techniques using such an assay format are available, see, e.g., U.S. Patent Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate, and the sample to be tested brought into contact with the bound molecule. After a suitable period of incubation, for a period of time sufficient to allow formation of an antibody-antigen complex, a second antibody specific to the antigen, labeled with a reporter molecule capable of producing a detectable signal is then added and incubated, allowing time sufficient for the formation of another complex of antibody-antigen-labeled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labeled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride, or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g., 2-40 minutes or overnight if more convenient) and under suitable conditions (e.g., from room temperature to 40°C such as between 25°C and 32°C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labeled with a reporter molecule. Depending on the amount of target and the strength of the reporter molecule signal, a bound target may be detectable by direct labeling with the antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule," as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e., radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase, and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

### B. Kits

For use in detection of the biomarkers, kits or articles of manufacture are also provided by the invention. Such kits can be used, for example, to diagnose a patient suffering from, or suspected of suffering from, a cancer (e.g., bladder cancer), to provide a prognosis for a patient suffering from a cancer, e.g., bladder cancer, and/or to determine if a patient with a cancer (e.g., bladder cancer) will be effectively responsive to an anti-cancer therapy. These kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be an antibody or polynucleotide specific for a protein or message, respectively. Where the kit utilizes nucleic acid hybridization to detect the target nucleic acid, the kit may also have containers containing nucleotide(s) for amplification of the target nucleic acid sequence and/or a container comprising a reporter-means, such as a biotin-binding protein, e.g., avidin or streptavidin, bound to a reporter molecule, such as an enzymatic, florescent, or radioisotope label.

Such kit will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific application, and may also indicate directions for either *in vivo* or *in vitro* use, such as those described above.

The kits of the invention have a number of embodiments. A typical embodiment is a kit comprising a container, a label on said container, and a composition contained within said container, wherein the composition includes a primary antibody that binds to a protein or autoantibody biomarker, and the label on said container indicates that the composition can be used to evaluate the presence of such proteins or antibodies in a sample, and wherein the kit includes instructions for using the antibody for evaluating the presence of biomarker proteins in a particular sample type. The kit can further comprise a set of instructions and materials for preparing a sample and applying antibody to the sample. The kit may include both a primary and secondary antibody, wherein the secondary antibody is conjugated to a label, e.g., an enzymatic label.

Another embodiment is a kit comprising a container, a label on said container, and a composition contained within said container, wherein the composition includes one or more polynucleotides that hybridize to a complement of a biomarker set forth in Table 1 under stringent conditions, and the label on said container indicates that the composition can be used to evaluate the presence of a biomarker set forth in Table 1 in a sample, and wherein the kit includes instructions for using the polynucleotide(s) for evaluating the presence of the biomarker RNA or DNA in a particular sample type.

Other optional components of the kit include one or more buffers (e.g., block buffer, wash buffer, substrate buffer, *etc.*), other reagents such as substrate (e.g., chromogen) that is chemically altered by an enzymatic label, epitope retrieval solution, control samples (positive and/or negative controls), control slide(s), etc. Kits can also include instructions for interpreting the results obtained using the kit.

In further specific embodiments, for antibody-based kits, the kit can comprise, for example: (1) a first antibody (e.g., attached to a solid support) that binds to a biomarker protein; and, optionally, (2) a second, different antibody that binds to either the protein or the first antibody and is conjugated to a detectable label.

For oligonucleotide-based kits, the kit can comprise, for example: (1) an oligonucleotide, e.g., a detectably labeled oligonucleotide, which hybridizes to a nucleic acid sequence encoding a biomarker protein or (2) a pair of primers useful for amplifying a biomarker nucleic acid molecule. The kit can also comprise, e.g., a buffering agent, a preservative, or a protein stabilizing agent. The kit can further comprise components necessary for detecting the detectable label (e.g., an enzyme or a substrate). The kit can also contain a control sample or a series of control samples that can be assayed and compared to the test sample. Each component of the kit can be enclosed within an individual container and all of the various containers can be within a single package, along with instructions for interpreting the results of the assays performed using the kit.

In some embodiments, a kit comprises any of the nucleotide sequences described herein, for example, one or more of SEQ ID NOs:1-51,

### V. Pharmaceutical Formulations

Therapeutic formulations of the therapeutic agent(s) of the anti-cancer therapies described herein (e.g., anti-cancer therapies including antagonists such as FGFR3 antagonists, TP53 antagonists, and/or EGFR antagonists) used in accordance with the present invention are prepared for storage by mixing the therapeutic agent(s) having the desired degree of purity with optional pharmaceutically acceptable carriers, excipients, or stabilizers in the form of lyophilized formulations or aqueous solutions. For general information concerning formulations, see, e.g., Gilman et al., (eds.) (1990), The Pharmacological Bases of Therapeutics, 8th Ed., Pergamon Press; A. Gennaro (ed.), Remington's Pharmaceutical Sciences, 18th Edition, (1990), Mack Publishing Co., Eastori, Pennsylvania; Avis et al., (eds.) (1993) Pharmaceutical Dosage Forms: Parenteral Medications Dekker, New York; Lieberman et al., (eds.) (1990) Pharmaceutical Dosage Forms: Tablets Dekker, New York; and Lieberman et al., (eds.) (1990), Pharmaceutical Dosage Forms: Disperse Systems Dekker, New York, Kenneth A. Walters (ed.) (2002) Dermatological and Transdermal Formulations (Drugs and the Pharmaceutical Sciences), Vol 119, Marcel Dekker.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™, or polyethylene glycol (PEG).

Lyophilized formulations adapted for subcutaneous administration are described, for example, in U.S. Patent No. 6,267,958 (Andya et al.). Such lyophilized formulations may be reconstituted with a suitable diluent to a high protein concentration and the reconstituted formulation may be administered subcutaneously to the mammal to be treated herein.

Crystallized forms of the therapeutic agent(s) are also contemplated. See, for example, US 2002/0136719A1.

The formulation herein may also contain more than one active compound (a second medicament as noted above), preferably those with complementary activities that do not adversely affect each other. The type and effective amounts of such medicaments depend, for example, on the amount and type of therapeutic agent(s) present in the formulation, and clinical parameters of the subjects. The preferred such second medicaments are noted above.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semi-permeable matrices of solid hydrophobic polymers containing the antagonist, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

### EXAMPLES

The following examples are provided to illustrate, but not to limit the presently claimed invention.

### Example 1. Materials and Methods

### Tumor samples

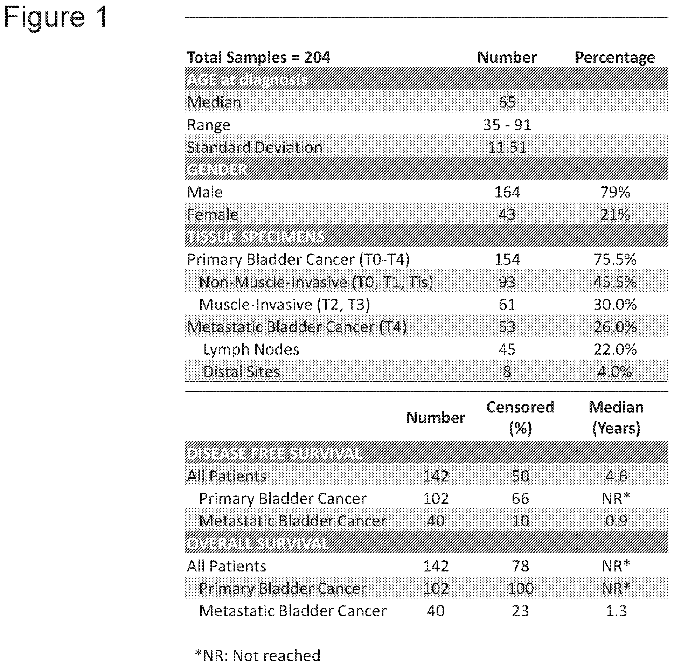

A collection of 204 formalin-fixed paraffin-embedded (FFPE) bladder cancer tumor samples were obtained from Cureline, Inc. (South San Francisco, CA) following approval of the Ethics Committee of Saint Petersburg City Clinical Oncology Hospital and appropriate confirmation of written informed consent, or from The MT Group (Van Nuys, CA) following Institutional Review Board approval (Sterling Institutional Review Board). The clinical samples faithfully recapitulated the expected ∼80%/20% male/female ratio that is typically observed in bladder cancer, and the proportions of non-muscle-invasive bladder cancers (NMIBCs; T0, T1), muscle-invasive bladder cancers (MIBCs; T2, T3) and metastases were ∼75% and ∼25%, respectively (Figure 1), consistent with the clinical incidence of these stages in the bladder cancer population (Jemal et al. CA Cancer J. Clin. 61(2): 69-90, 2011; Heney, Urol. Clin. North Am. 19(3): 429-433, 1992; Hall et al. Urology 52(4): 594-601, 1998). A subset of cases had accompanying treatment information, disease-free survival (DFS), and overall survival (OS) annotation similar to the expected clinical parameters for the respective groups (Figure 1, Table 3).

Hematoxylin & eosin (H&E) stained sections were evaluated by two independent pathologists, and the tumor areas for cases with less than 70% neoplastic cellularity were marked by a pathologist for macro-dissection. Overall, tumor content ranged from 70-90%. RNA and DNA were extracted from macrodissected samples as previously described (Schleifman et al. PLoS One 9(3): e90761; Schleifman et al. PLoS One 9(2): e88401).

### Expression analysis

Gene expression analysis was carried out on RNA extracted from FFPE tumor samples macrodissected using the High Pure FFPE RNA Micro Kit (Roche Diagnostics, Indianapolis, IN) after deparaffinization with ENVIRENE™, as described previously (Schleifman et al. PLoS One 9(2): e88401). Gene expression analysis of 96 unique mRNA transcripts of bladder cancer-relevant genes (see Table 1) was performed on patient specimens starting with 100 ng total RNA that was reverse-transcribed to cDNA and pre-amplified in a single reaction using SUPERSCRIPT® III/PLATINUM® Taq and pre-amplification reaction mix (Invitrogen, Carlsbad, CA). All 96 TAQMAN® primer/probe sets were included in the pre-amplification reaction at a final dilution of 0.05x original TAQMAN® assay concentration (Applied Biosystems, Foster City, CA). The thermocycling conditions were as follows: 1 cycle of 50°C for 15 min, 1 cycle of 70°C for 2 min, 14 cycles of 95°C for 15 sec and 1 cycle of 60°C for 4 min. Pre-amplified cDNA was diluted 2-fold and then amplified using TAQMAN® Universal PCR MasterMix (Applied Biosystems, Foster City, CA) on the BIOMARK™ BMK-M-96.96 platform (Fluidigm, South San Francisco, CA) according to the manufacturer's instructions. All samples were assayed in triplicate. Cycle threshold (Ct) values were converted to relative expression using the 2^{-ΔCt} method (Livak et al. Methods 25(4): 402-408, 2001), where ΔCt is the mean of the target gene minus the mean of the 96 Ct values calculated for the respective patient specimen. Table 2 shows the nucleotide sequences of custom TAQMAN® probes and oligonucleotides that were used for the indicated genes.

**Table 1. Custom Bladder Cancer Microfluidics Panel and Corresponding Assays**

| **Gene Symbol** | **Human Genome Organization (HUGO) Gene Nomenclature** | **HGNC ID** | **Location** | **Applied Biosystems Probe ID** |
|---|---|---|---|---|
| ACER3 | alkaline ceramidase 3 | 16066 | 11q13.5 | Hs00218034_m1 |
| ACVRL 1 | activin A receptor type II-like 1 | 175 | 12q13.13 | custom |
| ADAM9 | ADAM metallopeptidase domain 9 | 216 | 8p11.23 | Hs01106101_m1 |
| AKT1 | v-akt murine thymoma viral oncogene homolog 1 | 391 | 14q32.32-q32.33 | Hs00178289_m1 |
| AKT1S1 | AKT1 substrate 1 (proline-rich) | 28426 | 19q13.33 | Hs00260717_m1 |
| ARAF | v-raf murine sarcoma 3611 viral oncogene homolog | 646 | Xp11.3-p11.23 | Hs00176427_m1 |
| AXL | AXL receptor tyrosine kinase | 905 | 19q13.1 | Hs01064444_m1 |
| BCL2 | Alpha Isoform: B-cell CLL/lymphoma 2 | 990 | 18q21.3 | custom |
| BCL2 | Beta Isoform:B-cell CLL/lymphoma 2 | 990 | 18q21.3 | custom |
| BCL2L1 | BCL2-like 1 | 992 | 20q11.21 | custom |
| BID | BH3 interacting domain death agonist | 1050 | 22q11.2 | custom |
| BMP2 | bone morphogenetic protein 2 | 1069 | 20p12 | Hs00154192_m1 |
| BMX | BMX non-receptor tyrosine kinase | 1079 | Xp22.2 | Hs01107407_m1 |
| BSG | basigin (Ok blood group) | 1116 | 19p13.3 | Hs00936295_m1 |
| CBLB | Cbl proto-oncogene, E3 ubiquitin protein ligase B | 1542 | 3q | Hs00180288_m1 |
| CCND1 | cyclin D1 | 1582 | 11q13 | Hs00765553_m1 |
| CCND2 | cyclin D2 | 1583 | 12p13 | Hs00153380_m1 |
| CDC20 | cell division cycle 20 homolog (S. cerevisiae) | 1723 | 1p34.1 | Hs00415851_g1 |
| CDH1 | cadherin 1, type 1, E-cadherin (epithelial) | 1748 | 16q22.1 | Hs01023894_m1 |
| CDH2 | cadherin 2, type 1, N-cadherin (neuronal) | 1759 | 18q12.1 | Hs00983056_m1 |
| CDKN1A | cyclin-dependent kinase inhibitor 1A (p21, Cip1) | 1784 | 6p21.1 | Hs00355782_m1 |
| CDKN2A | cyclin-dependent kinase inhibitor 2A | 1787 | 9p21 | Hs02902543_mH |
| CSK | c-src tyrosine kinase | 2444 | 15q24.1 | Hs01062585_m1 |
| CXCL1 | chemokine (C-X-C motif) ligand 1 (melanoma growth stimulating activity, alpha) | 4602 | 4q13.3 | Hs00236937_m1 |
| DUSP1 | dual specificity phosphatase 1 | 3064 | 5q35.1 | Hs00610256_g1 |
| DUSP3 | dual specificity phosphatase 3 | 3069 | 17q21 | Hs01115776_m1 |
| DUSP6 | dual specificity phosphatase 6 | 3072 | 12q22-q23 | Hs00737962_m1 |
| EIF4EBP 1 | eukaryotic initiation factor 4E binding protein 1 | 3288 | 8p12 | Hs00607050_m1 |
| EPAS1 | endothelial PAS domain protein 1 | 3374 | 2p21-p16 | Hs01026149_m1 |
| ERBB2 | v-erb-b2 erythroblastic leukemia viral oncogene homolog 2, neuro/glioblastoma derived oncogene homolog (avian) | 3430 | 17q11.2-q12 | custom |
| ERBB3 | v-erb-b2 erythroblastic leukemia viral oncogene homolog 3 | 3431 | 12q13 | Hs00176538_m1 |
| FGF1 | fibroblast growth factor 1 (acidic) | 3665 | 5q31.3-q33.2 | Hs00265254_m1 |
| FGF10 | fibroblast growth factor 10 | 3666 | 5p13-p12 | Hs00610298_m1 |
| FGF2 | fibroblast growth factor 2 (basic) | 3676 | 4q26 | Hs00266645_m1 |
| FGF7 | fibroblast growth factor 7 | 3685 | 15q21.2 | Hs00940253_m1 |
| FGF9 | fibroblast growth factor 9 (glia-activating factor) | 3687 | 13q11-q12 | Hs00181829_m1 |
| FGFR1 | fibroblast growth factor receptor 1 | 3688 | 8p12 | Hs00241111_m1 |
| FGFR2 | fibroblast growth factor receptor 2 | 3689 | 10q25.3-q26 | Hs01552926_m1 |
| FGFR3 | C Isoform: fibroblast growth factor receptor 3 | 3690 | 4p16.3 | custom |
| FGFR3 | B Isoform: fibroblast growth factor receptor 3 | 3690 | 4p16.3 | custom |
| FGFR3 | fibroblast growth factor receptor 3 | 3690 | 4p16.3 | custom |
| FGFR4 | fibroblast growth factor receptor 4 | 3691 | 5q33-qter | Hs01106908_m1 |
| FLT1 | fms-related tyrosine kinase 1 (vascular endothelial growth factor/vascular permeability factor receptor) | 3763 | 13q12 | Hs01052961_m1 |
| FN1 | fibronectin 1 | 3778 | 2q34 | Hs01549976_m1 |
| FOSL1 | FOS-like antigen 1 | 13718 | 11q13 | Hs00759776_s1 |
| FRS2 | fibroblast growth factor receptor substrate 2 | 16971 | 12q15 | Hs00183614_m1 |
| FRS3 | fibroblast growth factor receptor substrate 3 | 16970 | 6p21.1 | Hs00183610_m1 |
| GATA3 | GATA binding protein 3 | 4172 | 10p15 | Hs00231122_m1 |
| GLI1 | GLI family zinc finger 1 | 4317 | 12q13.2-q13.3 | Hs01110766_m1 |
| HIF1A | hypoxia inducible factor 1, alpha subunit (basic helix-loop-helix transcription factor) | 4910 | 14q23.2 | Hs00153153_m1 |
| HPSE | heparanase | 5164 | 4q21.3 | Hs00935036_m1 |
| JAG1 | jagged 1 | 6188 | 20p12.1-p11.23 | Hs00164982_m1 |
| MAP2K2 | mitogen-activated protein kinase kinase 2 | 6842 | 19p13.3 | Hs00360961_m1 |
| MAPK14 | mitogen-activated protein kinase 14 | 6876 | 6p21.3-p21.2 | Hs00176247_m1 |
| MDM2 | Mdm2, p53 E3 ubiquitin protein ligase homolog | 6973 | 12q13-q14 | Hs00234753_m1 |
| MET | met proto-oncogene (hepatocyte growth factor receptor) | 7029 | 7q31 | custom |
| MMP2 | matrix metallopeptidase 2 (gelatinase A, 72kDa gelatinase, 72kDa type IV collagenase) | 7166 | 16q13-q21 | Hs01548727_m1 |
| MMP9 | matrix metallopeptidase 9 B, 92kDa gelatinase, 92kDa type IV collagenase) | 7176 | 20q12-q13 | Hs00234579_m1 |
| MTOR | mechanistic target of rapamycin (serine/threonine | 3942 | 1p36 | Hs00234522_m1 |
| MYC | v-myc myelocytomatosis viral oncogene homolog | 7553 | 8q24 | Hs00905030_m1 |
| NF1 | neurofibromin 1 | 7765 | 17q11.2 | Hs01035108_m1 |
| NOTCH1 | notch 1 | 7881 | 9q34.3 | custom |
| NOTCH2 | notch 2 | 7882 | 1p13-p11 | Hs01050702_m1 |
| PIK3CA | phosphatidylinositol-4,5-bisphosphate 3-kinase, catalytic subunit alpha | 8975 | 3q26.3 | custom |
| PIK3CB | phosphatidylinositol-4,5-bisphosphate 3-kinase, catalytic subunit beta | 8976 | 3q21-qter | Hs00178872_m1 |
| PIK3IP1 | phosphoinositide-3-kinase interacting protein 1 | 24942 | 22q12.2 | Hs01018206_m1 |
| PIK3R1 | phosphoinositide-3-kinase, regulatory subunit 1 (alpha) | 8979 | 5q13.1 | Hs00381459_m1 |
| POU5F1 | POU class 5 homeobox 1 | 9221 | 6p21.33 | Hs00999632_g1 |
| PTCH1 | patched 1 | 9585 | 9q22.1-q31 | Hs00181117_m1 |
| PTEN | phosphatase and tensin homolog | 9588 | 10q23 | Hs02621230_s1 |
| PTGS2 | prostaglandin-endoperoxide synthase 2 (prostaglandin G/H synthase cyclooxygenase) | 9605 | 1 q25.2-q25.3 | Hs00153133_m1 |
| PTP4A1 | protein tyrosine phosphatase type IVA, member 1 | 9634 | 6q12 | Hs00748591_s1 |
| PTPN1 | protein tyrosine phosphatase, non-receptor type 1 | 9642 | 20q13.1-q13.2 | Hs00182260_m1 |
| RB1 | retinoblastoma 1 | 9884 | 13q14.2 | Hs01078066_m1 |
| RPS6 | ribosomal protein S6 | 10429 | 9p21 | Hs01058685_g1 |
| S1PR1 | sphingosine-1-phosphate receptor 1 | 3165 | 1p21 | Hs00173499_m1 |
| SMO | smoothened, frizzled family receptor | 11119 | 7q32.1 | Hs01090242_m1 |
| SNAI1 | snail homolog 1 (Drosophila) | 11128 | 20q13.2 | Hs00195591_m1 |
| SNAI2 | snail homolog 2 (Drosophila) | 11094 | 8q11.21 | Hs00950344_m1 |
| SP2 | Housekeeping Enzyme: Sp2 transcription factor | 11207 | 17q21.3-q22 | custom |
| SPHK1 | sphingosine kinase 1 | 11240 | 17q25.2 | Hs00184211_m1 |
| SPRY1 | sprouty homolog 1, antagonist of FGF signaling | 11269 | 4q | Hs01083036_s1 |
| SRC | v-src sarcoma (Schmidt-Ruppin A-2) viral oncogene homolog | 11283 | 20q12-q13 | Hs01082246_m1 |
| TIMP1 | TIMP metallopeptidase inhibitor 1 | 11820 | Xp11.3-p11.23 | Hs00171558_m1 |
| TIMP2 | TIMP metallopeptidase inhibitor 2 | 11821 | 17q25 | Hs00234278_m1 |
| TP53 | tumor protein p53 | 11998 | 17p13.1 | custom |
| TP63 | tumor protein p63 | 15979 | 3q27-q29 | custom |
| TP73 | tumor protein p73 | 12003 | 1p36.3 | custom |
| TSC1 | tuberous sclerosis 1 | 12362 | 9q34 | Hs01060648_m1 |
| TSC2 | tuberous sclerosis 2 | 12363 | 16p13.3 | Hs01020387 _ m 1 |
| USP7 | ubiquitin specific peptidase 7 (herpes virus-associated) | 12630 | 16p13.3 | Hs00931763_m1 |
| VEGFA | vascular endothelial growth factor A | 12680 | 6p12 | Hs00900055_m1 |
| VPS33B | Housekeeping Enzyme: vacuolar protein sorting 33 homolog B | 12712 | 15q26.1 | custom |
| WNT2 | wingless-type MMTV integration site family member 2 | 12780 | 7q31 | Hs01128652_m1 |
| ZEB1 | zinc finger E-box binding homeobox 1 | 11642 | 10p11.22 | Hs00232783_m1 |
| ZEB2 | zinc finger E-box binding homeobox 2 | 14881 | 2q22.3 | Hs00207691_m1 |

| | | | | |
|---|---|---|---|---|
| HGNC= HUGO Gene Nomenclature Committee. | | | | |

**Table 2: Oligonucleotide Sequences for Custom TAQMAN® Probes and Primers**

| **Gene Symbol** | **Custom Probe** | **Forward Primer** | **Reverse Primer** |
|---|---|---|---|
| ACVRL 1 | CTGGCTGCAGACCCG GTCCT (SEQ ID NO: 1) | AGGTGGTGTGTGGATCA G (SEQ ID NO: 2) | CCGCATCATCTGAGCTAGG (SEQ ID NO: 3) |
| BCL2 (Alpha Isoform) | TGCACACCTGGATCC (SEQ ID NO: 4) | CCTGTGGATGACTGAGTAC | GGGCCGT ACAGTTCCACAAA (SEQ ID NO: 6) |
| | | CTGAA (SEQ ID NO: 5) | |
| BCL2 (Beta Isoform) | AGGCTGGGTAGGTGC A (SEQ ID NO: 7) | ACCTGCACACCTGGATCCA (SEQ ID NO: 8) | GCCCAGACTCACATCACCAA (SEQ ID NO: 9) |
| BCL2L1 | CGGCTGGGATACTT (SEQ ID NO: 10) | AATGACCACCTAGAGCCTTG GA (SEQ ID NO: 11) | CTCGGCTGCTGCATTGTTC (SEQ ID NO: 12) |
| BID | AGAGGCAGATTCTG (SEQ ID NO: 13) | CACTCCCGCTTGGGAAGAA (SEQ ID NO: 14) | CCTGGCAATATTCCGGATGA (SEQ ID NO: 15) |
| ERBB2 | TTTGGACCGGAGGCT G (SEQ ID NO: 16) | GAGTGTCAGCCCCAGAATG G (SEQ ID NO: 17) | GTGGGCACAGGCCACACA (SEQ ID NO: 18) |
| FGFR3 (C isoform) | TTAGCGCCCGCCGTCT TGAG (SEQ ID NO: 19) | GACGGCACACCCTACGTTA C (SEQ ID NO: 20) | TCTAGCTCCTTGTCGGTGGT (SEQ ID NO: 21) |
| FGFR3 (B isoform) | CGTCCCGCTCCGACA CATTG (SEQ ID NO: 22) | CTCAAGTCCTGGATCAGTGA GA (SEQ ID NO: 23) | GGTGGCTCGACAGAGGTACT (SEQ ID NO: 24) |
| FGFR3 | CCTCGGGAGATGAC (SEQ ID NO: 25) | ACTTCAGTGTGCGGGTGAC A (SEQ ID NO: 26) | CCTCGTCCTCCCCGTCTT (SEQ ID NO: 27) |
| MET | TGTCTGCCTGCAATC (SEQ ID NO: 28) | CGGGACATGGACTCAACAG A (SEQ ID NO: 29) | TGCACTATTTGGGAAAACCTT GT (SEQ ID NO: 30) |
| NOTCH 1 | TCTGCATGCCCGGCTA CGAG (SEQ ID NO: 31) | CACCTGCCTGGACCAGAT (SEQ ID NO: 32) | GTCTGTGTTGACCTCGCAGT (SEQ ID NO: 33) |
| PIK3CA | TTCGACACTCTTCAAG CCTGA (SEQ ID NO: 34) | CCCTGCTCATCAACTAGGAA ACC (SEQ ID NO: 35) | CAATTCAACCACAGTGGCCT TTT (SEQ ID NO: 36) |
| SP2 | TGGAGCAGCTTCCT (SEQ ID NO: 37) | GGCTCGAGACCAGCTCATC TA (SEQ ID NO: 38) | GAGATCTGCCTCAATGAATA AATCC (SEQ ID NO: 39) |
| TP53 | CAGATAGCGATGGTCT G (SEQ ID NO: 40) | GCTGCCCCCACCATGAG (SEQ ID NO: 41) | CCTTCCACTCGGATAAGATG CT (SEQ ID NO: 42) |
| TP63 | ACCTGGACGTATTCC (SEQ ID NO: 43) | GTCGAGCACCGCCAAGTC (SEQ ID NO: 44) | GCAATTTGGCAGTAGAGTTT CTTCA (SEQ ID NO: 45) |
| TP73 | CTGGACGTACTCCC (SEQ ID NO: 46) | CCAGCACGGCCAAGTCA (SEQ ID NO: 47) | CTTGGCGATCTGGCAGTAGA G (SEQ ID NO: 48) |
| VPS33B | AGAAGAGGTCTGGAG AGC (SEQ ID NO: 49) | CCTGCACGTGTCCCAACTG (SEQ ID NO: 50) | GGCACACGTGCTTCTTCTTG (SEQ ID NO: 51) |

### Mutation analyses and next-generation sequencing

Mutation analyses were carried out on genomic DNA extracted from macrodissected FFPE tissues using the QIAAMP® FFPE kit (Qiagen, Valencia, CA) after deparaffinization with ENVIRENE™ (Schleifman et al. PLoS One 9(3): e90761). Mutations in *PIK3CA*, *EGFR*, *KRAS*, *NRAS*, *HRAS*, *FGFR3*, *MET*, *BRAF*, *KIT*, *AKT1*, and *FLT3* were detected using mutation-specific qPCR as described previously (Schleifman et al. PLoS One 9(3): e90761). Next-generation sequencing (NGS) was carried out using ION TORRENT™ next-generation sequencing using the ION AMPLISEQ™ Cancer Hotspot Panel v2 (Life Technologies, Carlsbad, CA) according to manufacturer guidelines (see, e.g., Tsongalis et al. Clin. Chem. Lab. Med. 52(5): 707-714, 2014). Table 3 shows a summary of the results of the NGS mutation analysis of the bladder cancer samples (WT, wild-type; MUT, mutant).

**Table 3: NGS Mutation Analysis of Bladder Cancer Samples**

| **Sample ID** | **Gene** | **NGS call** | **NGS mutation** |
|---|---|---|---|
| HP-32278 | PIK3CA | MUT | H1047R |
| HP-32278 | FGFR3 | MUT | Y373C |
| HP-44236 | ERBB2 | MUT | V842I |
| HP-44242 | KRAS | MUT | G12D |
| HP-50330 | VHL | MUT | P86S |
| HP-50331 | VHL | MUT | P86S |
| HP-50332 | KRAS | MUT | G12A |
| HP-50335 | FGFR3 | WT | |
| HP-50336 | FGFR3 | MUT | S249C |
| HP-50337 | FGFR3 | MUT | S249C |
| HP-50684 | FGFR3 | MUT | S249C |
| HP-50685 | PIK3CA | MUT | Q546R |
| HP-50685 | FGFR3 | MUT | S249C |
| HP-50686 | FGFR3 | WT | |
| HP-50686 | EGFR | MUT | G721S |
| HP-50686 | VHL | MUT | P81S Q96* |
| HP-50686 | KRAS | NC | NC |
| HP-50688 | FGFR3 | WT | |
| HP-50699 | FGFR3 | MUT | S249C |
| HP-50699 | EGFR | MUT | G735S A767V |
| HP-50699 | ERBB4 | MUT | S341L |
| HP-50707 | FGFR3 | WT | |
| HP-50707 | EGFR | MUT | A698T |
| HP-50713 | FGFR3 | MUT | S249C |
| HP-50713 | EGFR | MUT | R108K |
| HP-50713 | PIK3CA | MUT | T1025T |
| HP-50714 | FGFR3 | WT | |
| HP-50715 | FGFR3 | MUT | R248C |
| HP-50715 | EGFR | MUT | H870Y |
| HP-50715 | VHL | MUT | W88* |
| HP-50715 | KRAS | MUT | G12D |
| HP-50722 | PIK3CA | MUT | E542K |
| HP-50722 | FGFR3 | MUT | R248C |
| HP-50727 | KRAS | MUT | G13D |
| HP-50728 | PIK3CA | MUT | R88Q |
| HP-50737 | PIK3CA | MUT | H1047R |
| HP-50737 | FGFR3 | MUT | R248C |
| HP-50737 | KRAS | WT | |
| HP-50737 | VHL | MUT | Q96* |
| HP-50742 | FGFR3 | MUT | Y373C |
| HP-50885 | FGFR3 | MUT | R248C |
| HP-50888 | HRAS | MUT | Q61R |
| HP-50892 | FGFR3 | MUT | Y373C |
| HP-50893 | PIK3CA | MUT | E545K |
| HP-50893 | FGFR3 | MUT | G370C |
| HP-50894 | FGFR3 | MUT | G370C |
| HP-50900 | PIK3CA | MUT | H1047R |
| HP-50900 | FGFR3 | MUT | S249C |
| HP-50906 | HRAS | MUT | Q61K |
| HP-50907 | FGFR3 | MUT | Y373C |
| HP-50908 | PIK3CA | MUT | E542K |
| HP-50912 | FGFR3 | MUT | S249C |
| HP-50914 | FGFR3 | MUT | S249C |
| HP-50918 | FGFR3 | MUT | R248C |
| HP-50920 | FGFR3 | MUT | S249C |
| HP-50922 | FGFR3 | MUT | S371C |
| HP-50923 | PIK3CA | MUT | E545K |
| HP-50923 | FGFR3 | MUT | S249C |
| HP-50927 | FGFR3 | WT | |
| HP-50927 | IDH2 | MUT | R140W |
| HP-50928 | FGFR3 | MUT | G370C |
| HP-50936 | FGFR3 | MUT | S249C |
| HP-50942 | PIK3CA | MUT | E542K |
| HP-50942 | FGFR3 | MUT | S249C |
| HP-50942 | KRAS | MUT | G12V |
| HP-50949 | FGFR3 | MUT | S249C |
| HP-51223 | FGFR3 | MUT | Y373C |
| HP-51223 | IDH2 | MUT | G171D |
| HP-51223 | PIK3CA | MUT | E545K |
| HP-51227 | FGFR3 | MUT | S249C |
| HP-51227 | PIK3CA | MUT | E542K |
| HP-51227 | IDH2 | MUT | G171D |
| HP-51930 | FGFR3 | MUT | K650E |
| HP-52710 | PIK3CA | MUT | K111E |
| HP-52715 | FGFR3 | WT | |
| HP-52716 | FGFR3 | WT | |
| HP-52717 | PIK3CA | MUT | E545K |
| HP-52724 | PIK3CA | MUT | R108H |
| HP-52724 | VHL | MUT | W117* C162Y Q164* |

### FGFR3 immunohistochemistry

Immunohistochemistry (IHC) was performed on 4-µm thick FFPE tissue section using the Ventana DISCOVERY® XT Autostainer platform (Ventana Medical Systems Inc, Tucson, AZ). For the detection of FGFR3, the slides underwent pre-treatment using CC1 extended antigen retrieval followed by staining with anti-FGFR3, clone 15C3 (Genentech) primary antibody diluted to 1 µg/mL and incubated for 60 minutes at room temperature. For amplification of FGFR3 signal, an unconjugated rabbit anti-mouse linker antibody (Jackson Immunoresearch, West Grove, PA) was applied at 1 µg/mL and incubated for 32 minutes at room temperature. This was followed by an anti-rabbit-OMNIMAP™ horseradish peroxidase (HRP) kit (Ventana Medical Systems Inc, Tucson, AZ).

Sections were counter-stained with hematoxylin, dehydrated, cleared and cover-slipped for viewing.

### Cell line viability studies

Bladder cancer cell lines were obtained from the American Type Culture Collection (ATCC, Manassas, VA) or from the Deutsche Sammlung von Mikroorganismen und Zellkulturen GmbH (DSMZ, Braunschweig, Germany). Cell lines were archived at an early passage in the Genentech cell bank and authenticated either by a multiplex short tandem repeat assay or as previously described (O'Brien et al. Clin. Cancer Res. 16(14): 3670-3683, 2010). All cell lines were maintained in RPMI 1640 or DMEM supplemented with 10% fetal bovine serum (Sigma, St. Louis, MO), nonessential amino acids, and 2 mmol/L L-glutamine.

Erlotinib (TARCEVA™) is a selective and potent inhibitor of EGFR. Details of its structure, selectivity, and biological properties have been previously described (Stamos et al. J. Biol. Chem. 277(48): 46265-46272, 2002). Cell viability studies were carried out as previously described (O'Brien et al. Clin. Cancer Res. 16(14): 3670-3683, 2010). Cells were plated in quadruplicate at a density of 2,500 cells per well in 384-well plates in normal growth medium and allowed to adhere overnight. Erlotinib dose-response was determined by treating with 10 concentrations based on a 3-fold dilution series starting with a 5uM dose. Cell viability was measured 72 hours later using the CELLTITER-GLO® Luminescent Cell Viability Assay (Promega, Madison, WI). The percent viability at each concentration was calculated compared to the control treated only with DMSO.

### Statistical Analysis

To identify the tissue sample clusters (subgroups), unsupervised hierarchical clustering was performed using Euclidean distance and complete linkage. Nonparametric Mann-Whitney tests (see, e.g., Mann and Whitney, Annals of Mathematical Statistics 18(1): 50-60, 1947) and Bonferroni corrections (see, e.g., Bonferroni, Pubblicazioni del R Instituto Superiore di Scienze Economiche e Commerciali di Firenze 8: 3-62, 1936 and Miller, Simultaneous Statistical Inference, 2nd Ed., Springer Verlag, pages 6-8, 1981) were applied to further identify differentially expressed genes among the bladder sample clusters. DFS was defined as the time from the date of surgery to the date of recurrence or death. OS was similarly defined as the time from the date of surgery to the date of death. Survival outcomes were censored at the latest observed time point when the patient was known to be recurrence-free (for DFS) or alive (for OS). Kaplan-Meier estimates (see, e.g., Kaplan and Meier, Journal of the American Statistical Association 53(282): 457-481, 1958) were used to estimate the DFS probability and survival probability over time. The 3-year and 5-year DFS rate and survival rate were also calculated from the Kaplan-Meier DFS/OS curve estimates. For the significance of differential mutation frequency between clusters, Fisher's exact test was performed. Fisher's Exact test (see, e.g., Fisher, Journal of the Royal Statistical Society 85(1): 87-94, 1922) and two-tailed T-tests (see, e.g., Student, Biometrika 6(1): 1-25, 1908) were used as indicated for statistical comparisons.

### Example 2: Development of a custom microfluidics-based bladder cancer gene expression panel and its application in stratifying archival bladder cancer clinical samples

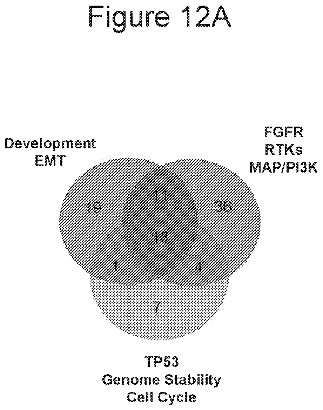

While genomic analyses have provided valuable insights into the molecular underpinnings of bladder cancer, the majority of previous studies rely on the use of frozen tissues that yield high-quality nucleic acids and are generally not well-suited for characterization of archival specimens from clinical trials. However, archival specimens from clinical trials are often associated with accompanying treatment information, disease-free survival (DFS) and overall survival (OS) information, and other information that makes them an excellent platform for cancer genomic analysis. Here we developed a microfluidics-based bladder cancer gene expression panel that is optimized for the analysis of formalin-fixed, paraffin-embedded tissues. The custom bladder cancer Fluidigm panel is comprised of 96 genes that were selected to capture key attributes of bladder cancer biology (see Table 1). The custom bladder cancer Fluidigm panel described in Table 1 includes two housekeeping genes for data quality control and normalization, along with 91 unique bladder cancer-relevant genes (see Figure 12A).

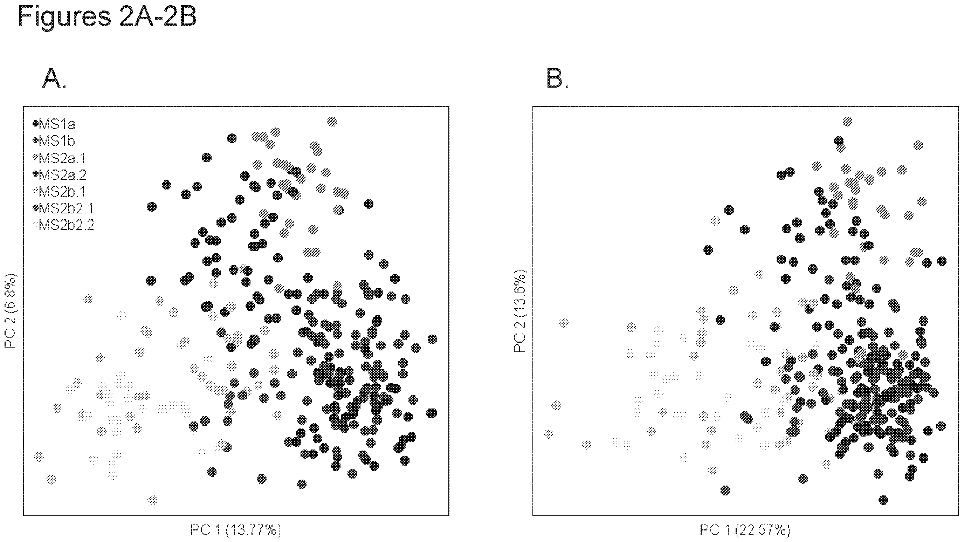

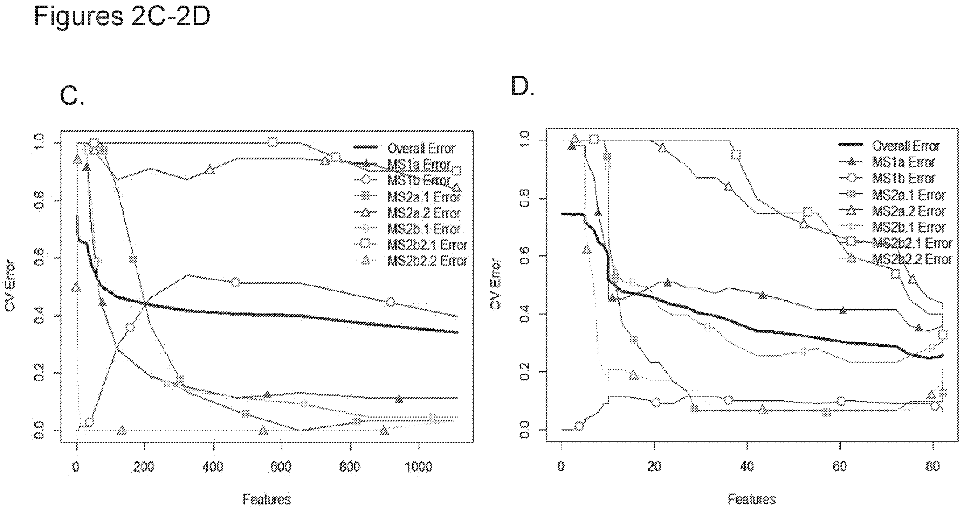

We assessed the ability of the custom bladder cancer Fluidigm panel to successfully identify bladder cancer subtypes in a large, publically-available dataset (Sjödahl et al. Clin. Cancer Res. 18(12): 3377-3386, 2012). Comparison of Principal Component Analysis (PCA) plots based on the expression of 18,000 genes from Sjödahl et al. (*supra*) to PCA plots using 82 overlapping genes from the custom bladder cancer Fluidigm panel showed comparable patterns of sample segregation (Figures 2A-2B). Next, we conducted Diagonal Linear Discriminant Analysis (DLDA) analysis (Diaz-Uriarte et al. BMC Bioinformatics 7: 3, 2006) to assess the accuracy of our panel in correctly classifying bladder cancer from the Sjödahl study. We found that approximately 200 out of 18000 genes from Sjödahl et al. (*supra*) were needed to correctly classify most subtypes reported in their study with >80% accuracy (Figure 2C). In comparison, as few as 20 genes from the bladder cancer Fluidigm panel correctly classified the Sjödahl dataset with >80 % accuracy (Figure 2D). This indicated that the carefully-selected genes on the panel capture the key transcriptional classes of the disease with a high degree of accuracy.

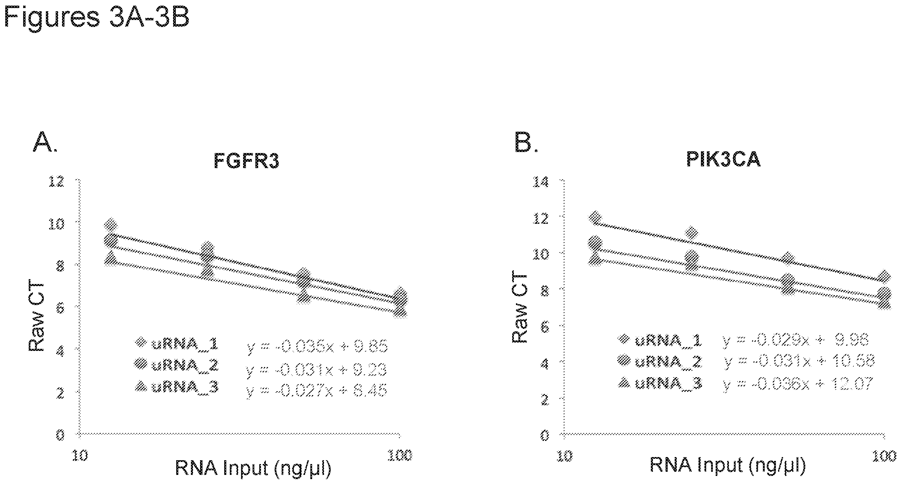

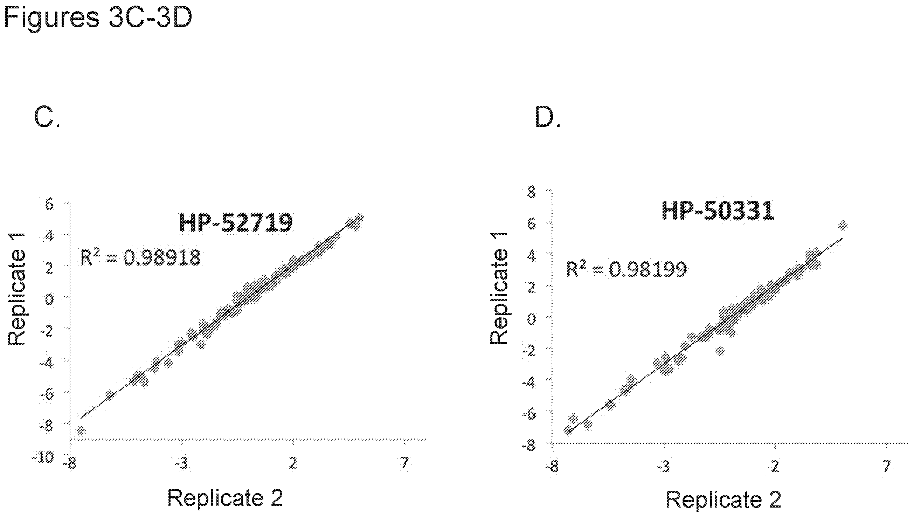

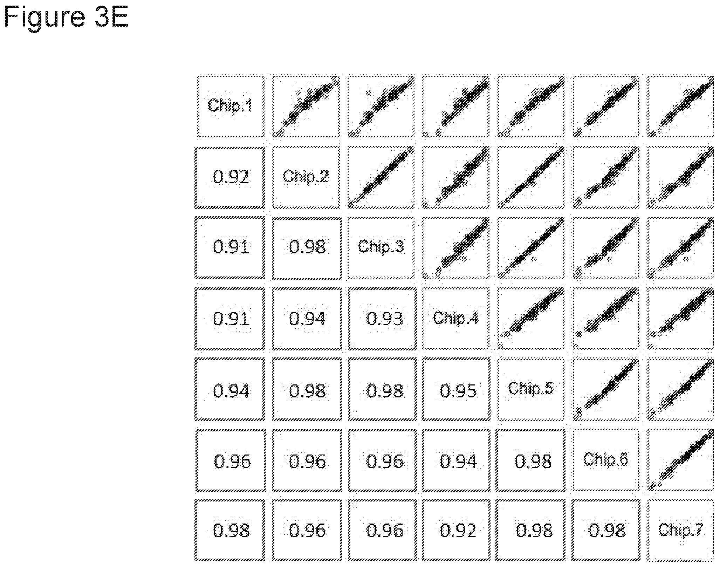

To confirm the robustness and reproducibility of the custom bladder cancer Fluidigm panel in measuring gene expression in FFPE samples, we conducted a series of quality control experiments (Figures 3A-3E). First, we carried out serial dilutions to evaluate the performance of each of the 96 assays, redesigning assays when necessary, to achieve linear standard dilution curves for all tests (Figures 3A and 3B). Secondly, we ran sets of FFPE-derived RNA samples and observed a high concordance in results for replicate samples run on different days (Figures 3C and 3D; R² > 0.98). Thirdly, we noted a high degree of chip-to-chip data reproducibility for control RNA samples that we included in each seven independent runs (Figure 3E; R² > 0.92). Altogether, the results from our qualitative and quantitative assessments indicate that the custom bladder cancer Fluidigm panel provides a robust and biologically-relevant platform for transcriptionally stratifying bladder cancers.

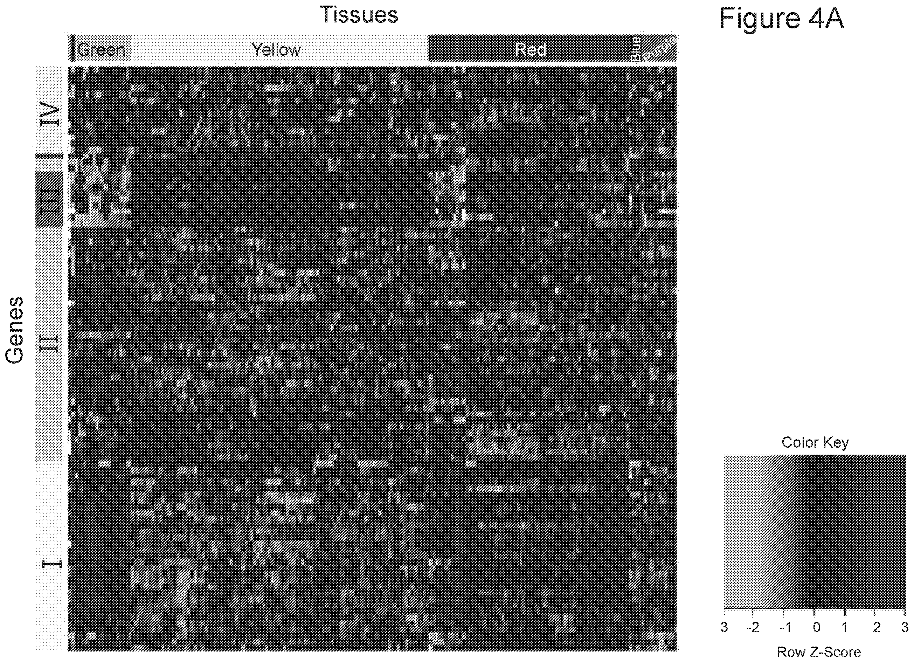

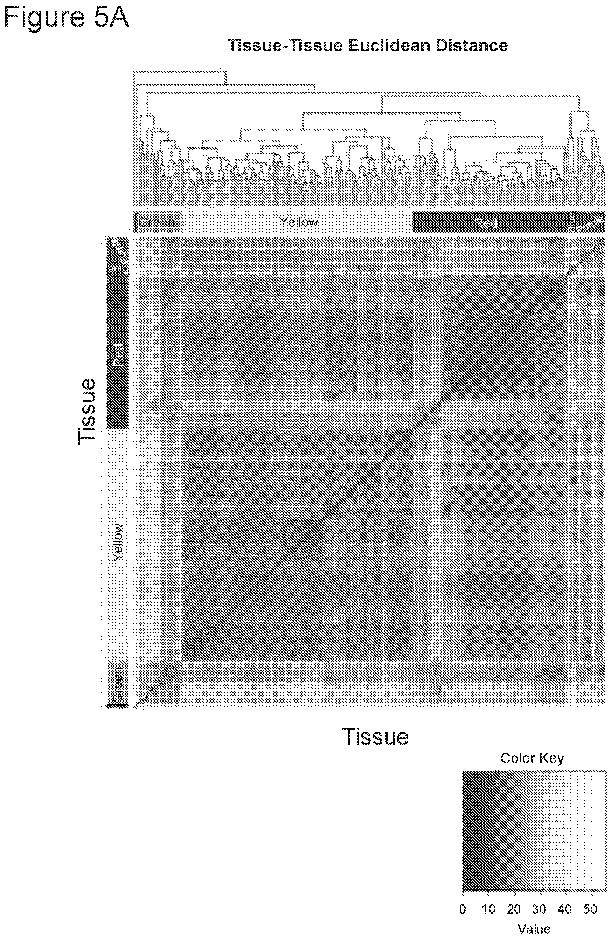

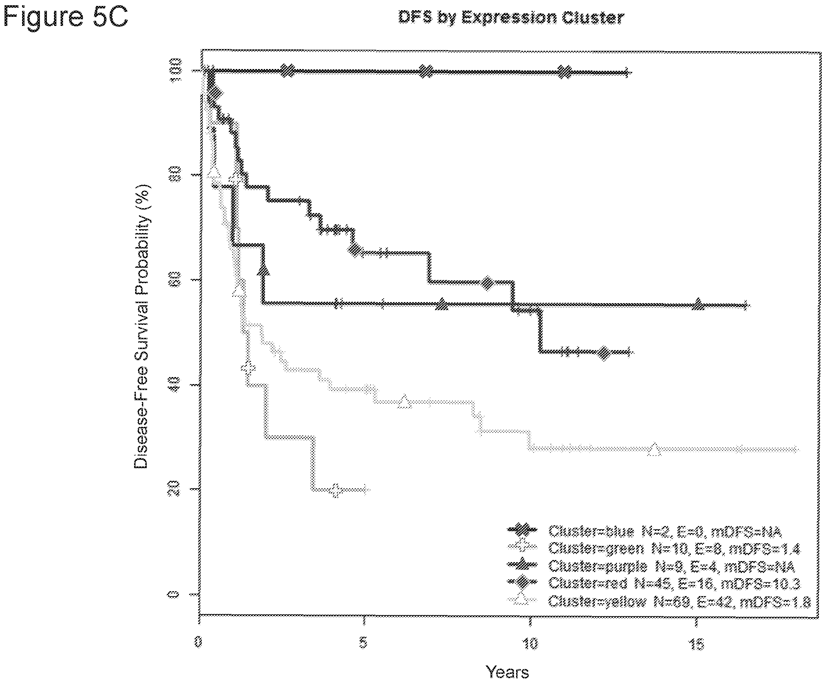

We employed the custom bladder cancer Fluidigm panel in the analysis of a set of 204 FFPE bladder cancer tissues (see Example 1 and Figure 1). Unsupervised hierarchical analysis of Fluidigm gene expression data revealed five transcriptionally-defined bladder cancer tissue clusters that were associated with distinct DFS probabilities (Figures 4A-4F, Figures 5A and 5C). Driving the tissue clustering were five main groups of co-regulated genes that included Receptor Tyrosine Kinase (RTK) signaling genes, such as Fibroblast Growth Factor (FGFR) and Epidermal Growth Factor (EGF) pathway members, apoptosis genes that included the tumor suppressor gene TP53, as well as Epithelial-to-Mesenchymal transition (EMT) genes (Figure 5B).

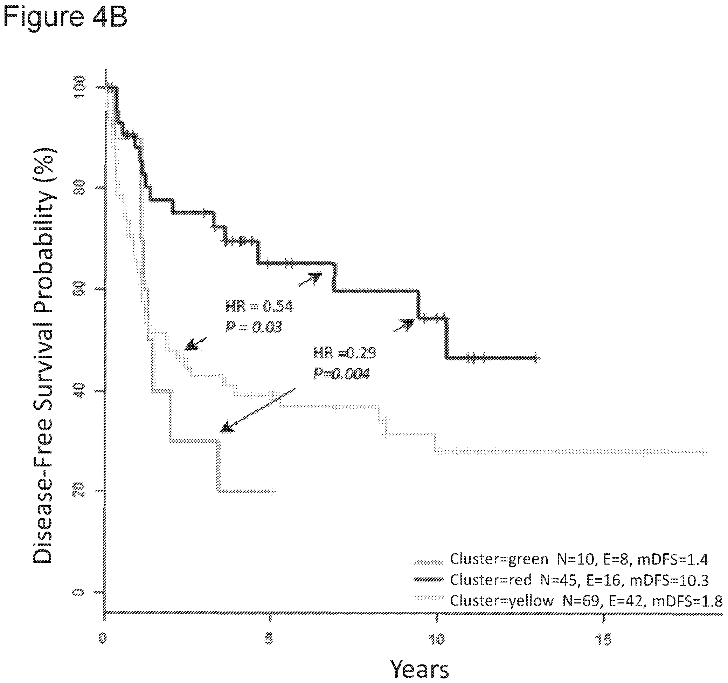

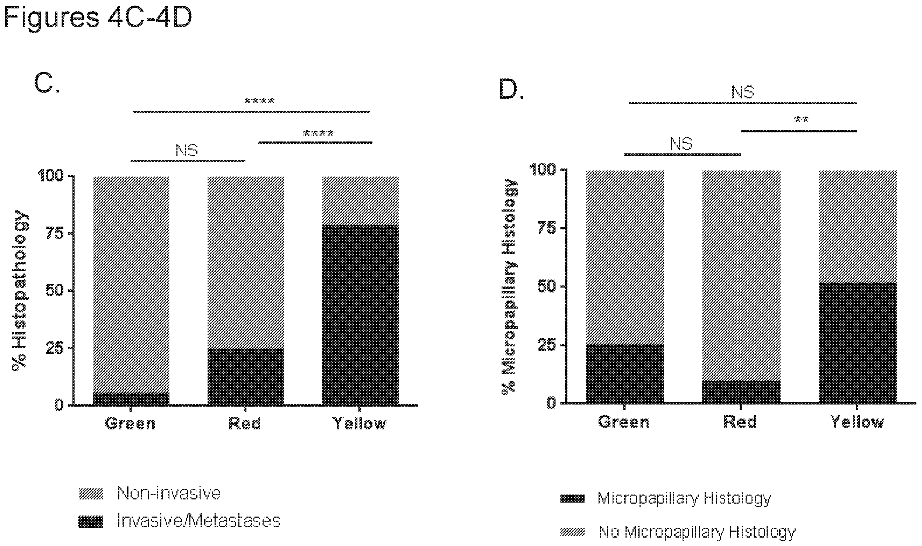

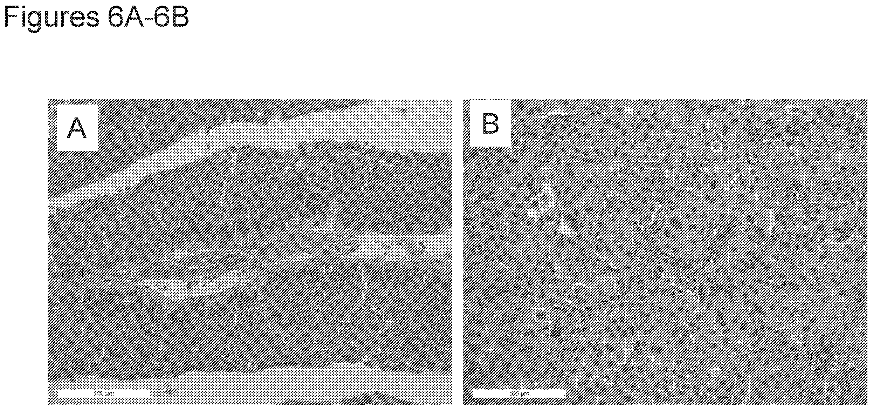

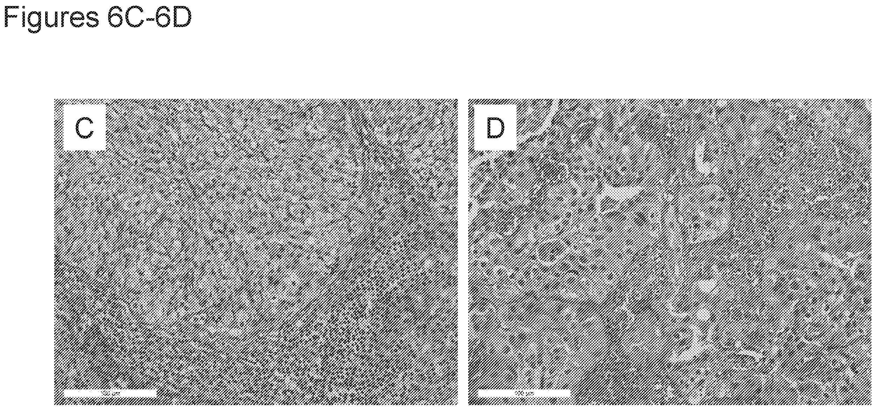

We focused our attention on three main tissue clusters because they included the majority of patient samples and refer to them from this point forward as the Green, Yellow, and Red groups (Figure 4A). Kaplan-Meier analysis revealed that the Red group was associated with better DFS probabilities than the Yellow group (Figure 4B; HR=0.54, P=0.03), and that the Yellow group had a significantly better DFS profiles than the Green group (Figure 4B; HR=0.29, P =0.004). Given the well-established association between bladder cancer histology and patient outcomes (Nargund et al. Semin. Oncol. 39(5): 559-572, 2012; Resnick et al. Curr. Opin. Oncol. 25(3): 281-288, 2013), we next examined the histophathological attributes of the Green, Red, and Yellow tissue groups. Not surprisingly, we observed that the majority of samples in the Red group, which was associated with the best DFS profile, were of the NMIBC histology (Figure 4B and 4C, Figures 6A-D). The Yellow group was comprised of a significantly higher proportion of MIBC cases than the Red group, and the metastatic cases clustered exclusively into this group without any need for supervised analysis (Figure 4C).

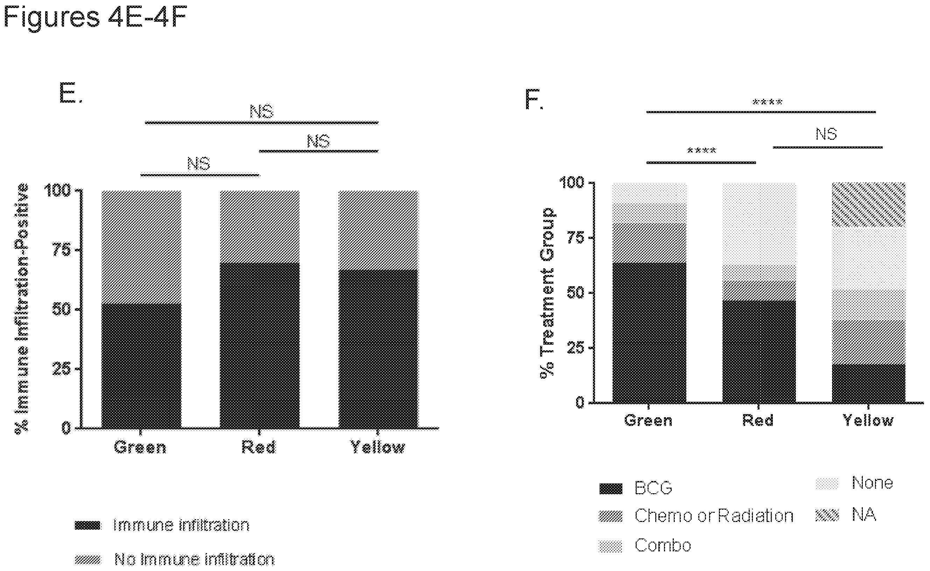

Surprisingly, the Green group, which was associated with the worst DFS likelihoods, was almost entirely comprised of samples of the typically non-aggressive NMIBC histology (Figure 4B and 4C, Figures 6A-D). This was a surprising observation that suggested that the custom Fluidigm panel might have identified a group of patients with rapidly-recurring NMIBCs who might benefit from close clinical monitoring and therapeutic intervention. To exclude the possibility that the Green group might represent an aggressive histological variant, we conducted a comprehensive examination of tissues from the Green, Red, and Yellow group (see, e.g., Figures 4D and 4E). As expected, there was a significantly higher incidence of the pre-invasive micropapillary histology in the MIBC and metastatic Yellow group compared to the NMIBC Red group (Figure 4D; P=0.0063, Figures 6A-6D). However, we did not observe a statistically-significant difference in the incidence of micropapillary histology in the aggressive NMIBC Green group compared to the Red group (Figure 4D; P=0.2351, Figures 6A-6D). We also examined the immune infiltrate that have been reported to be associated with bladder cancer patient clinical outcomes (Otto et al. World J. Urol. 30(6): 875-877, 2012) in the three tissue groups and did not observe a significant difference between them (Figure 4E). There were no significant differences in the tumor stage and grade of samples belonging to the Green and Red groups. Therefore, the transcriptionally-defined Green group does not appear to represent a disease subtype that is histologically different from the favorable outcome Red group.

Next, we determined whether the rapid disease recurrence observed in the Green group could be due to inadequate treatment of this patient population. We examined the frequency of treatment with Bacillus Calmette-Guérin (BCG) vaccine, chemotherapy, or radiation therapy, as well as combinations thereof, in the three transcriptionally-defined groups (Figure 4F). Comparable fractions of patients (>60%) from the NMIBC Red and invasive/metastatic Yellow groups received treatment (Figure 4F; P=0.8836). Patients of the rapidly-recurring NMIBC Green group were treated at a significantly higher rate than both Red and Yellow groups (Figure 4F; P<0.0001 for both comparisons). These results indicated that the rapid recurrence of cases from the Green group was not due to inadequate adjuvant treatment, and that there could be molecular drivers of aggressive tumors from this NMIBC subtype.

Therefore, the custom bladder cancer Fluidigm panel provides a robust platform for stratifying archival bladder cancer tissues. Analysis of the clinically-annotated set of bladder cancer samples on this panel revealed three transcriptionally-distinct bladder cancer subtypes, including a NMIBC Green group that was associated with poor DFS likelihoods.

### Example 3: FGFR3 is hyper-mutated and overexpressed in rapidly-recurrent NMIBCs, and high expression in a subset of invasive and metastatic tumors is associated with poor clinical outcomes

One of the characteristic features of NMIBCs is that they carry somatic activating mutations in Fibroblast Growth Factor Receptor 3 (FGFR3) in approximately 60-70% of cases (van Rhijn et al. J. Pathol. 198(2): 245-251; Tomlinson et al. J. Pathol. 213(1): 91-98, 2007; Martinez-Torrecuadrada et al. Clin. Cancer Res. 11(17): 6280-6290, 2005; Kompier et al. PLoS One 5(11): e13821, 2010; Juanpere et al. Hum. Pathol. 43(10): 1573-1582, 2012; Gust et al. Mol. Cancer Ther. 12(7): 1245-1254, 2013; Cappellen et al. Nat. Genet. 23(1): 18-20, 1999; Ah-Ahmadie et al. J. Pathol. 224(2): 270-279, 2011). The majority of FGFR3 mutations are missense substitutions in the extracellular or juxtamembrane domains that lead to ligand-independent receptor dimerization and subsequent activation (Tomlinson et al. *supra;* Cappellen et al. *supra*)*.* The fact that the mutation frequency in FGFR3 drops to 11-16% in invasive and metastatic disease has lead to uncertainty around whether FGFR3 continues to drive tumorigenesis in advanced disease, or if its tumorigenic role is mostly confined to early stages of bladder cancer development (Tomlinson et al. *supra;* Al-Ahmadie et al. *supra*, Qing et al. J. Clin. Invest. 119(5): 1216-1229, 2009; Liu et al. Genet. Mol. Res. 13(1): 1109-1120, 2014).

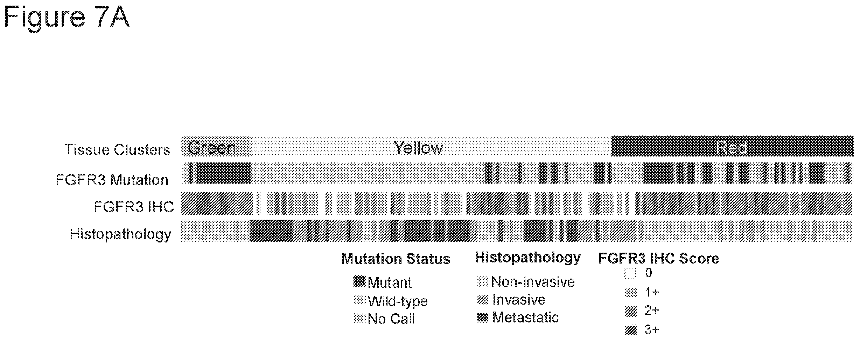

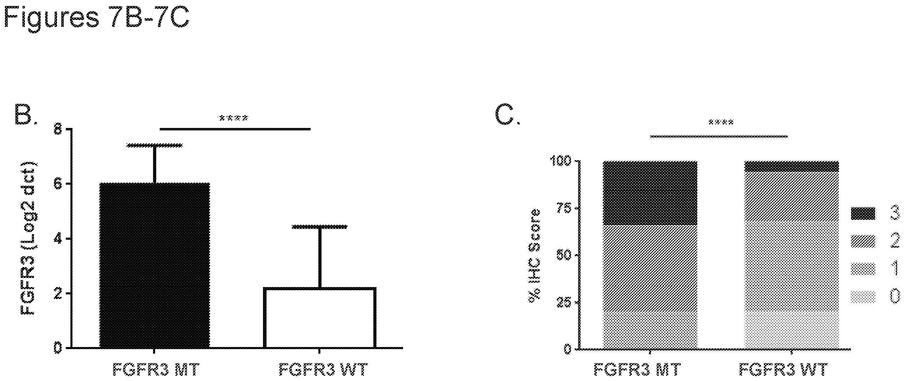

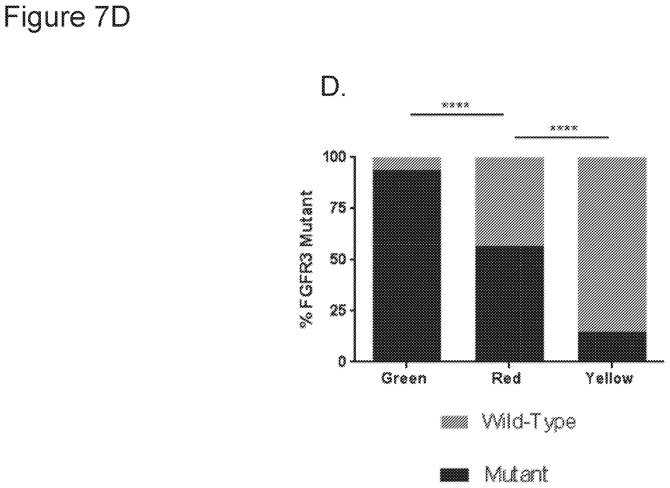

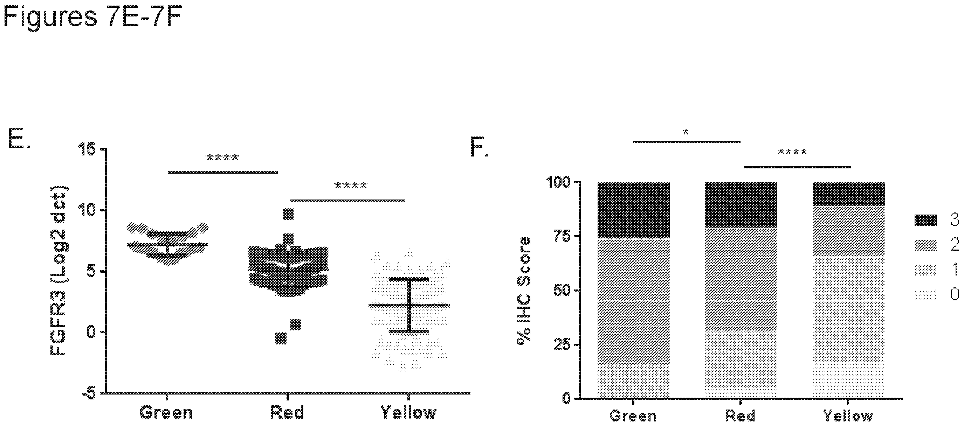

We assessed the mutation status of FGFR3 using a custom multiplex PCR panel that captured the vast majority of known FGFR3 mutations (Schleifman et al. PLoS One 9(3): e90761). Analysis of NMIBC samples of the Red group revealed an expected FGFR3 mutation frequency of approximately 60%, and, as anticipated, samples of the MIBC and metastatic Yellow group exhibited significantly lower FGFR mutation frequency of approximately 15% (Figure 7A). Mutations in FGFR3 have been reported to drive higher expression levels of FGFR3 (Tomlinson et al. *supra*)*.* Consistent with these reports, we detected significantly higher levels of FGFR3 expression in mutant compared to wild-type samples on both the transcriptional and protein levels (Figure 7B and 7C; P<0.0001 for both comparisons). We reasoned that the presence of a high frequency of FGFR3 mutations in the Green group could be a further confirmation that it belongs to the NMIBC subtype. Indeed, we observed frequent FGFR3 mutations in the rapidly-recurring NMIBC green group (Figure 7A). Surprisingly, however, the vast majority of samples from Green group harbored mutations in FGFR3, even at a significantly higher frequency than that observed in the NMIBC Red group (Figures 7A and 7D; P<0.0001). Hyper-FGFR3 mutation in the Green group was associated with significantly higher FGFR expression than in the NMIBC Red and Invasive/Metastatic Yellow groups on both the transcriptional (Figure 7E; P<0.0001 for both comparisons) and protein levels (Figure 7F; P=0.0343 for Green vs. Red, and P<0.0001 for Red vs. Yellow).

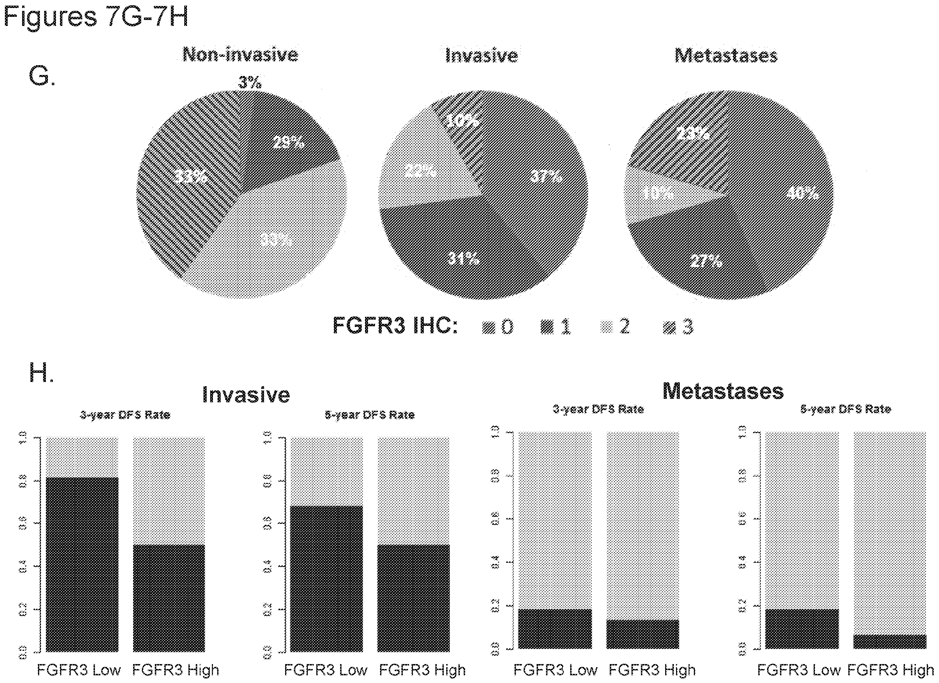

The data described above indicate that hyper-mutation and concomitant overexpression of FGFR3 expression correlates with rapidly-recurring NMIBCs. If FGFR3 continues to act as a cancer driver in later stages of disease, then elevated FGFR3 expression should be maintained during bladder cancer development, at least in a subset of cases. To determine whether this was the case, we examined the expression levels of FGFR3 at both the RNA and protein levels and found that 66% of NMIBCs expressed high levels of FGFR3 as determined by immunohistochemistry (IHC) (Figure 7G). Although the incidence of high FGFR3-expressing cases was reduced in MIBCs and bladder cancer metastases, >30% of tumors from these more advanced stages maintained high expression levels of FGFR3 (Figure 7G, IHC scores 2+/3+), consistent with continued requirement for FGFR3 in advanced disease.

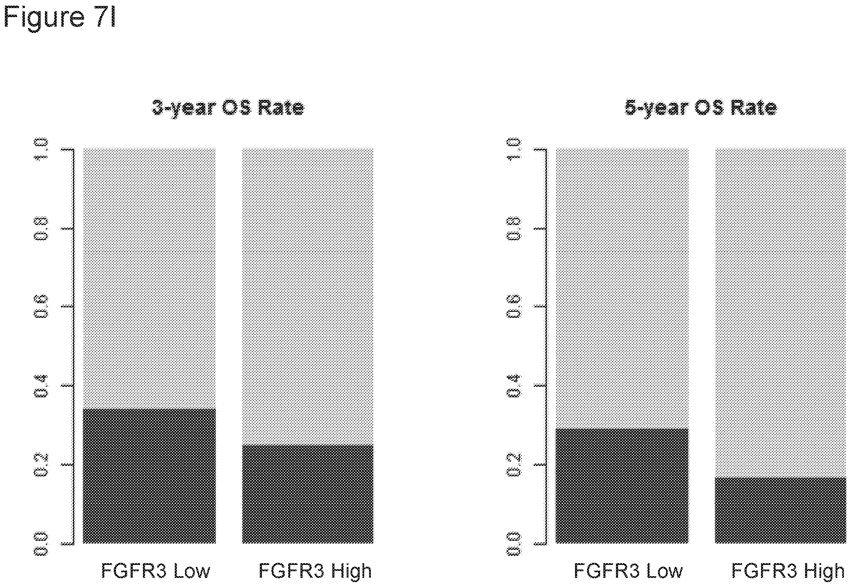

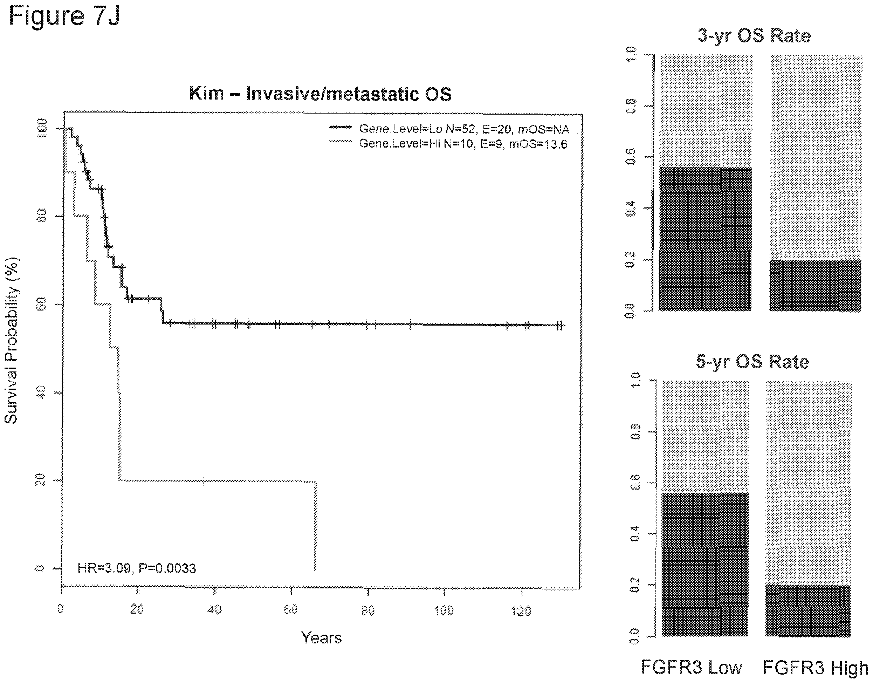

In the overall bladder cancer cohort of our study, we observed favorable DFS and OS survival probabilities for high FGFR3-expressing cases, which is in line with previously published reports (Sjödahl et al. Clin. Cancer Res. 18(12): 3377-3386, 2012; Dyrskjot et al. Nat. Genet. 33(1): 90-96, 2003; Kim et al. Mol. Cancer9: 3, 2010). Unexpectedly, however, high FGFR3 expression in both the MIBCs and metastatic settings was associated with decreased 3- and 5-year DFS probabilities (Figure 7H). Furthermore, high FGFR3 expression in advanced disease (MIBC and metastatic bladder cancer) in our sample series was linked to reduced 3- and 5-year OS likelihoods (Figure 7I). To validate these observations, we examined several public datasets that provided both FGFR3 expression and OS data (Sjödahl et al. Clin. Cancer Res. 18(12): 3377-3386, 2012; Kim et al. Mol. Cancer 9: 3, 2010). Although high levels of FGFR3 conferred a good prognosis in the overall population in those two studies, similar to the observations in our dataset, we noted significantly worse OS likelihoods in high compared to low FGFR3-expressing invasive/metastatic bladder cancers from the study by Kim et al. *supra*, thus providing independent confirmation of our findings (Figure 7J). We also observed a trend for worse OS in FGFR3-high versus -low tumors of advanced stages from the study by Sjödahl et al. (*supra*).

Taken together, these data support a role for FGFR3 as a driver of rapidly-recurrent NMIBCs, as well as a promoter of aggressive disease in the invasive (MIBC) and metastatic settings. Several studies have demonstrated that inhibition of FGFR3 suppresses tumor growth of high FGFR3-expressing bladder cancers *in vitro* and *in vivo* (Martinez-Torrecuadrada et al. Clin. Cancer Res. 11(17): 6280-6290, 2005; Gust et al. Mol. Cancer Ther. 12(7): 1245-1254, 2013; Qing et al. J. Clin. Invest. 119(5): 1216-1229, 2009; Gomez-Roman et al. Clin. Cancer Res. 11(2 Pt 1): 459-465, 2005; Lamont et al. Br. J. Cancer 104(1) 75-82, 2011; Tomlinson et al. Oncogene 26(40): 5889-5899, 2007). Therefore, high-risk bladder cancer patients (for example, patients with rapidly-recurrent NMIBC, MIBC, or metastatic bladder cancer) whose cancers express high FGFR3 represent good candidates for treatment with FGFR3 antagonists.

### Example 4: The tumor suppressor TP53 is mutated and overexpressed in rapidly-recurring NMIBCs

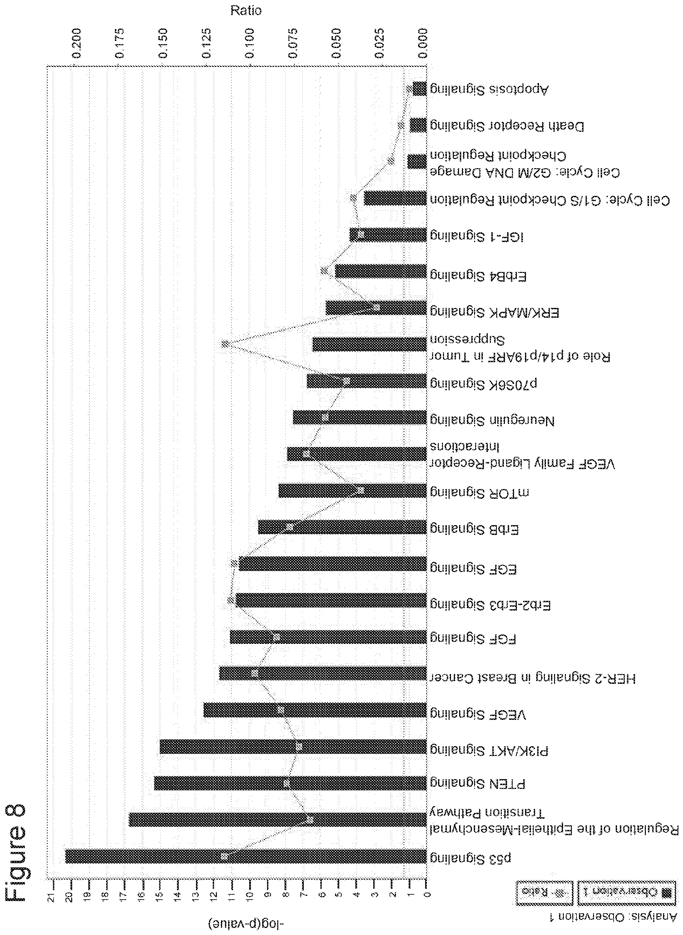

The custom bladder cancer Fluidigm panel identified a transcriptionally-defined aggressive subset of NMIBCs. Comparative expression analysis showed that 45/96 genes (47%) were significantly differentially expressed between the rapidly recurring NMIBC Green and the more benign Red groups (P<0.05, with multiple testing correction). The differentially-expressed genes belonged to the FGFR3 pathway, as described above, and also the tumor suppressor TP53, Avian Erythroblastosis Oncogene B (ERBB), Epithelial-Mesenchymal Transition (EMT), Phosphatidylinositol-3-Kinase and Protein Kinase B (PI3K-AKT) pathways (Figure 8).

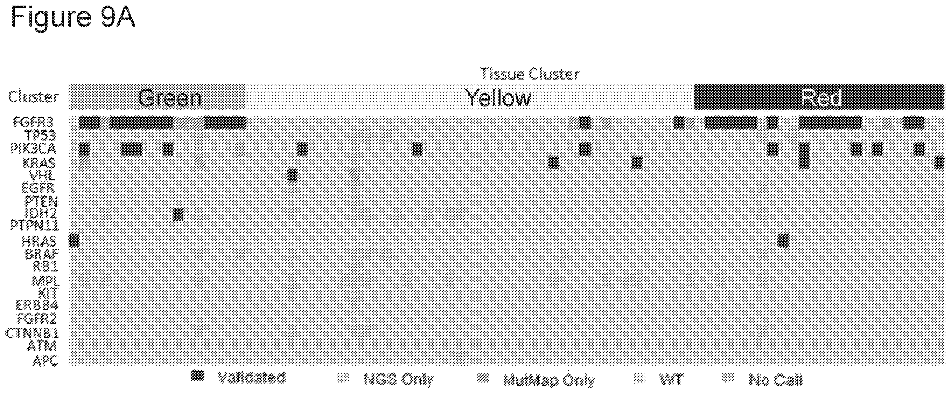

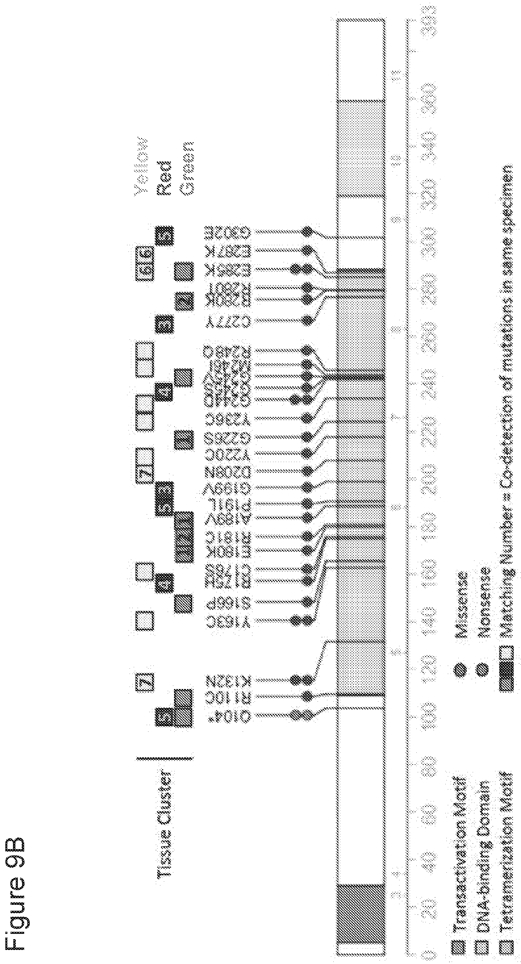

To further characterize our samples on a mutational level, we conduced NGS analysis (Tsongalis et al. Clin. Chem. Lab. Med. 52(5): 707-714, 2014). As expected, NGS data confirmed the presence of FGFR3 mutations described above and presented in Figure 7A (Figure 9A, Table 3). Additionally, NGS analysis revealed mutations in several other genes, including *TP53*, *PIK3CA*, *KRAS*, *VHL*, *EGFR*, *PTEN*, and *IDH2*, among others (Figure 9A, Table 3). Mutations in *TP53* have been reported in invasive and metastatic bladder cancers and to a lesser extent in superficial disease (George et al. J. Clin. Oncol. 25(34): 5352-5358, 2007). We noted the presence of *TP53* mutations in the invasive/metastatic Yellow group, and in a few cases of the NMIBC Red group (Figure 9A). Surprisingly, however, we observed a high frequency of *TP53* mutations in the rapidly-recurring NMIBC Green group (Figure 9A). In all but three cases, the mutations mapped to the DNA-binding domain of TP53, and the TP53 mutation sites in each of the Yellow, Red, and Green groups were mostly non-overlapping (Figure 9B).

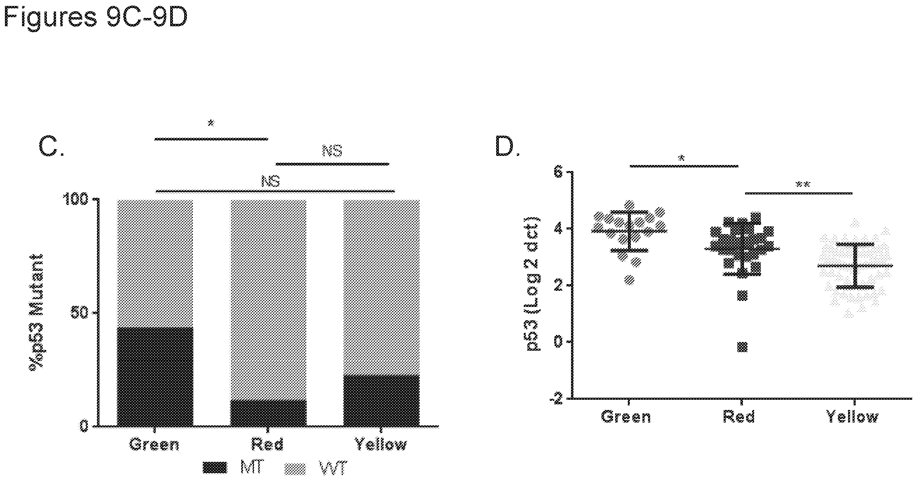

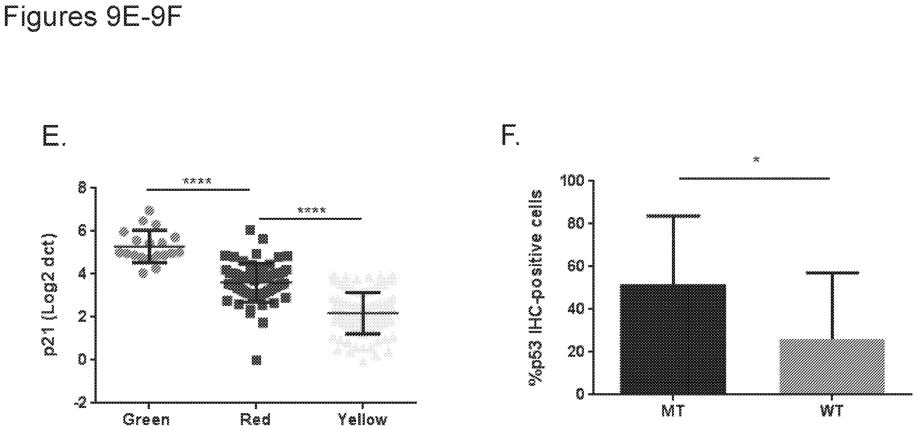

We hypothesized that mutations in *TP53* could be contributing to the poor DFS likelihoods observed in the Green group. Consistent with this, we noted a significantly higher frequency of *TP53* mutations in NMIBCs of the rapidly-recurring Green group compared to the Red group, but not statistically different from that observed in the invasive/metastatic Yellow group (Figure 9C; P=0.03 and P=0.3605, respectively). The expression levels of *TP53* were also significantly higher in the Green than in the Red and Yellow groups (Figure 9D; P=0.0187 and P=0.002, respectively). The expression levels of the TP53 transcriptional target p21 were also significantly higher in the Green compared to the other two groups, further confirming the increased expression levels of *TP53* (Figure 9E; P<0.0001 for both comparisons). We observed significantly higher TP53 protein levels in mutant compare to wild-type samples from the study cohort (Figure 9F; P=0.0201). These findings indicate that the *TP53* gene is mutated and preferentially overexpressed in rapidly-recurring NMIBCs of the Green group.

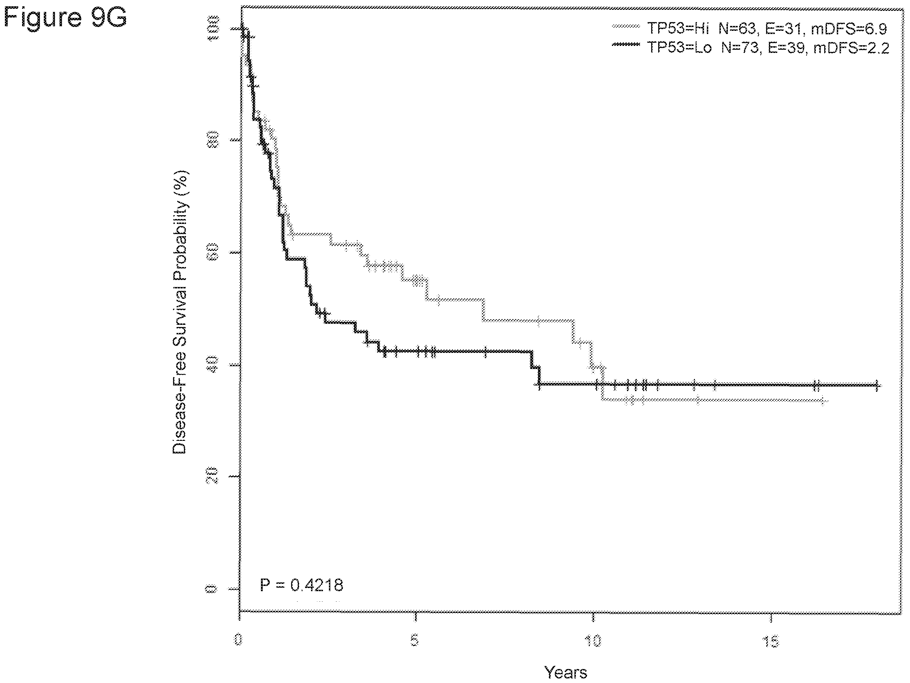

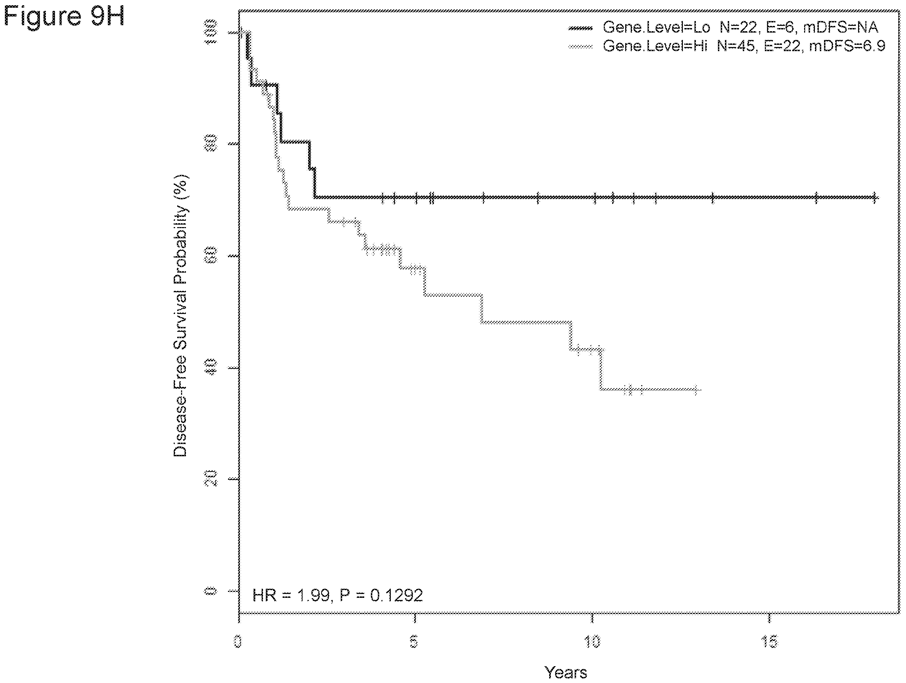

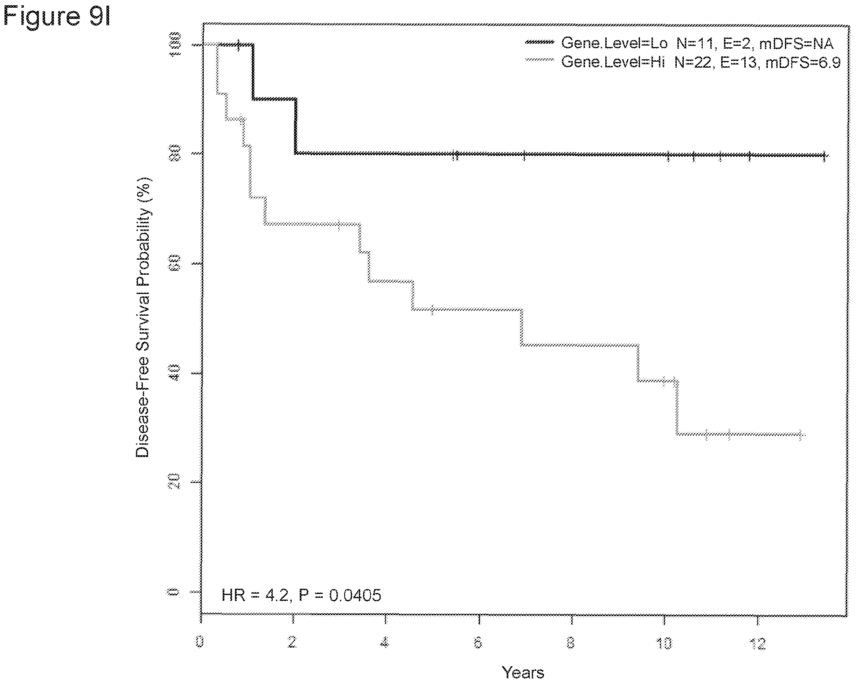

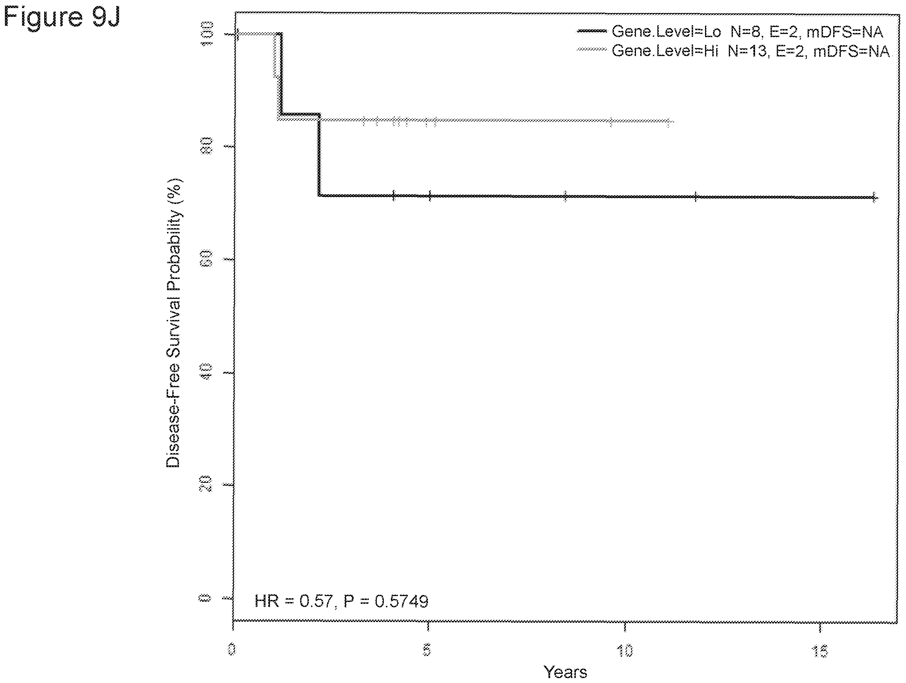

We next determined the clinical impact of TP53 overexpression in our patient cohort. We found that in our overall bladder cancer population tumors with high TP53 expression levels tended to have a more favorable DFS profile than low-expressing tumors (Figure 9G; P=0.4218). Interestingly, this relationship between high TP53 expression and better DFS probabilities was reversed when we looked at TP53 expression in the NMIBC group separately from the other groups (Figure 9H). In the NMIBC group, we found that elevated levels of the tumor suppressor conferred a worse, albeit not statistically significant, DFS likelihood compared to low-expressing cases (Figure 9H; HR=1.99, P=0.1292). A TP53 signature has been shown to be associated with resistance to chemotherapy in the invasive bladder cancer setting (Choi et al. Cancer Cell 25(2): 152-165, 2014). In the NMIBC subset of our study cohort, high TP53 expression was associated with a significantly worse DFS in BCG-treated patients, a treatment commonly administered to bladder cancer patients (Figure 9I; HR=4.2, P=0.0405). This association between high TP53 expression and poor DFS in NMIBCs, however, was not seen in untreated patients (i.e., treatment naive patients) (Figure 9J; HR=0.57, P=0.5749). Our data suggest that a high frequency of TP53 mutation may contribute to aggressive tumor behavior in patient with NMIBCs, and that this could be due, in part, to a counter-productive effect of BCG treatment in this patient population.

### Example 5: High EGFR expression is associated with poor clinical outcomes and confers sensitivity to erlotinib in bladder cancer cell lines

Mutations in *ERBB2* have been reported in tumors of the aggressive micropapillary histology (Ross et al. Clin Cancer Res. 20(1): 68-75, 2014). In our patient cohort, we did not detect *ERBB2* mutations; however, we identified mutations in *EGFR* in tumors from the rapidly-recurring NMIBC Green group but not in tumors from the more benign superficial Red group or from the invasive/metastatic Yellow group (Figure 3A). We conducted a more in-depth genomic analysis of ERBB family ligands and receptors to investigate a potential role for this pathway as driver of bladder cancer (Figure 10). We observed EGFR copy number gains in 2/39 (∼5%) and ERBB2 in 4/39 (∼10%) of samples from the Yellow group, as well as ERBB2 copy number gains in 1/30 (∼3%) samples from the Red group (Figure 10). These findings are consistent with previous reports (Weinstein et al. Nature 507(7492): 315-322, 2014; Capellen et al. Nat. Genet. 23(1): 18-20, 1999), and indicate that the pathway could play a role in bladder carcinogenesis.

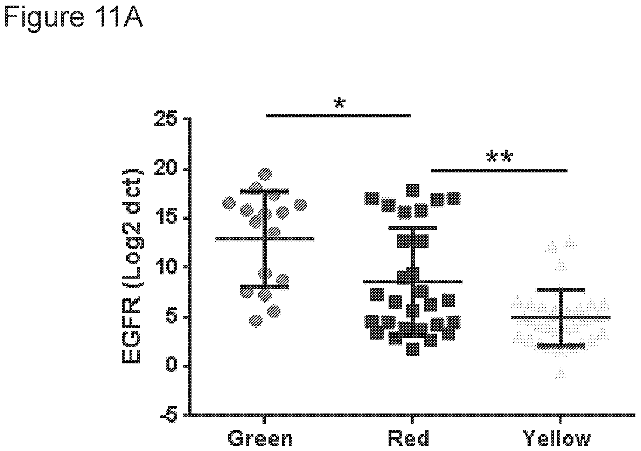

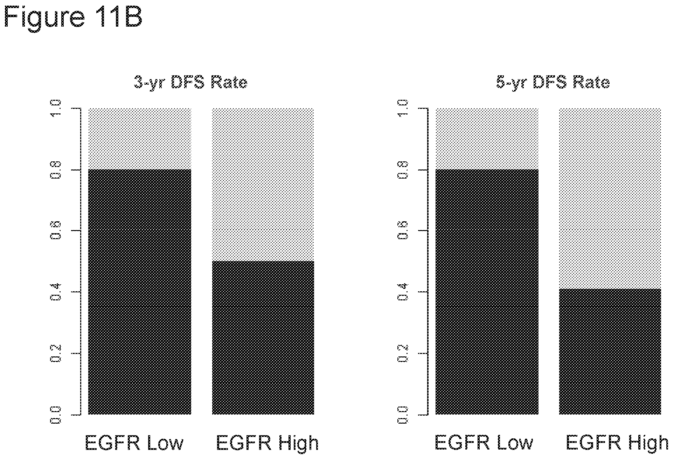

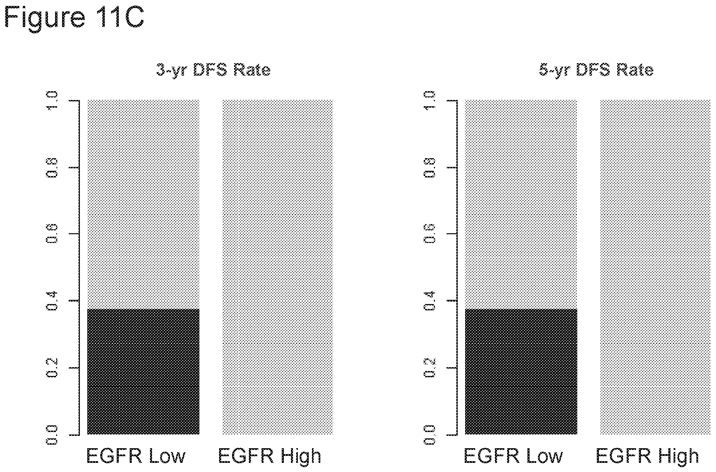

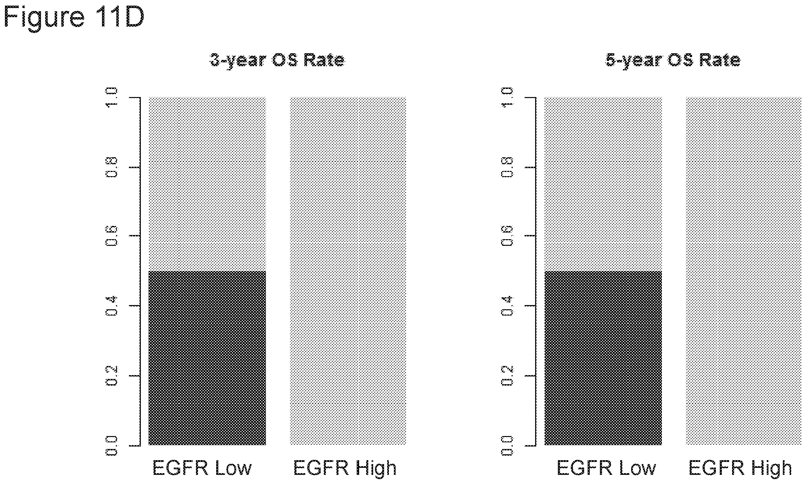

Although we did not detect DNA copy number alterations in ERBB family members in the rapidly-recurring NMIBCs, Fluidigm data analysis revealed that expression levels of EGFR were significantly higher in the rapidly-recurring NMIBC Green group compared to both the more benign NMIBC Red group as well as samples from the Invasive/metastatic Yellow group (Figure 11A; P=0.012 and P=0.0012, respectively). To investigate the possibility that EGFR could be contributing, at least in part, to the aggressive bladder cancer behavior in the NMIBCs, we examined the DFS probabilities of patients whose tumors expressed high versus low levels of EGFR. In the non-invasive setting, patients with NMIBCs that expressed high levels of EGFR had reduced 3- as well as 5-year DFS likelihoods compared to patients with low-expressing malignancies (Figure 11B). In the advanced metastatic setting, high EGFR expression levels were also associated with decreased 3- and 5-year DFS probabilities (Figure 11C). Furthermore, high EGFR expression in patients from our cohort with metastatic disease was associated with unfavorable 3- and 5-year OS likelihoods compared to low-expressing cases (Figure 11D).

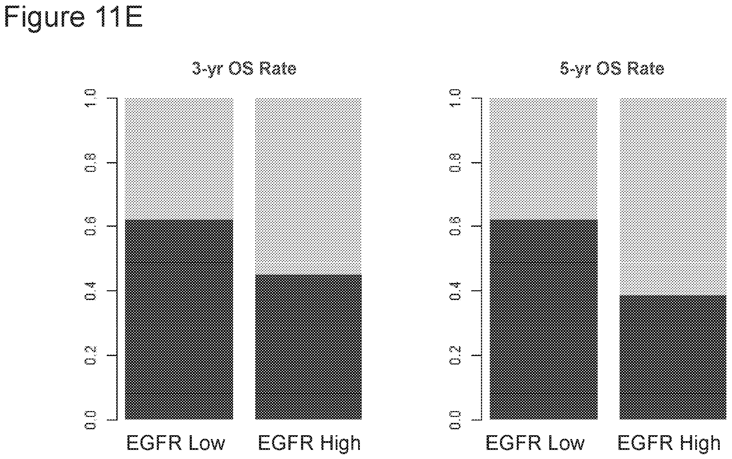

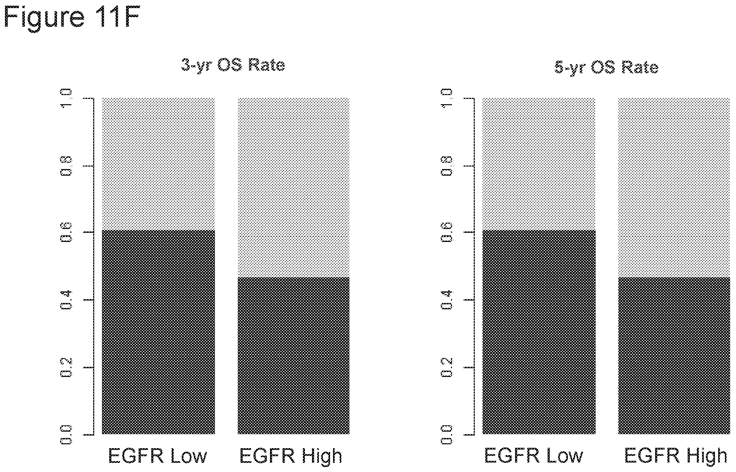

To validate these findings, we assessed the prognostic value of EGFR expression levels in two independent datasets (Sjödahl et al. Clin. Cancer Res. 18(12): 3377-3386, 2012; Kim et al. Mol. Cancer 9: 3, 2010). In invasive/metastatic cases from these two studies, we observed that high EGFR expression was accompanied by reduced OS probabilities, both at 3- and 5-year follow-up timeframes (Figures 11E and 11F). Thus, data from our cohort as well as that from two independent studies suggest that high EGFR expression could be contributing, at least in part, to aggressive behavior of bladder cancers.

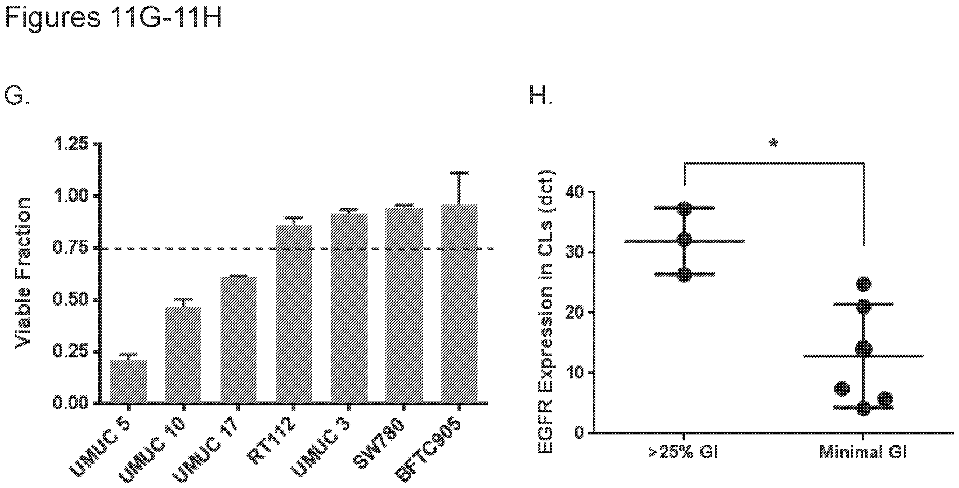

To assess EGFR expression levels as a predictive biomarker for response to anti-EGFR therapies in bladder cancer, we screened a panel of bladder cancer cell lines for sensitivity to the EGFR small molecule inhibitor erlotinib (Stamos et al. J. Biol. Chem. 277(48): 46265-46272, 2002). The UMUC-5, UMUC-10 and UMUC-17 cell lines exhibited varying degrees of sensitivity to erlotinib, ranging from >75% growth inhibition (GI) for UMUC-5 to approximately 40% GI for UMUC-17 (Figure 11G). On the other hand, four cell lines (RT-112, UMUC-3, SW780, and BFTC905) exhibited minimal GI in response to erlotinib treatment (Figure 11G). Next, we examined the levels of EGFR expression in our cell lines. The cell lines that were sensitive (>25% GI) expressed significantly higher levels of EGFR compared to insensitive bladder cancer cell lines (minimal GI of <25%) (Figure 11H; P=0.0106). These results indicate that the expression level of EGFR in a bladder cancer predicts its sensitivity to EGFR pathway inhibitors (e.g., erlotinib).

### Example 6: Custom bladder cancer Fluidigm panel accurately classifies bladder cancer samples into basal and luminal subtypes

This example describes additional development and *in silico* validation of the custom bladder cancer Fluidigm panel for transcriptional analysis of bladder cancer, including archival FFPE bladder cancer clinical samples. As described in Examples 1 and 2, the genes of the panel include components of receptor tyrosine kinase (RTK) pathways such as FGFR, ERBB, MET, PI3K/AKT, and MAPK axes; cell cycle and genome stability genes like TP53; as well as genes involved in the regulation of cell differentiation and development, and epithelial-mesenchymal transition (EMT) (Figure 12A).

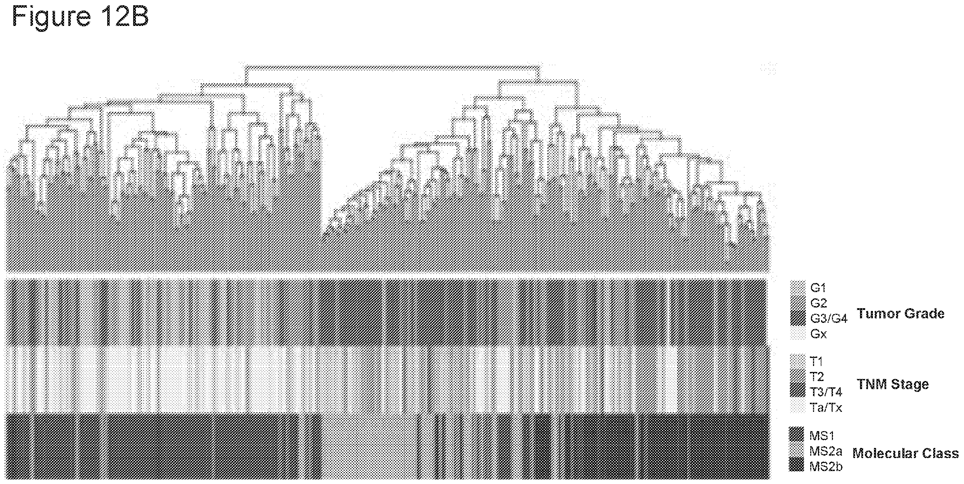

*In silico,* we assessed the ability of the custom bladder cancer Fluidigm panel (see Table 1) to capture key molecular and histological attributes of samples from a large public dataset (Sjödahl et al. Clin. Cancer Res. 18(12): 3377-3386, 2012). Unsupervised hierarchical clustering of samples based on the expression of 125 probes that correspond to the genes in the custom bladder cancer Fluidigm panel revealed two main branches (Figure 12B). The left branch was enriched for low grade (G1/G2) tumors, cancers of the T1 stage, and the MS1 molecular class, as defined by Sjödahl et al. (*supra*) (Figure 12B). Under the right branch we noted a preponderance of G3/G4 tumors, and enrichment for T3/T4 malignancies, co-clustering of MS2a class of bladder cancers, and co-segregation of MS2b tumors from the Sjödahl-defined molecular subtype (Figure 12B).

Next, we calculated the misclassification error rate for tumor grade, TNM stage, and transcriptional classes, as defined by Sjödahl et al. (*supra*), using either all 24,394 Illumina probe sets or using the 125 probe sets that are overlapping with the content of custom bladder cancer Fluidigm panel, and determined the predictive capabilities of diminishing gene subsets when used in a centroid classifier and evaluated through cross-validation (Tibshirani et al. Proc. Natl. Acad. Sci. USA 99:6567-6572, 2002) (Figure 12C). We found comparable misclassification error rates of approximately 40% for tumor grade and TNM stage, and an approximately 20% misclassification error frequency for the MS1, MS2a, and MS2b classes (Figure 12C). A similar increase in the misclassification error rates was observed in both cases as the number of probes was reduced (Figure 12C). These results suggest that the genes of the custom bladder cancer Fluidigm panel capture molecular classes with a high degree of accuracy, as well as tumor grade and TNM stage with similar effectiveness as using the entire content of the Illumina microarray used in Sjödahl et al. (*supra*).

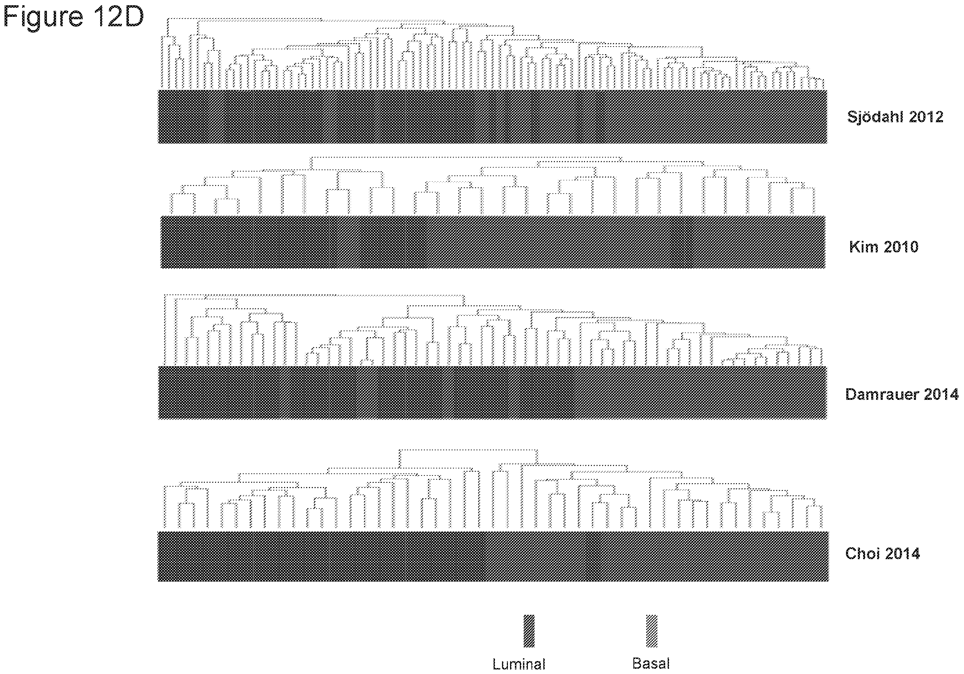

Given the biological and clinical relevance of the recently described basal and luminal subtypes of bladder cancer (Damrauer et al. Proc. Natl. Acad. Sci. USA 111:3110-3115, 2014; Choi et al. PLoS One 7:e30206, 2012), we examined the ability of the custom bladder cancer Fluidigm panel to distinguish between these two molecular subgroups in several public datasets. Using the basal/luminal assignments determined by Damrauer et al. (*supra*) and Choi et al. (Cancer Cell 25:152-165, 2014) in their discovery and validation cohorts, we utilized an unsupervised approach to hierarchically cluster samples based on the expression of probes corresponding to genes in the bladder cancer Fluidigm panel (Figure 12D). This approach correctly classified 39/44 (80%) of the luminal and 42/47 (89%) of the basal samples from the Sjödahl et al. (*supra*) dataset, and 10/12 (83%) of the luminal and 17/18 (94%) of the basal tumors from the Kim cohort (Kim et al. Molecular Cancer 9:3, 2010) (Figure 12D). Furthermore, this approach accurately classified specimens from the Damrauer discovery set (Damrauer et al., *supra*) into luminal and basal subtypes in 33/33 (100%) and 23/28 (82%) of the cases, respectively (Figure 12D). Finally, the genes in the custom bladder cancer Fluidigm panel were able to correctly classify samples based on luminal and basal status from the Choi discovery dataset (Choi et al. 2012, *supra)* in 23/24 (96%) and 23/23 (100%) instances, respectively. In summary, the findings from this *in silico* analysis demonstrate that the carefully selected genes in the custom bladder cancer Fluidigm panel accurately detect basal/luminal status in four public datasets.

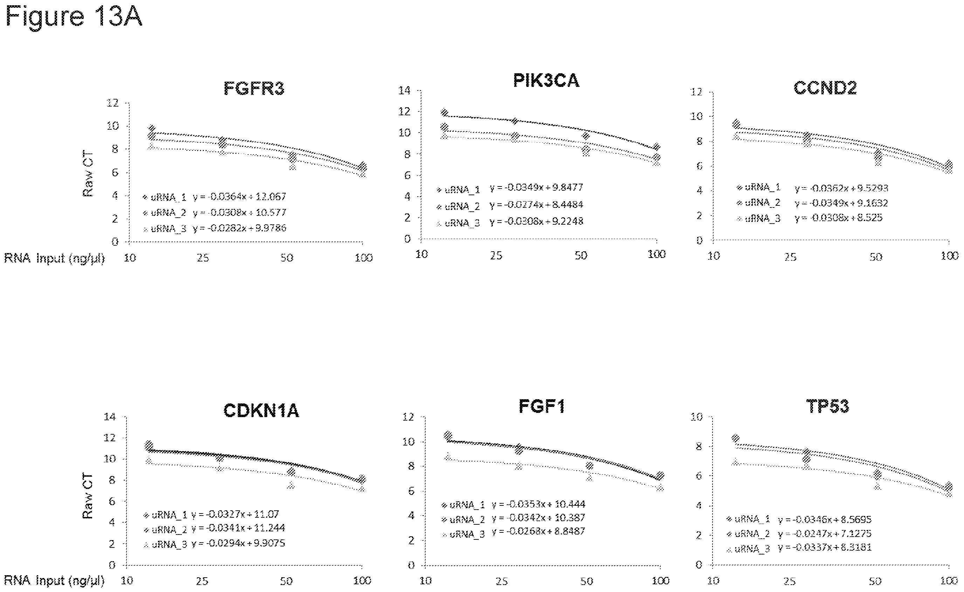

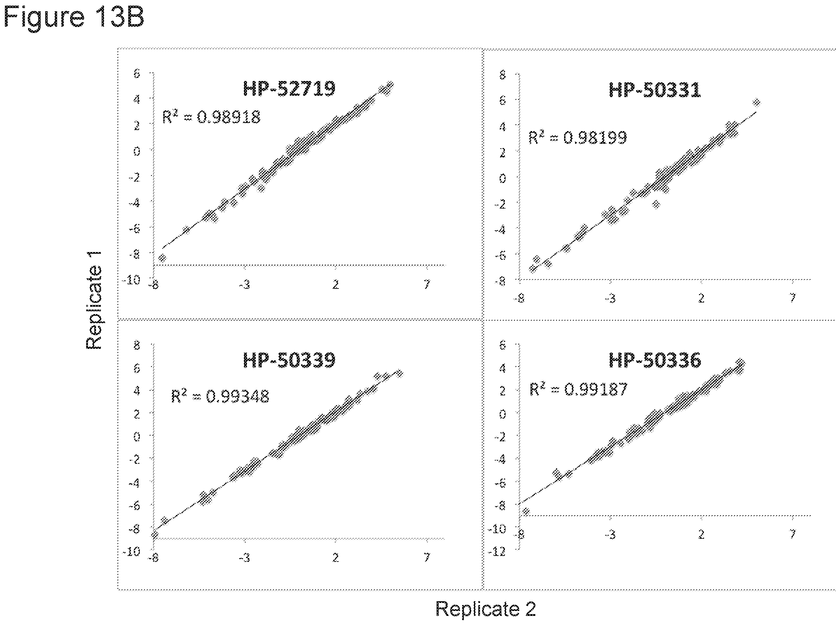

To assess the robustness of the custom bladder cancer Fluidigm panel and reproducibility in measuring gene expression in FFPE samples, we conducted a series of quality control experiments. First, we carried out serial dilutions to evaluate the performance of each of the assays, redesigning primers when necessary, to achieve linear standard dilution curves for all tests (Figure 13A). We also ran sets of FFPE-derived RNA samples and observed a high concordance in normalized expression of each gene on the panel from replicate samples run on different days (Figure 13B, R² range = 0.98-0.99). Finally, we noted a high degree of chip-to-chip data reproducibility for a universal human RNA control that was included in each of seven independent runs (Figure 3E, R² range = 0.91-0.98).

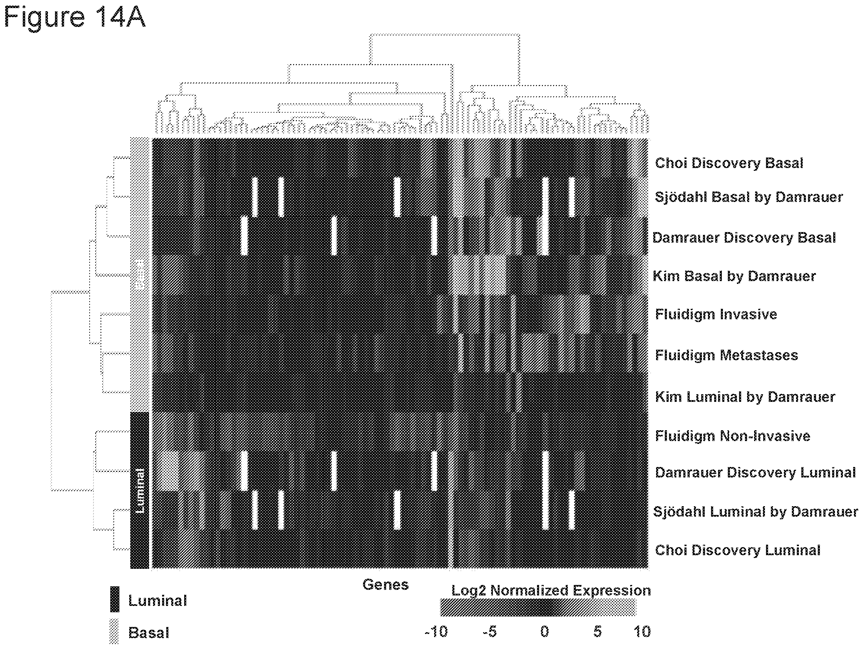

Altogether, the results from our qualitative and quantitative assessments indicate that the custom bladder cancer Fluidigm panel is technically robust and can be used for transcriptionally stratifying FFPE bladder cancer tissues. To further demonstrate the utility of the custom bladder cancer Fluidigm panel for transcriptional characterization of FFPE tissues, we analyzed the set of 204 samples comprised of primary NIMBCs, MIBCs, as well as lymph node and distal METs, described above in Example 1 and Table 1. We examined how these samples compared to those from several public datasets with respect to basal/luminal transcriptional features. As expected, unsupervised hierarchical clustering of samples from four public datasets (Damrauer et al. 2014, *supra;* Kim et al. 2010, *supra*; Sjödahl et al. 2012, *supra*; and Choi et al. 2014, *supra)* using median centered expression from probes corresponding to genes on our panel revealed a clear separation of basal and luminal samples (Figure 14A). Notably, Kim luminal samples clustered with the Basal samples from the three other datasets and, thus, were outliers in this analysis (Figure 14A). We observed that NMIBCs in the bladder cancer cohort described in Example 1 and Figure 1 clustered with luminal samples, while MIBCs and METs appeared to be more basal and clustered alongside with basal samples from the public datasets (Figure 14A).

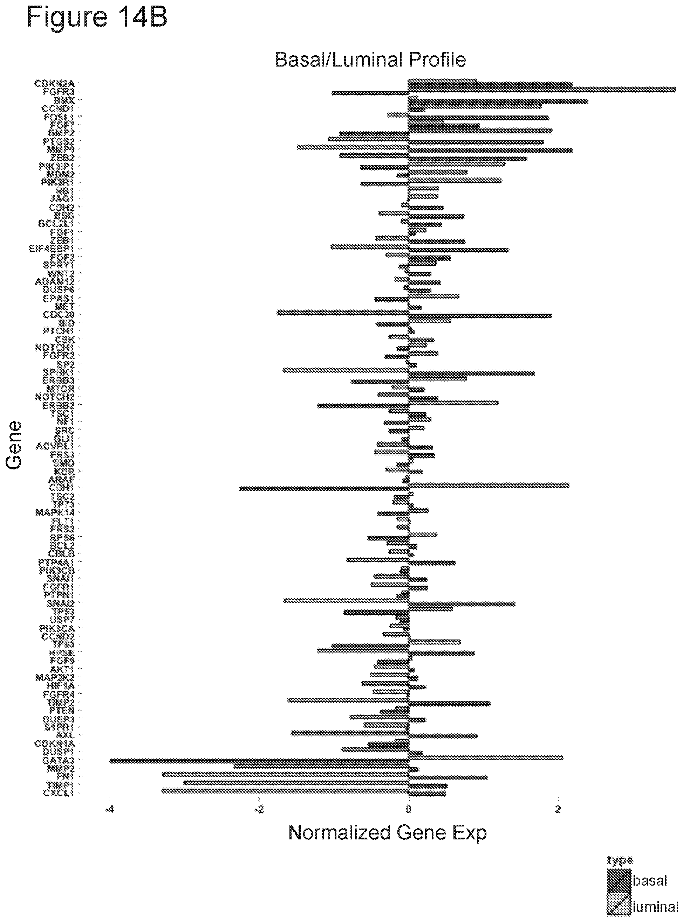

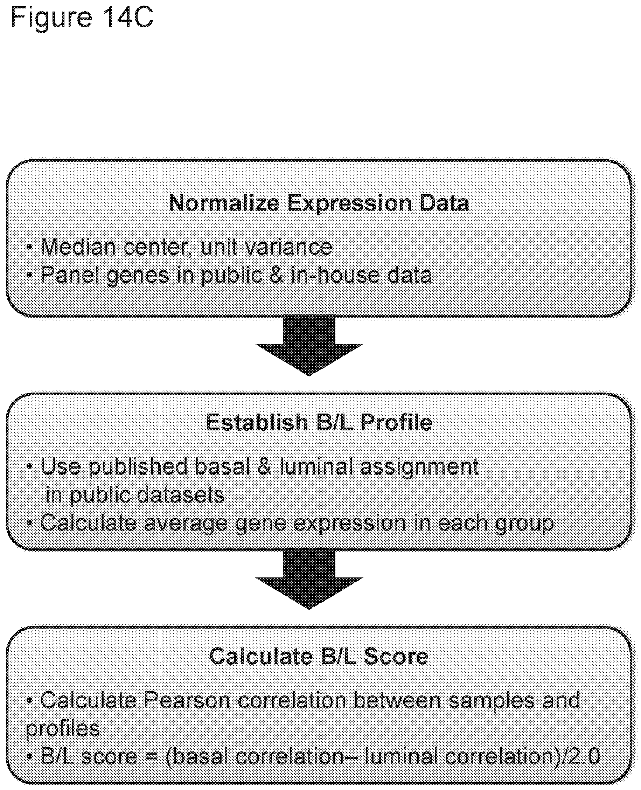

To further evaluate the basal/luminal (B/L) transcriptional signatures of the 204 FFPE samples described in Example 1 and Figure 1, we next established a method for calculating B/L similarity scores for each of these tumors. First, we median-centered the expression of all probes corresponding to the genes of the custom bladder cancer Fluidigm panel in public datasets and calculated an average gene expression value in basal and in luminal groups, as assigned by Damrauer et al. (*supra*) and Choi et al. (*supra*) (Figure 14B). This allowed us to obtain an average view of the expression of each of these genes in the luminal and basal groups from the public datasets (Figures 14B and 14C). For example, *FGFR3* had the highest average expression value in luminal samples than any other gene on our panel, and the average *FGFR3* expression level was one of the lowest in basal samples from the public datasets (Figure 14B). We then derived a B/L similarity score for each FFPE sample by calculating the correlation to the public basal and luminal profiles (Figure 14C).

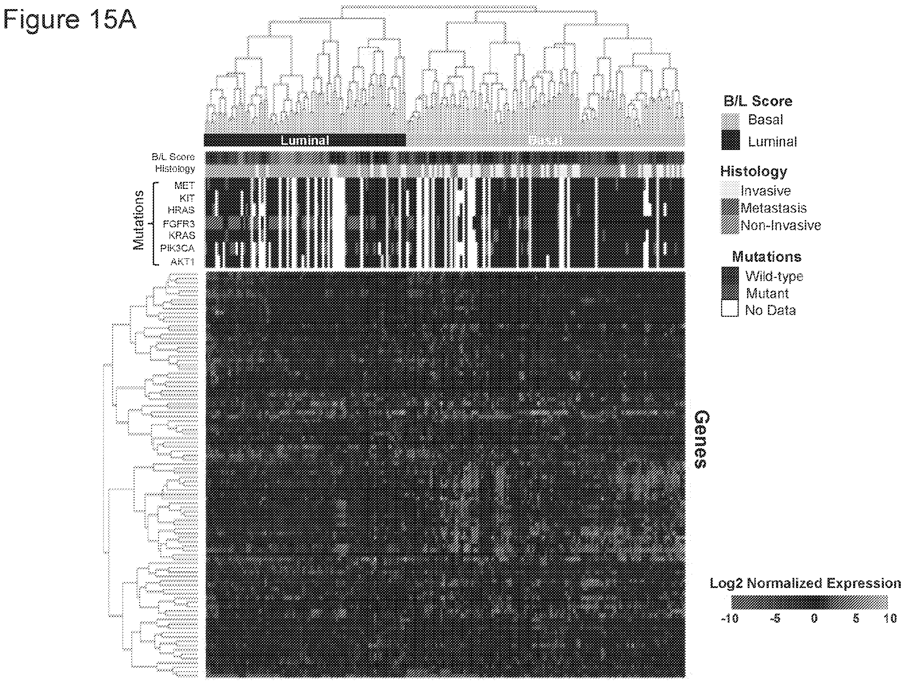

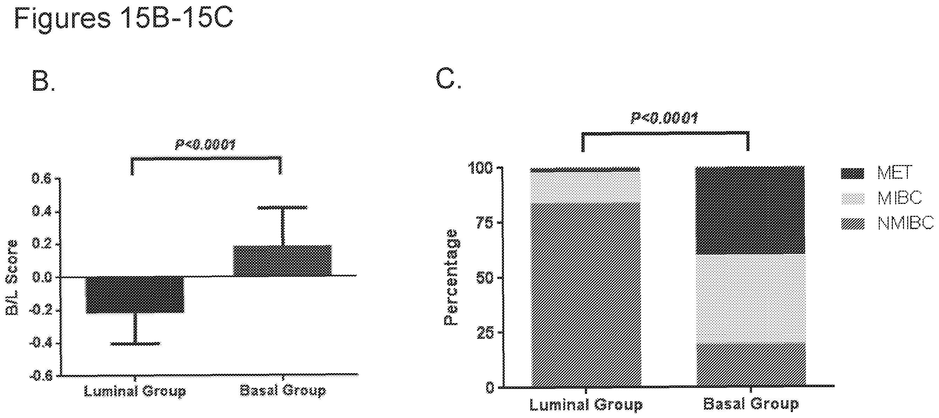

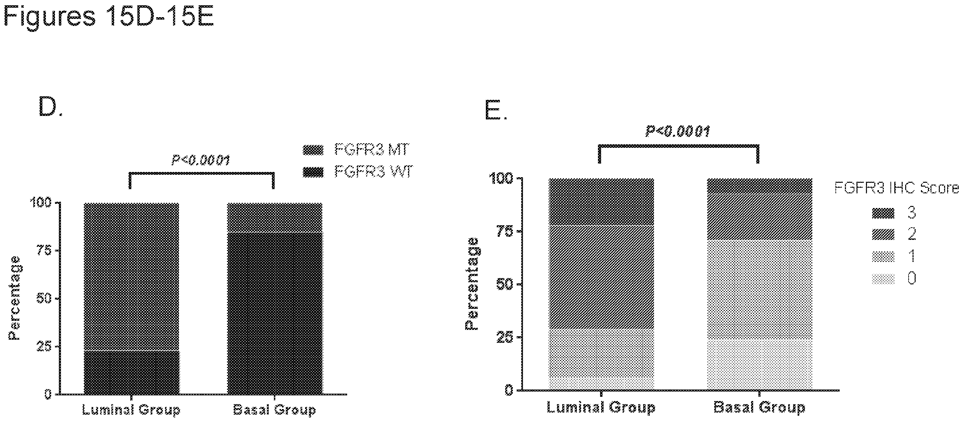

Unsupervised hierarchical clustering of the 204 FFPE samples described in Example 1 and Figure 1 showed two main branches with distinct patterns of gene expression: (1) a right branch under which samples with low B/L similarity scores (luminal-like) were co-clustered; and (2) a left branch that was comprised of samples with high B/L scores (basal-like) (Figure 15A). Statistical analysis confirmed a significantly lower B/L score in the left branch indicative of luminal status, and a significantly higher B/L score consistent with basal status under the right arm of the hierarchical tree (Figure 15B, top left). We observed a significantly higher fraction of NMIBCs in the luminal compared to the basal group (Figure 15B, top right). MIBCs were represented in both basal and luminal groups; however, a significantly higher proportion of MIBCs was observed in the basal group (Figure 15B). Interestingly, almost all METs clustered under the basal arm of the hierarchical tree, suggesting that the majority of the METs in the bladder cancer cohort described in Example 1 and Figure 1 exhibited a basal transcriptional profile (Figures 15A and 15C).

As described above, one of the characteristic features of NMIBCs is that they carry somatic activating mutations in *FGFR3* in approximately 60-80% of cases, and a much lower frequency of approximately 15% has been reported in MIBCs and METs. We assessed mutational status of *FGFR3* and other cancer-relevant genes in samples from cohort using a custom allele-specific PCR panel (Schleifman et al. *supra*; Tomlinson et al. *supra;* van Rhijn et al. *supra*; Martinez-Torrecuadrada et al. *supra*; Cappellen et al. *supra*, 1999; and Al-Ahmadie et al. *supra*). As expected, we found that approximately 75% of the tumors co-clustered under the luminal label, a significantly higher fraction than the approximately 20% observed in samples from the basal group (Figures 15A and 15D). Mutations in *FGFR3* have been reported to drive higher expression levels of FGFR3 protein. Consistent with these reports, we noted significantly higher levels of FGFR3 protein, as measured by IHC, in samples from the luminal group compared to those from the basal group (Figures 15A and 15D). The *FGFR3* mutational and expression data provides molecular confirmation of the luminal and basal status of our samples.

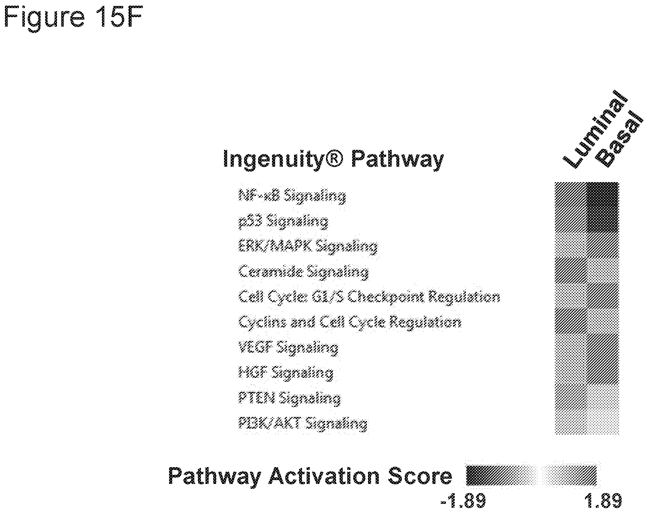

Beyond B/L transcriptional characteristics, we assessed the utility of the custom bladder cancer Fluidigm panel in measuring the expression of genes belonging to pathways known to be involved in bladder carcinogenesis, such as the TP53 pathway, PI3K/AKT pathway, ERK/MAPK pathway, and components of the cell cycle signaling axis. We used permutation-adjusted p-values to control for multiple hypothesis testing, identified 49/91 unique genes as being significantly differentially expressed between the basal and luminal groups (Table 4), and mapped these genes to pathways using INGENUITY® software (Figure 15F).

**Table 4: Genes differentially expressed between basal and luminal groups**

| **Gene** | **index** | **Raw P value** | **Adjusted P Value** | **Luminal mean gene** | **Luminal Std. Dev. gene** | **Basal mean gene** | **Basal Std. Dev gene** |
|---|---|---|---|---|---|---|---|
| ACVRL1 | 4.470992 | 1.00E-04 | 6.00E-04 | -0.377392 | 0.897960 | 0.202966 | 0.850912 |
| ADAM12 | 10.714632 | 1.00E-04 | 1.00E-04 | -1.007148 | 1.132818 | 1.073608 | 1.529736 |
| ARAF | -5.315843 | 1.00E-04 | 2.00E-04 | 0.187836 | 0.418587 | -0.159546 | 0.472994 |
| AXL | 7.402348 | 1.00E-04 | 1.00E-04 | -0.568450 | 0.864129 | 0.468449 | 1.052838 |
| BCL2 | 3.327797 | 0.0012 | 0.0392 | -0.599461 | 2.561637 | 0.476490 | 1.529934 |
| BMP2 | -5.506106 | 1.00E-04 | 2.00E-04 | 0.511055 | 1.388453 | -0.842703 | 1.982201 |
| BMX | 3.869332 | 3.00E-04 | 0.007 | -0.365686 | 1.125407 | 0.432270 | 1.699462 |
| CCND1 | -6.648141 | 1.00E-04 | 1.00E-04 | 0.614065 | 1.302003 | -0.828593 | 1.671470 |
| CDH1 | -6.186010 | 1.00E-04 | 1.00E-04 | 0.308209 | 0.626927 | -0.554378 | 1.255825 |
| CDH2 | 6.767331 | 1.00E-04 | 1.00E-04 | -0.902216 | 1.477841 | 0.789598 | 1.949093 |
| CDKN1A | -6.302967 | 1.00E-04 | 1.00E-04 | 0.379429 | 0.948515 | -0.569761 | 1.109340 |
| CXCL1 | 6.101031 | 1.00E-04 | 1.00E-04 | -3.427841 | 5.233372 | 0.556695 | 2.950078 |
| DUSP1 | 8.894016 | 1.00E-04 | 1.00E-04 | -0.569185 | 1.050108 | 1.194473 | 1.662705 |
| DUSP6 | -4.465429 | 1.00E-04 | 6.00E-04 | 0.417010 | 1.107095 | -0.372185 | 1.309016 |
| ERBB3 | -6.987278 | 1.00E-04 | 1.00E-04 | 0.389256 | 0.766742 | -0.753400 | 1.450475 |
| FGF1 | 4.885552 | 1.00E-04 | 3.00E-04 | -0.540165 | 1.013078 | 0.318677 | 1.397265 |
| FGF10 | 7.953402 | 1.00E-04 | 1.00E-04 | -2.155413 | 2.835254 | 0.693114 | 1.700124 |
| FGF2 | 4.811158 | 1.00E-04 | 3.00E-04 | -0.573742 | 1.578183 | 0.416837 | 1.088209 |
| FGF7 | 10.092045 | 1.00E-04 | 1.00E-04 | -3.061246 | 3.278922 | 1.283664 | 2.317224 |
| FGFR1 | 9.680571 | 1.00E-04 | 1.00E-04 | -0.951231 | 1.204470 | 0.572352 | 0.835639 |
| FGFR2 | -4.331482 | 2.00E-04 | 0.0013 | 0.297819 | 0.883154 | -0.529580 | 1.703266 |
| FGFR3 | -8.404252 | 1.00E-04 | 1.00E-04 | 0.839686 | 1.482499 | -1.598363 | 2.478029 |
| FN1 | 8.759580 | 1.00E-04 | 1.00E-04 | -1.026195 | 1.398537 | 0.927437 | 1.650205 |
| GATA3 | -5.228866 | 1.00E-04 | 2.00E-04 | 0.302732 | 1.061456 | -0.922919 | 2.105784 |
| HPSE | 7.679572 | 1.00E-04 | 1.00E-04 | -0.715992 | 1.228429 | 0.728751 | 1.332539 |
| JAG1 | -4.036317 | 1.00E-04 | 0.0034 | 0.255673 | 0.778658 | -0.289823 | 1.074384 |
| KDR | -4.325874 | 1.00E-04 | 0.0013 | 0.258180 | 0.953954 | -0.394809 | 1.108172 |
| MET | -5.005419 | 1.00E-04 | 2.00E-04 | 0.255420 | 0.616484 | -0.350746 | 1.036428 |
| MMP9 | 8.924212 | 1.00E-04 | 1.00E-04 | -2.041185 | 2.736507 | 1.112735 | 1.811595 |
| NF1 | -6.641973 | 1.00E-04 | 1.00E-04 | 0.227033 | 0.418262 | -0.248162 | 0.562626 |
| NOTCH1 | -4.467071 | 1.00E-04 | 6.00E-04 | 0.330623 | 0.996084 | -0.296385 | 0.882330 |
| PIK3CB | -4.982987 | 1.00E-04 | 3.00E-04 | 0.202197 | 0.459353 | -0.158992 | 0.530615 |
| PIK31P1 | -4.399226 | 1.00E-04 | 0.001 | 0.217791 | 0.743936 | -0.333888 | 0.974954 |
| POU5F1 | -4.881466 | 1.00E-04 | 3.00E-04 | 0.432596 | 0.827815 | -0.253551 | 1.099119 |
| RB1 | -4.511634 | 1.00E-04 | 6.00E-04 | 0.433954 | 0.990320 | -0.250475 | 1.074869 |
| S1PR1 | 3.347376 | 8.00E-04 | 0.0368 | -0.936400 | 2.098207 | 0.115313 | 2.164647 |
| SMO | 4.108037 | 2.00E-04 | 0.0029 | -0.570215 | 1.316376 | 0.232323 | 1.330061 |
| SNAI1 | 9.720121 | 1.00E-04 | 1.00E-04 | -0.738706 | 0.826979 | 0.693255 | 1.192486 |
| SPHK1 | 9.390921 | 1.00E-04 | 1.00E-04 | -1.073262 | 1.331036 | 0.798386 | 1.373050 |
| SRC | -8.409151 | 1.00E-04 | 1.00E-04 | 0.440559 | 0.616653 | -0.588228 | 1.052038 |
| TIMP1 | 5.789346 | 1.00E-04 | 1.00E-04 | -0.440995 | 0.777238 | 0.424382 | 1.265678 |
| TIMP2 | 7.997448 | 1.00E-04 | 1.00E-04 | -0.693068 | 0.994608 | 0.562759 | 1.150134 |
| TP53 | -6.756884 | 1.00E-04 | 1.00E-04 | 0.283202 | 0.585345 | -0.359823 | 0.717235 |
| TP63 | -9.036731 | 1.00E-04 | 1.00E-04 | 0.541066 | 1.058843 | -1.725567 | 2.304475 |
| TP73 | -3.264090 | 0.0014 | 0.0477 | 0.355626 | 1.529634 | -0.413410 | 1.679960 |
| TSC1 | -4.289175 | 1.00E-04 | 0.0015 | 0.239069 | 0.686542 | -0.196540 | 0.688436 |
| ZEB1 | 6.681040 | 1.00E-04 | 1.00E-04 | -0.451146 | 0.807057 | 0.310871 | 0.720614 |
| ZEB2 | 9.851331 | 1.00E-04 | 1.00E-04 | -0.718784 | 0.956315 | 0.632488 | 0.888236 |

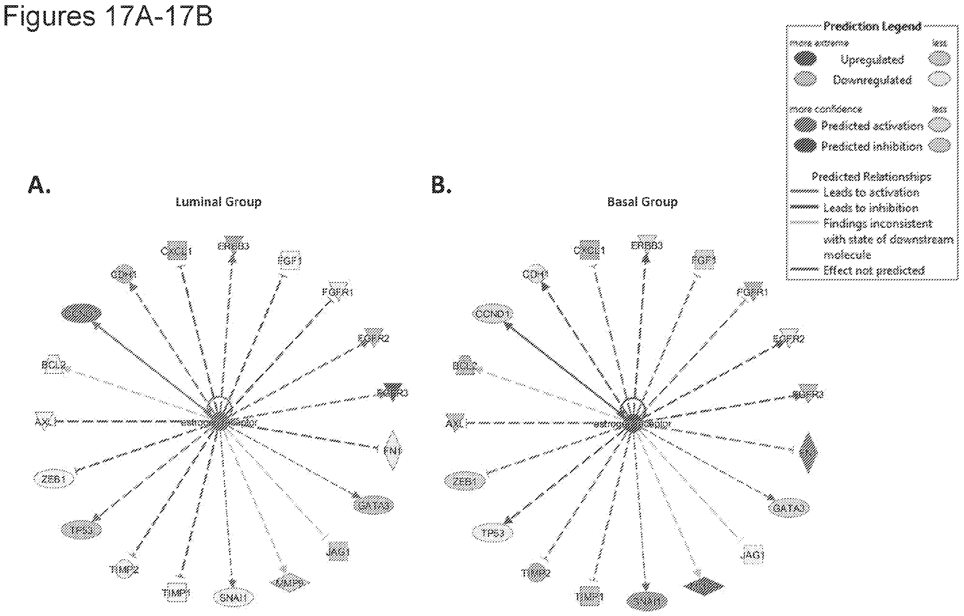

Pathways that exhibited a significant activation score in luminal tumors included the NF-κB, TP53, ERK/MAP, G1/S cell cycle checkpoint, HGF, and VEGF signaling pathways (Figure 15F). On the other hand, pathways that were significantly activated in the basal group included the PTEN, PI3K/AKT, ceramide, and cell cycle regulation signaling pathways (Figure 15F). Upstream pathway activation analysis using INGENUITY® software also revealed that samples belonging to the luminal group had an expression profile consistent with estrogen receptor (ER) activation, including high levels of FGFR3, GATA3, CCND1, and ERBB3, low levels of AXL1, CXCL1, FGFR1, and BCL2, as well as low expression levels of EMT genes, such as SNAI1 and ZEB1 (Figure 17A). In contrast, samples belonging to the basal group exhibited the opposite expression profiles for these genes (Figure 17B). These findings demonstrate that our panel is informative for measuring the transcriptional status of bladder cancer-relevant pathways in FFPE tissues.

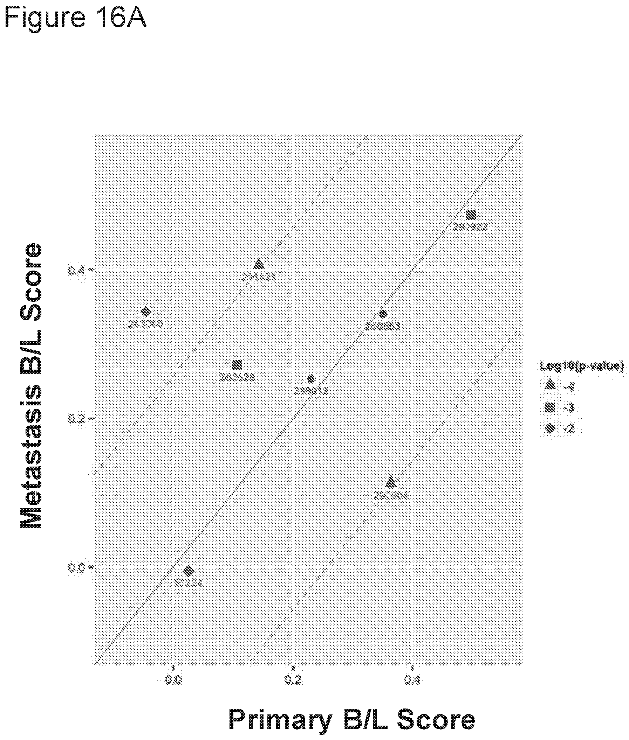

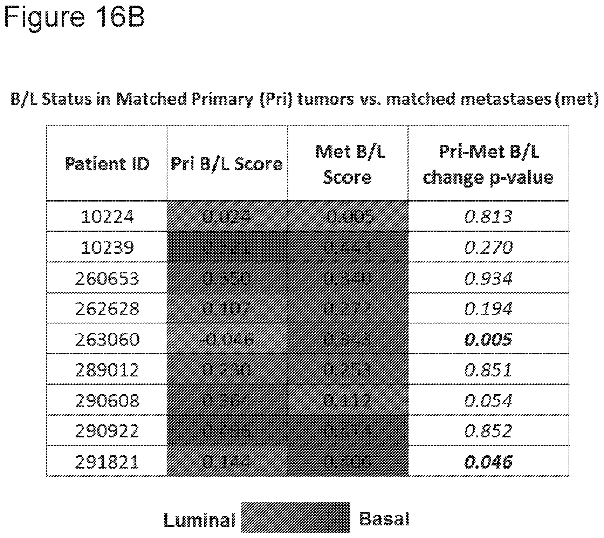

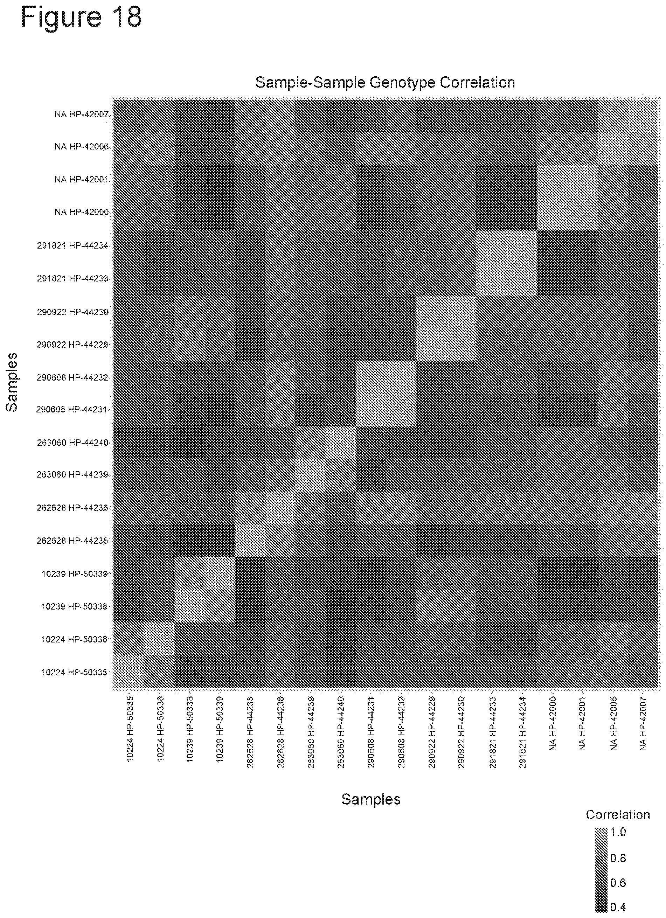

Histopathological features of primary bladder cancer have historically driven clinical management decisions for bladder cancer patients. With increased understanding of the genetic drivers of bladder cancers, new avenues are being paved for therapeutic intervention based on the molecular attributes of the disease. However, molecular characteristics of primary tumors are often used to guide clinical decisions with targeted agents, even for drugs that are being developed in the metastatic setting. To begin to address whether the key molecular features of primary tumors are representative of those in bladder cancer metastases on a transcriptional level, we compared the B/L scores of 9 matched primary tumor/MET pairs from the same patients. We first examined next generation sequencing (NGS) data and found that variants from all 9 primary/metastasis pairs were highly correlated, thus establishing that the matched tumors were indeed from the same patients (Figures 16A and 18). Correlation analysis between the B/L scores of primary tumors and matched METs indicated that 5/9 pairs had nearly identical B/L scores, and 2/9 exhibited insignificant differences in their B/L status (Figures 17A and 17B). Interestingly, we observed a significant change in the B/L scores in 2/9 cases, both of which were characterized as having luminal primary tumors and basal METs (Figures 17A and 17B). These findings suggest that the B/L status of primary tumor does not always reflect that observed in metastatic lesions, and indicates that molecular characterization of bladder cancer metastases might be warranted to more accurately guide treatment decisions in the clinic.

### Additional Materials and Methods for Example 6

### Analysis of public data sets

Several public datasets were used to ascertain the predictive ability the custom bladder cancer Fluidigm panel genes to capture tumor stage, grade, and key transcriptional features of the disease, such as basal/luminal status. Sjödahl Illumina Human HT-12 V3.0 gene expression data (Sjödahl et al. 2012, *supra*) was downloaded from the Gene Expression Omnibus (GEO) website (ncbi.nlm.nih.gov/geo/GSE32894). Expression data was normalized using median polish (Tukey et al. Exploratory Data Analysis, Addison-Wesley, publishers, 1977). Unsupervised hierarchical clustering analysis was applied using an average-linkage, 1 - Pearson correlation distance metric to find sample groupings, and reported sample tumor grade, TMN stage, and molecular class was used (Sjödahl et al. 2012, *supra*)*.* The ability of the 91 unique genes of the custom bladder cancer Fluidigm panel was assessed for correctly identifying tumor grade, TNM stage, and molecular class was determined using a centroid classifier approach. Cross-validated misclassification error curves were created using the PAMR R library (Tibshirani et al. Proc. Natl. Acad. Sci. USA 99:6567-6572, 2002) using the full array or the corresponding 125 probe subset of the Sjödahl et al. (*supra*) expression data set. The ability of the genes of the custom bladder cancer Fluidigm panel to identify basal-like and luminal-like samples through transcriptional profiling was assessed in four literature data sets (GSE32894, GSE5287, GSE13507, GSE48075).

Basal or luminal classifications made on these public data was as described by Damrauer et al. (*supra*)*.* Briefly, the Damrauer et al. (*supra*) discovery samples (N=30, Affymetrix Human Genome U133A Array) were downloaded from the NCBI Gene Expression Omnibus (GSE5287). Affymetrix probe sets corresponding to genes on the custom bladder cancer Fluidigm panel were used. The remaining subset of log-transformed expression data were then mean centered and normalized to unit variance. Average gene expression values were then calculated for the 91 unique genes across the 12 samples classified as luminal-like and 18 samples classified as basal-like (classifications received through author correspondence) to produce basal and luminal expression profiles. This process was repeated for three other public datasets (GSE32894, GSE13507, GSE48075).

### Tumors

The collection of 204 formalin-fixed paraffin-embedded (FFPE) bladder tumor samples as described in Example 1 were used in the experiments described in this Example. RNA and DNA were extracted from macrodissected samples as described in Example 1.

### Fluidigm expression analysis of FFPE tumors

Gene expression analysis was carried out on RNA extracted from FFPE macrodissected using the High Pure FFPE RNA Micro Kit (Roche Diagnostics, Indianapolis, IN) after de-paraffinization with ENVIRENE®, as described previously (O'Brien et al. Clin. Cancer Res. 16:3670-3683, 2010). Gene expression analysis of 96 unique mRNA transcripts of bladder cancer-relevant genes was performed on patient specimens starting with 100 ng total RNA that was reverse-transcribed to cDNA and pre-amplified in a single reaction using Superscript III/Platinum Taq and pre-amplification reaction mix (Invitrogen, Carlsbad, CA). All 96 Taqman primer/probe sets were included in the pre-amplification reaction at a final dilution of 0.05x original TAQMAN® assay concentration (Applied Biosystems, Foster City, CA). The thermocycling conditions were as follows: 1 cycle of 50°C for 15 min, 1 cycle of 70°C for 2 min, 14 cycles of 95°C for 15 sec, and 60°C for 4 min. Pre-amplified cDNA was diluted 2-fold and then amplified using TAQMAN® Universal PCR MasterMix (Applied Biosystems, Foster City, CA) on the BIOMARK™ BMK-M-96.96 platform (Fluidigm, South San Francisco, CA) according to the manufacturer's instructions. All samples were assayed in triplicate. Cycle threshold (Ct) values were converted to relative expression using the ΔCt method (O'Brien et al. *supra*), where ΔCt was the mean of the target gene minus the geometric mean of reference genes calculated for the respective patient specimen. For genes assessed on the custom bladder cancer Fluidigm panel, Cycle threshold (Ct) values were normalized using median polish (Tukey et al. *supra*). Hierarchical clustering of differentially expressed genes was carried out on normalized data with the average-linkage method using 1 - Pearson correlation as a distance metric and subsequently visualized using R.

### Mutation analysis

Mutation analyses were carried out on genomic DNA extracted from macrodissected FFPE tissues using the QIAamp FFPE kit (Qiagen, Valencia, CA) after deparaffinization with ENVIRENE® (Lindgren et al. PLoS One 7:e38863, 2012). Mutations in *PIK3CA*, *EGFR*, *KRAS*, *NRAS*, *HRAS*, *FGFR3*, *MET*, *BRAF*, *KIT*, *AKT1*, *FLT3* were detected using mutation-specific qPCR as described previously (Schliefman et al. PLoS One 9:e88401, 2014).

### Immunohistochemistry

Immunohistochemistry was performed as described in Example 1.

### Calculation of basal/luminal similarity scores, analysis of matched primary tumors and metastases, and statistical analysis

Basal/luminal similarity scores for samples from the 204 bladder cancer cohort FFPE samples described in Example 1 were determined by first deriving basal/luminal expression profiles from the public Damrauer et al. discovery data set, as previously described (Damrauer et al. *supra*)*.* Next, basal and luminal Pearson correlations between each profile and each mean-centered, unit variance-normalized FFPE sample were determined. These two correlation scores were combined to produce an overall B/L similarity score: B/L score = (basal profile correlation - luminal profile correlation)/2.0. The B/L score had a range of +1.0 to -1.0, with scores above zero indicating a basal-like sample, and scores below zero indicating a luminal-like sample. Similarly, the B/L similarity scores of 9 matched primary and metastases samples were calculated in a similar manner. To confirm the matched samples were from the same patients, next generation sequencing (NGS) was carried out using the ION AMPLISEQ™ Cancer Hotspot Panel v2 (Life Technologies, Carlsbad, CA) according to manufacturer guidelines (Tsongalis et al. CCLM/FESCC 52:707-714, 2014), and Pearson correlation was calculated by comparing the allele frequency at all variant sites where both samples had at least 100x read coverage and at least one sample had an allele frequency > 10% (on average, 120 sites were compared between any two samples). The likelihood that the primary-metastases samples were mismatched was found by comparing their correlation scores to the 3,500 correlations of random sample pairings. Differences in B/L score between primaries and matched metastases were compared to differences expected due to typical systemic error. Estimation of systemic error were achieved by sampling genes from the custom bladder cancer Fluidigm panel with replacement for randomly selected samples and re-calculating B/L scores at 100,000 permutations. For the significance of differential mutation frequency between clusters, Fisher's exact test was performed. Fisher's exact test and two-tailed t-tests were used for all other statistical comparisons.

## Claims

1. An FGFR3 antagonist, a TP53 antagonist, or an EGFR antagonist for use in treating a patient suffering from a non-muscle invasive bladder cancer (NMIBC), wherein the expression level of at least two of the following genes: *FGFR3*, *TP53*, and *EGFR*, in a sample obtained from the patient has been determined to be increased relative to a reference level of the at least two genes, optionally wherein the NMIBC is a recurrent NMIBC.

2. A method for the prognosis of a patient suffering from a NMIBC, the method comprising:
(a) determining the expression level of at least two of the following genes: *FGFR3*, *TP53*, and *EGFR*, in a sample obtained from the patient;
(b) comparing the expression level of the at least two genes to a reference level of the at least two genes; and
(c) determining a prognosis for the patient, wherein a poor prognosis is indicated by an expression level of the at least two genes in the patient sample that is increased relative to the reference level; optionally wherein the prognosis is a prognosis of survival, preferably wherein the survival is disease-free survival or overall survival; and further optionally wherein the NMIBC is a recurrent NMIBC.

3. The method of claim 2, wherein the method is carried out prior to treatment of the patient with an anti-cancer therapy.

4. The method of claim 2 or 3, further comprising (d) identifying the patient as likely to benefit from administration of an anti-cancer therapy when the patient is determined to have a poor prognosis of survival.

5. The method of any one of claims 2-4, wherein the anti-cancer therapy comprises an FGFR3 antagonist, a TP53 antagonist, and/or an EGFR antagonist, optionally wherein the anti-cancer therapy comprises an FGFR3 antagonist and an EGFR antagonist.

6. The method of claim 5, wherein the FGFR3 antagonist, EGFR antagonist, or TP53 antagonist is (i) an antibody or a functional fragment thereof, or (ii) a small molecule antagonist.

7. The method of claim 6, wherein:
(i) the FGFR3 antagonist is an anti-FGFR3 antibody, or a functional fragment thereof;
(ii) the EGFR antagonist is an anti-EGFR antibody, or a functional fragment thereof; and/or
(iii) the TP53 antagonist is an anti-TP53 antibody, or a functional fragment thereof.

8. The method of claim 6, wherein the FGFR3 antagonist or EGFR antagonist is a tyrosine kinase inhibitor, optionally wherein the EGFR antagonist is erlotinib (TARCEVA™).

9. The method of any one of claims 5-8, wherein the anti-cancer therapy further comprises (i) an agent selected from the group consisting of an anti-neoplastic agent, a chemotherapeutic agent, a growth-inhibitory agent, and a cytotoxic agent, (ii) radiotherapy, or (iii) a combination thereof.

10. The method of any one of claims 2-9, wherein:
(i) the expression level of the at least two genes in the sample obtained from the patient is determined by measuring mRNA, optionally wherein the expression level of the at least two genes in the sample obtained from the patient is determined by a polymerase chain reaction (PCR) assay, further optionally wherein the PCR assay is a quantitative PCR assay; or
(ii) the expression level of the at least two genes in the sample obtained from the patient is determined by measuring protein, optionally wherein the expression level of the at least two genes in the sample obtained from the patient is determined by an immunohistochemical (IHC) method.

11. The method of any one of claims 1-10, wherein the sample obtained from the patient is a tumor sample, optionally wherein the tumor sample is a formalin-fixed paraffin-embedded (FFPE) tumor sample.

12. The method of any one of claims 1-11, further comprising determining the expression level of all three of the genes.

13. The method of any one of claims 1-12, wherein:
(i) the expression level of *FGFR3* has been determined to be increased at least 2-fold relative to a reference level, optionally wherein the expression level of *FGFR3* has been determined to be increased at least 4-fold relative to a reference level; or
(ii) the expression level of *EGFR* has been determined to be increased at least 4-fold relative to a reference level, optionally wherein the expression level of *EGFR* has been determined to be increased at least 8-fold relative to a reference level.

14. The method of any one of claims 2-13, further comprising determining the expression level of at least one additional gene selected from the group consisting of: *DUSB3*, *FRS2*, *TSC1*, *ERBB3*, *CDKN1A*, *CCND1*, *TP63*, *MMP2*, *ZEB2*, *PIK3CB, PIK3R1*, *MDM2*, *SNAI2*, *AXL*, *ZEB1*, *BCL2B*, *TSC2*, *RB1*, *FGFR32*, *PIK3IP1*, *MTOR*, *PIK3CA*, *PTEN*, *AKT1*, *BCL2A*, *FRS3*, *ERBB2*, *FGFR31*, *FGF1*, *SNAI1*, *FGFR34, FGF9,* and *FGF2*, in a sample obtained from the patient, wherein the expression level of the at least one additional gene is changed relative to a reference level of the at least one additional gene.

15. The method any one of claims 2-14, wherein the NMIBC is a recurrent NMIBC.

## Patentansprüche

1. FGFR3-Antagonist, TP53-Antagonist oder EGFR-Antagonist zur Verwendung bei der Behandlung eines Patienten, der unter einem nicht-muskelinvasiven Blasenkrebs (NMIBC) leidet, wobei bestimmt wurde, dass das Expressionsniveau von mindestens zwei der folgenden Gene: *FGFR3*, *TP53* und *EGFR* in einer von dem Patienten erhaltenen Probe bezogen auf ein Referenzniveau der mindestens zwei Gene erhöht ist, gegebenenfalls wobei der NMIBC ein rezidivierender NMIBC ist.

2. Verfahren für die Prognose eines Patienten, der unter einem NMIBC leidet, wobei das Verfahren Folgendes umfasst:
(a) Bestimmen des Expressionsniveaus von mindestens zwei der folgenden Gene: *FGFR3*, *TP53* und *EGFR* in einer von dem Patienten erhaltenen Probe;
(b) Vergleichen des Expressionsniveaus der mindestens zwei Gene mit einem Referenzniveau der mindestens zwei Gene; und
(c) Bestimmen einer Prognose für den Patienten, wobei eine schlechte Prognose durch ein Expressionsniveau der mindestens zwei Gene in der Patientenprobe angegeben wird, das bezogen auf das Referenzniveau erhöht ist; gegebenenfalls wobei die Prognose eine Überlebensprognose ist, vorzugsweise wobei das Überleben ein krankheitsfreies Überleben oder Gesamtüberleben ist; und ferner gegebenenfalls wobei der NMIBC ein rezidivierender NMIBC ist.

3. Verfahren nach Anspruch 2, wobei das Verfahren vor der Behandlung des Patienten mit einer Antikrebstherapie durchgeführt wird.

4. Verfahren nach Anspruch 2 oder 3, ferner umfassend (d) Identifizieren des Patienten als wahrscheinlich von der Verabfolgung einer Antikrebstherapie profitierend, wenn bestimmt wird, dass der Patient eine schlechte Überlebensprognose hat.

5. Verfahren nach einem der Ansprüche 2-4, wobei die Antikrebstherapie einen FGFR3-Antagonisten, einen TP53-Antagonisten und/oder einen EGFR-Antagonisten umfasst, gegebenenfalls wobei die Antikrebstherapie einen FGFR3-Antagonisten und einen EGFR-Antagonisten umfasst.

6. Verfahren nach Anspruch 5, wobei der FGFR3-Antagonist, der EGFR-Antagonist oder der TP53-Antagonist (i) ein Antikörper oder ein funktionelles Fragment davon oder (ii) ein niedermolekularer Antagonist ist.

7. Verfahren nach Anspruch 6, wobei:
(i) der FGFR3-Antagonist ein Anti-FGFR3-Antikörper oder ein funktionelles Fragment davon ist;
(ii) der EGFR-Antagonist ein Anti-EGFR-Antikörper oder ein funktionelles Fragment davon ist; und/oder
(iii) der TP53-Antagonist ein Anti-TP53-Antikörper oder ein funktionelles Fragment davon ist.

8. Verfahren nach Anspruch 6, wobei der FGFR3-Antagonist oder der EGFR-Antagonist ein Tyrosinkinaseinhibitor ist, wobei der EGFR-Antagonist gegebenenfalls Erlotinib (TARCEVA™) ist.

9. Verfahren nach einem der Ansprüche 5-8, wobei die Antikrebstherapie ferner
(i) ein Mittel, ausgewählt aus der Gruppe, bestehend aus einem Antineoplastikum, einem Chemotherapeutikum, einem wachstumshemmenden Mittel und einem zytotoxischen Mittel,
(ii) Strahlentherapie oder (iii) eine Kombination davon umfasst.

10. Verfahren nach einem der Ansprüche 2-9, wobei:
(i) das Expressionsniveau der mindestens zwei Gene in der von dem Patienten erhaltenen Probe durch Messen von mRNA bestimmt wird, gegebenenfalls wobei das Expressionsniveau der mindestens zwei Gene in der von dem Patienten erhaltenen Probe durch einen Polymerasekettenreaktion- (PCR-) Assay bestimmt wird, ferner gegebenenfalls wobei der PCR-Assay ein quantitativer PCR-Assay ist; oder
(ii) das Expressionsniveau der mindestens zwei Gene in der von dem Patienten erhaltenen Probe durch Messen von Protein bestimmt wird, gegebenenfalls wobei das Expressionsniveau der mindestens zwei Gene in der von dem Patienten erhaltenen Probe durch ein immunhistochemisches (IHC) Verfahren bestimmt wird.

11. Verfahren nach einem der Ansprüche 1-10, wobei die von dem Patienten erhaltene Probe eine Tumorprobe ist, gegebenenfalls wobei die Tumorprobe eine formalinfixierte, paraffineingebettete (FFPE) Tumorprobe ist.

12. Verfahren nach einem der Ansprüche 1-11, ferner umfassend das Bestimmen des Expressionsniveaus aller drei Gene.

13. Verfahren nach einem der Ansprüche 1-12, wobei:
(i) bestimmt wurde, dass das Expressionsniveau von *FGFR3* um mindestens das 2-Fache bezogen auf ein Referenzniveau erhöht ist, gegebenenfalls wobei bestimmt wurde, dass das Expressionsniveau von *FGFR3* um mindestens das 4-Fache bezogen auf ein Referenzniveau erhöht ist; oder
(ii) bestimmt wurde, dass das Expressionsniveau von *EGFR* um mindestens das 4-Fache bezogen auf ein Referenzniveau erhöht ist, gegebenenfalls wobei bestimmt wurde, dass das Expressionsniveau von *EGFR* um mindestens das 8-Fache bezogen auf ein Referenzniveau erhöht ist.

14. Verfahren nach einem der Ansprüche 2-13, ferner umfassend das Bestimmen des Expressionsniveaus mindestens eines zusätzlichen Gens, ausgewählt aus der Gruppe, bestehend aus: *DUSB3*, *FRS2*, *TSC1*, *ERBB3*, *CDKN1A*, *CCND1*, *TP63*, *MMP2*, *ZEB2*, *PIK3CB*, *PIK3R1*, *MDM2*, *SNAI2*, *AXL*, *ZEB1*, *BCL2B*, *TSC2*, *RB1*, *FGFR32*, *PIK3IP1, MTOR*, *PIK3CA*, *PTEN*, *AKT1*, *BCL2A*, *FRS3*, *ERBB2*, *FGFR31*, *FGF1, SNAI1*, *FGFR34*, *FGF9* und *FGF2*, in einer von dem Patienten erhaltenen Probe, wobei das Expressionsniveau des mindestens einen zusätzlichen Gens bezogen auf ein Referenzniveau des mindestens einen zusätzlichen Gens verändert ist.

15. Verfahren nach einem der Ansprüche 2-14, wobei der NMIBC ein rezidivierender NMIBC ist.

## Revendications

1. Antagoniste de FGFR3, antagoniste de TP53 ou antagoniste d'EGFR pour une utilisation dans le traitement d'un patient souffrant d'un cancer de la vessie non invasif sur le plan musculaire (NMIBC), dans lequel le taux d'expression d'au moins deux des gènes suivants : *FGFR3*, *TP53* et *EGFR*, dans un échantillon obtenu auprès du patient a été déterminé comme étant augmenté par rapport à un taux de référence des au moins deux gènes, éventuellement dans lequel le NMIBC est un NMIBC récurrent.

2. Méthode de pronostic d'un patient souffrant d'un NMIBC, la méthode comprenant :
(a) la détermination du taux d'expression d'au moins deux des gènes suivants : *FGFR3*, *TP53* et *EGFR*, dans un échantillon obtenu auprès du patient ;
(b) la comparaison du taux d'expression des au moins deux gènes à un taux de référence des au moins deux gènes ; et
(c) la détermination d'un pronostic pour le patient, dans laquelle un mauvais pronostic est indiqué par un taux d'expression des au moins deux gènes dans l'échantillon du patient qui est augmenté par rapport au taux de référence ; éventuellement dans laquelle le pronostic est un pronostic de survie, de préférence dans laquelle la survie est une survie sans maladie ou une survie globale ; et en outre éventuellement dans laquelle le NMIBC est un NMIBC récurrent.

3. Méthode selon la revendication 2, dans laquelle la méthode est mise en œuvre avant traitement du patient par une thérapie anticancéreuse.

4. Méthode selon la revendication 2 ou 3, comprenant en outre (d) l'identification du patient comme étant susceptible de bénéficier de l'administration d'une thérapie anticancéreuse lorsqu'il est déterminé que le patient présente un mauvais pronostic de survie.

5. Méthode selon l'une quelconque des revendications 2 à 4, dans laquelle la thérapie anticancéreuse comprend un antagoniste de FGFR3, un antagoniste de TP53 et/ou un antagoniste d'EGFR, éventuellement dans laquelle la thérapie anticancéreuse comprend un antagoniste de FGFR3 et un antagoniste d'EGFR.

6. Méthode selon la revendication 5, dans laquelle l'antagoniste de FGFR3, l'antagoniste d'EGFR ou l'antagoniste de TP53 est (i) un anticorps ou un fragment fonctionnel de celui-ci, ou (ii) un antagoniste de type petite molécule.

7. Méthode selon la revendication 6, dans laquelle :
(i) l'antagoniste de FGFR3 est un anticorps anti-FGFR3, ou un fragment fonctionnel de celui-ci ;
(ii) l'antagoniste d'EGFR est un anticorps anti-EGFR, ou un fragment fonctionnel de celui-ci ; et/ou
(iii) l'antagoniste de TP53 est un anticorps anti-TP53, ou un fragment fonctionnel de celui-ci.

8. Méthode selon la revendication 6, dans laquelle l'antagoniste de FGFR3 ou l'antagoniste d'EGFR est un inhibiteur de la tyrosine kinase, éventuellement dans laquelle l'antagoniste d'EGFR est l'erlotinib (TARCEVA™).

9. Méthode selon l'une quelconque des revendications 5 à 8, dans laquelle la thérapie anticancéreuse comprend en outre (i) un agent choisi dans le groupe constitué par un agent antinéoplasique, un agent chimiothérapique, un agent inhibiteur de croissance et un agent cytotoxique, (ii) une radiothérapie, ou (iii) une combinaison de ceux-ci.

10. Méthode selon l'une quelconque des revendications 2 à 9, dans laquelle :
(i) le taux d'expression des au moins deux gènes dans l'échantillon obtenu auprès du patient est déterminé par la mesure de l'ARNm, éventuellement dans laquelle le taux d'expression des au moins deux gènes dans l'échantillon obtenu auprès du patient est déterminé par un dosage par réaction en chaîne par polymérase (PCR), en outre éventuellement dans laquelle le dosage par PCR est un dosage par PCR quantitative ; ou
(ii) le taux d'expression des au moins deux gènes dans l'échantillon obtenu auprès du patient est déterminé par la mesure d'une protéine, éventuellement dans laquelle le taux d'expression des au moins deux gènes dans l'échantillon obtenu auprès du patient est déterminé par une méthode immunohistochimique (IHC).

11. Méthode selon l'une quelconque des revendications 1 à 10, dans laquelle l'échantillon obtenu auprès du patient est un échantillon tumoral, éventuellement dans laquelle l'échantillon tumoral est un échantillon tumoral fixé au formol et inclus en paraffine (FFPE).

12. Méthode selon l'une quelconque des revendications 1 à 11, comprenant en outre la détermination du taux d'expression des trois gènes.

13. Méthode selon l'une quelconque des revendications 1 à 12, dans laquelle :
(i) le taux d'expression de *FGFR3* a été déterminé comme étant augmenté d'au moins un facteur 2 par rapport à un taux de référence, éventuellement dans laquelle le taux d'expression de *FGFR3* a été déterminé comme étant augmenté d'au moins un facteur 4 par rapport à un taux de référence ; ou
(ii) le taux d'expression d'*EGFR* a été déterminé comme étant augmenté d'au moins un facteur 4 par rapport à un taux de référence, éventuellement dans laquelle le taux d'expression d'*EGFR* a été déterminé comme étant augmenté d'au moins un facteur 8 par rapport à un taux de référence.

14. Méthode selon l'une quelconque des revendications 2 à 13, comprenant en outre la détermination du taux d'expression d'au moins un gène supplémentaire choisi dans le groupe constitué par : *DUSB3*, *FRS2*, *TSC1*, *ERBB3*, *CDKN1A*, *CCND1*, *TP63*, *MMP2*, *ZEB2*, *PIK3CB*, *PIK3R1*, *MDM2*, *SNAI2*, *AXL*, *ZEB1*, *BCL2B*, *TSC2*, *RB1, FGFR32*, *PIK3IP1*, *MTOR*, *PIK3CA*, *PTEN*, *AKT1*, *BCL2A*, *FRS3*, *ERBB2*, *FGFR31*, *FGF1*, *SNAI1*, *FGFR34*, *FGF9* et *FGF2*, dans un échantillon obtenu auprès du patient, dans laquelle le taux d'expression de l'au moins un gène supplémentaire est modifié par rapport à un taux de référence de l'au moins un gène supplémentaire.

15. Méthode selon l'une quelconque des revendications 2 à 14, dans laquelle le NMIBC est un NMIBC récurrent.
